# EUROPEAN PATENT APPLICATION

(11) **EP 1 939 299 A1**
(43) Date of publication of application: **02.07.2008**
(21) Application number: 06127373.6
(22) Date of filing: 29.12.2006
(51) Int. Cl.: C12N 15/85, A01K 67/027, C12N 15/90, C07K 14/705

(54) **Trpm4-deficient transgenic mammals and use thereof**

(71) Applicant: Universität des Saarlandes, 66123 Saarbrücken (DE)
(72) Inventor: Flockerzi, Veit, 66440 Bierbach (DE); Freichel, Marc, 66740 Saarlouis (DE)
(74) Representative: von Kreisler Selting Werner

(57) **Abstract**

The present invention provides TRPM4-deficient non-human mammals. The invention further provides the production of such non-human mammals and its use as a test model for developing therapeutic strategy in allergy and hypersensitivity reactions.

SEQUENCE LISTING

<110>  Universitaet de Saarlandes

<120>  TRPM4-deficient  transgenic mammals and use thereof

<130>  061070ep/JH

<160>  2

<170>  PatentIn version 3.3

<210>  1
<211>  59704
<212>  DNA
<213>  Homo sapiens


<220>
<221>  misc_feature
<222>  (10737)..(10756)
<223>  n is a, c, g, or t

<220>
<221>  misc_feature
<222>  (15310)..(15359)
<223>  n is a, c, g, or t

<220>
<221>  misc_feature
<222>  (27559)..(27733)
<223>  n is a, c, g, or t

<220>
<221>  misc_feature
<222>  (36632)..(36712)
<223>  n is a, c, g, or t

<220>
<221>  misc_feature
<222>  (49662)..(49917)
<223>  n is a, c, g, or t

<400>  1
tcatggtgag catgtctagg agactggtgc cagtgttcag tggatacagc ctcatcttcc      60

tcttcctcat tttggtggct ttggcggcca tggctgggga catgatggta gtcttcttct     120

gagtcatcct catcatcttt gtgctcatgg tctctgtgtc tgtgggcttg atgcacatgc     180

tcagtagagt catcatctcc atcatcatca tcatcatctt cttcttcttc atagccttgg     240

cgtccatgcc tggagacatg atgatcatct tcatctgact catcctcatc ttcttccttc     300

ctgtggcctt gggtctggtg ccggtgctca gtggagtctt ctttatcttc atcatcatca     360

tcatcatcat catcgtcatc ttgctcttcg ttgtggcctt ggtggtcctg ggtatggtgt     420

ctgtattcac cagagtcacc gtcttcatca tcattttcat cttctcggcc ttgaactctg     480

tgggtgtgac tgtcttcatc tgagtcccca tcgtcttcct cctcatgatc ctgatatctg     540

tgagcttggt gtccatgctc agtagagaca ccatcgtcat cgtcatcatc actgtggcct     600

tggtgcctgt ggctgtgggc atgatggagt tcatctctct cctttgagtg gctctggtga 660
ccatggacct ggtgttcatt atcatcagag tcctccatca catccaccct ctcctcctcc 720
tctcctccat catcgtcatc atcttcctct ccgtggccat ggtgggcatg cctggggacg 780
tgacggtgat actcactgga gacaatgcca tcctcatctt cgtggctgtg gctcctctgt 840
ctggggaggt ggttctctgc cgagtctcct aaatcttcat tgtcatgttc ccggtgcccg 900
tggagctgtc taacatgccc tctgaagacc tcttcagaga cattctcctc tccaacctcg 960
cggccagggt acctgtggtc ttggaccctg tggccatatt ccctggagaa gtcttcaccc 1020
tcttctctgt ggcctctgtc cttctcacct tgatatcccc ggtggatgtg gtgaccaaac 1080
tcagttgata ggtcctctga ggacccaggg atgcctgcat tgttgttcca gttgcccagg 1140
cccagaccgg cccctctcaa ctcctgggtc accactggag ggaccagcag gatggccact 1200
gtggcccaaa ggagacaagt gtgcaaccat ggcccctgga agcccatggg gacgtttagc 1260
agctgagcgt ctgaccaggc tggcttcagc tgcctctgat gggtccttgt gcctttttgg 1320
ctttgacttt cagattgtcc cttttgtcgt ggtatagctc ccactctctg cctctctatc 1380
ttcctcaggt cttggatgac cctctgagga tgtccccttt gcagctgtga ccctggttcc 1440
aggacctccc ttcccactgc tggaccaatg gggtctgtcc cctccccttc ttgctgctaa 1500
atttatccag agcctaagtt attctggtca ctccctgcct gcccctcccc tcctgttcct 1560
ctggctcagg actgggtggt ggaggggtct tcatggcggg ggcggggggg gggaggcagg 1620
gacgtgacaa gctcaagttt ggcattttta gcacaaaggc tgaacgttta gagtgtcttg 1680
ctgggaaagc ctggggctct gggagaggag gatgcaggag cagagggccc cctgcaaggt 1740
aaacactggg aagcgtgcag gcagaatggg acattcggtg tgacagtggt gtcctgcaga 1800
agctgtggaa gacagtgaag gaatactgtg cccaacaccc catgacctcc tctcactgga 1860
actcagatct ggggacaggg gaccagacca gtgctataaa tacaacaggg gggaggtgct 1920
gggggctcag gttactaggc ctcagctgtc cccaccaaag ggacttggct gcaaacaggt 1980
gtcactgcct tctctgctgg tcagtgtcac ccaccccaca cttgtcagta tatccaccgt 2040
ctttgagttt ttgtccttta tgagtcaacc cccccccccc ccattatagt cactgttctg 2100
agcttccgtg tttcttctca ggattctaac ctctcctctc ctactgtgtc ttccctgatc 2160
cttctctttg attcttctct ctctctcttt ctgtgtgtgt gtgtctgtct gtctgtttct 2220
ctgtgtgtgt ctgtctgtct ctgtgtgtgt gtgcctgtct ctctgtgtgt ctgtctgtct 2280
ctgtgtgtgt ctgtgtttgt ctctgtctct cctctgtgtg tgtgttttct ctctctctgt 2340
ctctctcttt ctccctgtat ctctgtctct gtgtgtgtgt gtctctctct agttctctct 2400
gtgtctctgt tctctctgtc tctcctctct gaatctggga ctctgttgct ctatcacttt 2460

gtatttctat ctgtctctaa ctatctctgg atatctcatt ctttttctgt attgctgtct 2520

tcatattctc tctctctctc tctctctctc tctctctctc tctctctctc tctctctctc 2580

tctgggtctc tgcccccttt tctttctctg ggtccctctc ttagtttctg tctctctctc 2640

tctgggtctt tctctcagtc tctgtctctc cgaatctatg tttgtcccat ggctgacatc 2700

tatcgtgtgc cccactctgc ttccttcgtt cctccgcttg ctctgccttc caactctcgg 2760

tctttcttgt cccccaaacc gaccccatca ctggtttggc agcagagttt gtccctcctc 2820

cgcagccgcc ctctccccgc ctcccgtgac gtgtcccggg actccccgcc ccgggctggg 2880

ctgcgaagtt gcggatccgg tttggtcggg cgggtcggcg gctggaggaa atacacggag 2940

cctcaggggc tgcggcagca gcatggtggg gccggagaag gagcaggtat cgcacagcgt 3000

ggggctggga gtgagtgggg tccagaggcg gagccagctc cggggatccg ggtcagatgc 3060

tgcagtggct ggaccaggag ttcccagacc gagctgcgtg ggttcctggc tgagcccgag 3120

ggaggagttc ccagtgtgtc ccgtgggcgc gcggggttgt cttcgaatcc tgtgggtctg 3180

actcgactcc ctgccctgcc cttcttgttc cggttctcag agctggatcc ctaagatctt 3240

caggaagaag gtgtgcacga cgttcatagt ggacctctcc gatgatgcgg ggtgagcagt 3300

tcggggctgc tgacctggca gcgtaggttc gcgggccctg ccccactgca gacaggctcc 3360

gcccccatag actggactcg ccccactcaa cgcagacaag ggtgcccacc acagactgac 3420

cttcactgcc taccgcagga ggtcttggtg cacagacaca ccccacccac cacagaagtc 3480

tcagctcaac acactcagac cccgcccact aaggaccttc ccgccttcac ttgagacaga 3540

cccctccccc tgcccttgtt ttgggccacc ctgcttaggg cacgccccga attcttcatt 3600

cccctctagg tggggatcca ttctgtgtct ttgtgtctct gtctctttgt gtcatttgtc 3660

tctgcttcgg ctttctgtgt catcccggct cagcgagcat agccacacag ccctgccagc 3720

cttacatagc tctgcagctt tgggatagta attcccttct agggctgcgc ttgtcacctg 3780

ggcacagaac agctcctccc ttctggggcc gctgtgcaaa ctaaacaagg tcactctggg 3840

acatttctgg aactctgccc atcacctaat aacatctaat aaacatccgg gaatatctgc 3900

aactcccatg agaagaagag ggcaattgcc actgcaagct gtgctgtgct gtaggtggca 3960

ggggacagga gcaagcccta taggcttctt ggctgcatgc ttagtctccc tagcggggtc 4020

cagctttaac tcagttctca gggtagactt ttggccttgg ggcagtgaca cctgatttcc 4080

ttagtgtgac tttatcagga gcctgttgtc ccaccagtgt gggacaggaa aatgttgtat 4140

aaataaacag agatactggt ttactggtgg gtctggcaga gggtcacaga cagcgtgact 4200

aaagtcagtg ggacaagatg ggagctggtg ggctacaggt gtcattggaa agatggtgca 4260

gtgtggtgct ttgaaggtgg ctctgggcct tggaagtgct cggtcgaagt cctccctaag 4320

gacagcccag cgtggcttga aagtagagca gagttcaggc tgtcaagagt gttgtgagtg 4380

ggaaggttgg tggtagacct catgggctct cagaggccat cccgagtgtg gtttcctggg 4440

gctgcgtgag ggcgggtagg aattcagaaa ggtgagaaga gattgggacc atcaccaaga 4500

tgatgagccc aggagaagaa gcaggtttgg aaggggatgc tgagtgccgg tccttctgtg 4560

tacatgatgg gccttggaga cgggcagggg aggattggga ccctgctggt acttagcaga 4620

agagtgagat tcatactggg gtctgaggtc ccagctatag gtggagattg aatccatgga 4680

ggcaggtgag gaaaatacaa aggccccctg tctctctcgc cttcctcttc tttccttctt 4740

acacctctgg gcttagggtg taattcaggg gtagcacaca tacttagcat gtgttgggtt 4800

ccgttcccag gaccaaaaac ccaaacccaa gaaacctgag caccagaaag ccaccagggg 4860

ctgtagagat ggtcccgtgg ttagtaactc tggctgctgt tatagacaag cactcagtgc 4920

ccagcactgc ccaggcagct cacaaccacc tgtaactccc attccagtgg atctgacgcc 4980

ctcttctggc cttcacaggc actgcacacc tacggtacac agacacacat gcaggcaaat 5040

cctcacccac aaaattaaaa accaaaacac tcacccaaca cccccccccc acacacacac 5100

atctcttaac acctttaaga caagtgctag cggctgctga agtgtcttaa ctgtctctct 5160

cggcctctgg ggttgatggg ctagcgtgtc attgaggggt agccaggagg ctagcatgga 5220

tcctgggtgc tctgagggaa gcagagggaa aagaaagaca ccatgagagg tgcattacag 5280

ttttgaggag aggaaggacc aggccaaggt ttccagaagg ggtgcattcc atggtggcca 5340

gtggaatgtc tcaccttctg acagtgagat gacacttgaa tggagccatt gtgcctcaca 5400

gccacacgga gcaaaggtga caggaggcag gcgggcaagg gaaaggttgc cagcaccatg 5460

ggaccagaac ctactttctt ctgtgacagt gatgctcact gtccacattg agtacggtga 5520

ctagtatcag tgacagagcc ctgagcgatg aactgtgtga ctagaaacgg agtttttctt 5580

tgatttaatt ttgtgtgtgc tcctgagtgc aggtgcaaat gtgtgccctc gtgcatatag 5640

aggccagggg atggcctcag gaagtgttcc ccacgcgccg tgtgccttgt tttgtaagac 5700

agtgtttctc actagcgtgg gggtgacctg gctagccccg gggatccttc tgtcttcttc 5760

tcccctgcac tgggattgca acttgtttat gggtcctagg gataaactca agtccttctg 5820

tgctcaaggt gggcactttc tcccctgagc tccgcttaag tcattaggtt agggtctaag 5880

gagcccgtgt ggataacagg cccctggggc caggcgcttc ggaggccaga tgctccacaa 5940

cattccgtgc agaagttttg actaaaagat agagggatca agggggtccg agatgaggtc 6000

ctgatggata aaaggattaa gtggggtgaa acggaagcat gtcagggtcc cctggcatta 6060

gagaaggggg ttgtgtctct tataactctg gagggacacc aggccatctg tatgcacctg 6120

acgtgtgtcc tgtgaatgac cagtaccatg gcctgcctgc ctcacgacat taccttaggc 6180

cgggtgtgca ctggtgtgca ctggtgtgca ctggtgtgca cttgtcatca ggaggtggag 6240

caggggttca aggccagctt gggccacaaa gttagttttt gattggcttg gactctcaaa 6300

agacaagggg cggagggctg cagttatggc tcagtggtta gaagcactgg tcactcttgc 6360

agaggacctg ggttccgctc ccagcaccca tgggaggcct cacaatcatc tttaactcca 6420

gttccaggga attcaacgtc ttcttccagc ctccacaggc agcgtgcaca tgtatatgtg 6480

gtgtgctgat atacgggaag acaaatattc atacacataa aatataaaaa tgttgaagaa 6540

aaccaaaccc ccagggcttg ggtcactgct cagttactag aaagcttgtt taccacgcgc 6600

tcaacccagg gttctgtcct cagcaccaaa ataagccagg gctggtagtg cacacctgta 6660

attccagagc tcttgaggct gagaccagag gatcagaaat ttgaggccaa aaataaataa 6720

atcagaagaa ataatttgtg atcagcttta gctaatgtaa gtttgaagcc agcctgggct 6780

attgagaacc tagtcaaaaa accatctccc cgccgggtga tggtggcaca cacctttaat 6840

cccagcactc gggaggcaga gacgggtgga tttctgagtt cgaggccagc ctggtctaca 6900

gagtgagttc caggacagcc agggctacac agagaaaccc tgtctcgaaa aaccaaaaac 6960

aaaaaaacaa acaaaaaacc acctccccac cttcctccat cagtggttca gctaacttct 7020

gaaactacag gtgagcccca ctatttgaat tccttccaga atcagggtca aggagaatta 7080

gctatgttgt ccccaagtcc ctgctcatta ctctggggaa ctaaggtgac agtcaccgga 7140

gcccaaggga cgattgtacg tttatgattt ttaaatattc cttttgttat ttttgacatt 7200

tatatttctt ttacctgtat aggaattttt ttcctgtgtg tgtttgtgta tcacacgcat 7260

gtatggtgtc cgagaaggcc tggagagagc gttggatccc ccaggtctgg agttacagat 7320

ggttgtgagc caccatgtgg gtgctgggaa tttatttcgg gccttctgtg tgcactttta 7380

acactgagcc atctctccag cccactgtgt cctatttaga gtggaaaacc ttctatctaa 7440

ggagacaaga aatggaagcc atcatctgtt ctgtttctca gaaccagaag gaatggagtt 7500

gggaaggaag gattgtcctg gggtacagtg aacagcgaat aaggcgattt taccttctag 7560

cttctgcccg gggccggctg ctcgctgggg cctggggcca ggcagagact ggctggcagg 7620

ccagcgccca gccgagggcc ctcctcacag ttatggagct ctgaaagagg ctgggctttt 7680

ctcctgtgaa gttctgagtt atttaaagct ctgcctggag ttcatgcagc cggggctggt 7740

tgctatccta gcttcactgt gatacactgt gattttcttg gctcctctca gttccttggg 7800

gcttttacca tctttgtatc tttggaggag gaaatttagt ttcagagagg ggaagatgcc 7860

ccaaaccggc ctcaacagta gccaaactct tttgaatctt tttttttttt tttttttaat 7920

tttgttttgt tgagactgag tttctctgta gccccggcta tcctagaatt ctctgcatat 7980

caggctgacc tcaaatcagg attatagatg tgtgccacca tgcctgccct tttcttcctt 8040

ccttcctttc ttccttcctt cctttcttcc tttctttctt tctttttttt tttttaatac 8100

ttattgattt attatatgta agtgcactgt agctgtcttc agacacccca gaagagggca 8160

tcagatctca ttatggatgg ttgtgagcca ccatgtggtt gctgggattt gaactcagga 8220

tcttcagcaa gggcattcag tgcccttaac tgctgagcca tctctccagc cctctttctt 8280

tctttctttc tttctttctt tctttctttc tttctttctt tctttctttc tttctttctt 8340

tctttctttc tttctttctt tctttctttc tttctttctt ccttccttcc ttccttcctt 8400

ccttccttcc ttccttcctt cctttcttct tttcttcttt ctttattact attatctttt 8460

ggttgaatca ttctttaaag ttgctttatc atagtgtatg tccttctctg gggcttgggg 8520

cagcaagggc acctgctgat ggcctcgtgc tattcttgat tctgaccccg caggctactc 8580

tccctgtcct ggcttatttg cggtgaagct tggctataag ggctccttac ctcagttttt 8640

tctccagaca gtcagagcca gtgctgggtt catggtacac tgtctctagg gatacagggt 8700

cccatgtgga caggatggat tcagtgggga cacccagggt tcatactcta ccctcatgcc 8760

cccagaggga ccttgtgcca gtgtgggcaa cctcgggatg cccacccttc tgtggctgtg 8820

gaggatgcct ttggggcagc cgtagttacc gagtggaaca gtgatgagca caccacggag 8880

aagcccacag atgcctacgg ggacctggac ttcacgtact ctggccgaaa gcacagcaac 8940

gtgaggcctg catgtccgag gcgccaggct gggtggggct ggggcaacgc caatgtgtct 9000

ccccattggt ccaccctgtc tttgcgtgcc tctgtcttaa gagtctgtcc ctctctctct 9060

ctctaggtcc ttgcagcctc ccgttctgtc tgtctgcttc tctctaggtc tctgtcctct 9120

ctctaggcca ctatctgcgt ttgtctggat ctgtctcctg gtgtctcagt ctctgtcccc 9180

catgtgtctc ttcctcctgt gtccacagtt tctccggctg tctgaccgca cggatccggc 9240

cactgtgtac agtctcgtta cccgctcttg gggctttcgt gccccaaact tggtggtgtc 9300

ggtgctgggg gggtcggggg gccccgtgct ccagacctgg ctgcaggacc ttctgcgtcg 9360

cgggctggtg agagccgccc agagcacagg tgaccacggg ggttgggaac tgtctgtcct 9420

gggccgcgtc cccactgttg ccctggttgg aggacattgc tcccctttcc atgtctttgc 9480

tcactgtttc ccactctgtc catccctctg tcctacatag gggcctggat cgtcactggg 9540

ggactgcaca caggcattgg ccggcacgtg ggtgtggctg taaggaccca ccagacagcc 9600

agcactggga gcagcaaggt ggtggccatg ggtgtggccc cctggggagt ggtccggaac 9660

agagacatgc tgatcaaccc caaggtggga atggggttca gagaagaggc tctggcctgg 9720

agtgctggtc tgagggtgga gggcagagag taatcctgaa ttctgaggga ggaggctggg 9780

aaggactcct gggtttaaga aagtctgggg aagatacttg gatctgaggg aggaggccaa 9840

ggtctggtcc tcgggtctgt ggggggagtg tcacatccct tctaaaagac ttctctatct 9900

ggcattgtcc ccagggctca ttccctgcaa ggtaccggtg gcgtggtgac cctgaggatg 9960

gtgttgagtt ccccctggac tataactatt ctgctttctt cttggtggat gatggcacct 10020

atggccgcct gggtggtgag aaccgcttcc gccttcggtt tgagtcctat gtggctcagc 10080

agaagaccgg agtgggaggt gagtgggatt tgtgtttcga agccagcaca caggacctca 10140

cagccaggtg tggtcaagac tgggcaactt cagttaagag tcttgtccct gctggggatg 10200

gtggcacaga tctttaatcc cagcactggg aggcagaggt aggcagacct ctgtgaattt 10260

gaggccaacc tgctctacac aggatagcca gagttacata aaaagactct ttctcaaaag 10320

taagttaaag cagggcgtgg tggcatacgc ctttaatccc agcactcggg aggaagaggc 10380

aggtgaattt ctgagttcga ggccagcctg gtctacaaag tgagttccag gacagccagg 10440

gctatacaga gaaaccctgt ctctaaaaaa aaccacacac acacacacac acacacacac 10500

acacacacac acacacacac acacacacac aaagtaagtt aaaaagaaat acaataaagg 10560

gagtgtaaag agatagctca gtagttaaaa tcacttgctg ctcttataaa ggacctgggg 10620

tccatctgat aactccagct ccaggggatc caggctccct cttctggcct tcctaggcat 10680

ggcactcaca ggcacataca cacagagaca catatacata cagaagtttg tttttgnnnn 10740

nnnnnnnnnn nnnnnngcat taaagtgtgt gccaccactg cccagccatt tttttttttt 10800

tttttttttt tttttttttt ggtttggggg gcacactttt tttttttttt tttttttttt 10860

taccaccggc aaaaaaaaac ttttattatt ctgggtagcc tcaccctttt ttgccagccg 10920

cctttggagc aggggctttc tgggctgggg tcttctgacc cttttgacct ttaacaggag 10980

gggccacctt ttggcctgca gccttttgag caggagcctt ttggcctgca gccttcttgc 11040

ctggtcctgg ggccttcttg gctgggactt tagctttaac agcagcagca gctgcagccg 11100

caatggcagc tttggccaca gctgctttct taggaaaagc tttcaggata gcagctcttt 11160

gaagtttctt tacttcagtc ttgattattc tgttcctcat tttctttgcc ttcatgacct 11220

tgaaacgatc aaagtctgtc atcttggctt tcctttctct ggcatcaatt ttcttggccc 11280

atcttgtggc tgcccatttc gtattgatat ccgccttctc ccaagctttt cggacatact 11340

tctggcgggc actgtgtggg aacttgagga tgaagtcagt gagctgcatg catttgaagg 11400

gcatggcctg cctcctcacc cgagtacagg gtccatccac taaagccctg ttctgatcaa 11460

tgacatctac aattgcgacc agctttccag catggggccc gaaggaaatg taggccaccc 11520

ggccaacctc cacgtaacgc ctgaacacca tgttggcggc gctcagcgag aagcgtgggg 11580

cacactttta atctcagtga ttggcaggca gaggcaggca gagcccaatg agttccaggt 11640

ctacacaggg agttctagga caggcagagc tatatctaat gagaccctgc ctcaaaaaaa 11700

aaaaaaaaaa aagtgatgga aagtgaatta tcttttcctt atccccgccc cccacactgg 11760

ggtcccctgt gtcagacagt tccatgcatg cgctttccat atacaatctt tgtcgccttt 11820

tctttttctc tcttctcttc tccttccatc ccttcctctt ggcaatcctg ctgcctcggc 11880

ctcccaagtg ctgggatttc tactgcatgg catttgcttc tctttttctt gtggtgttag 11940

acacaatcca tttcatttcc cttgtgtaga atcttcatgc acaggcttct ttaccttctc 12000

tttcggccat gctggcagct gaaccaggcc tgtgtgcaca ccaggcgagt gctggaccat 12060

gtgtgctaca tcgccagcat tcccagctct tgggtggaat tttcctaggt gacaaatgct 12120

agggagtcat acatttgaaa tcatctggca cgagtaaccg tggtgaatgt gtgtgtttgt 12180

gtgttgcctt catgtgcaga tgtgagttct ctccaggtct ccatctctcg tggcctgact 12240

cgcagctagg cctccatagt tactgtccta ttcctgtgat gagatgccat gtccgaggta 12300

acacctacaa aagagagcat ttaactgggg actggctggc tcacaggttc agagctgtag 12360

tctctggtga cacacaggca ggcttgatgc tgtagaagtg gctgctactt ctacatctgg 12420

attcacagca tcagggagag ggtgacgctg ggcttgatgt gatggtttga aacctgacaa 12480

cccacttcct gtgacacact tcctccaaca agaccacacc tcctgattct tcttaaacat 12540

tgccactccc tgatgactaa gtattcaaat atatgagcca gtgggactat ccttgttcaa 12600

accagagcaa taactttcta ttactttgca gcagtttcgt ttttattaag aatacatgat 12660

tgtggggcca gcaagatgac tcagctgagg gtacttgctg ctaagctcaa cgaactgaat 12720

cgaatctctg aaacccacga ggcgcaaaga gagaatcaat ctctgcaagt tgtcctctga 12780

cttagacaca cacacacaca cacacacaca cacacacaca cacactgtgg ctcccacata 12840

cacgcgtgaa ataaattaat ctaaaagaga gaggaaataa ttttaggtga atgtggggac 12900

acctgggagg ctggagcagg aaagccatga agcagcagac tgggctacag agtgagaccc 12960

tgtctcctag aaacacaaac cagcaacagt agcaacaacc acaaaagcac acatttaaat 13020

ttaaccatct tgcttggtgc tgttttgtct tcagcccaga gctccaggat gctgtgatgc 13080

cgcccagccc agcacagtct attttattat tttactgagg gtggaaccaa agtgccattc 13140

cctcggggct ttgactattg gactttgtta atggttttat ggtgctaaga ttgcccatct 13200

acttgtatcc ccttccttga actctcactt cactggcctt tggagtgctt agccaatcat 13260

aagactgact tgagagccat gagtccaggt cccaagctct gctcccgtct gaagctaagg 13320

tgaccgccct ggacttctgg acctggtgtt gtctgtcatc cccaacattc tgtaaaagga 13380

tcaaagttca cagtcatcct ctgcttcata atgagtcgga ggccagtttg ggatgtatga 13440

ggctttgctt aagaagtatg gggtccgggg tgctggggcc tgacttgaca gatatgtgct 13500

tcttgtgcaa gagtgaggac cttagagttc acattgtgtt ggctgcagca gctcacatct 13560

gcagtccccg ctccccttgg tgaggtaggg gacagaggct gctagcttgg tgtatgctaa 13620

acaagagacc ctgcctcaaa tcaaggtggg aggcgaggag caacacctga gcttatgaca 13680

cacatgacac gtgcacacct ctattcatgc gcatgatcac acgtgtgcat acacacacac 13740

acgccataca acacacacac accccatata acaccaacac acacacacac acacattaat 13800

aataaaaccc agaaagtctt cagcaattaa tggtacctgc tgccaagcct aatcacgagt 13860

tctatcctca ggacccacag agccgaaaga gaaaacagac tcccacaaat tgtcctctga 13920

cctccacaca tttgtgaaca ccatcctccc aacactatat acatgaataa taataataaa 13980

aaaattcaaa gaagaaggac aattcccatg actcttggcc aatgttagga caaggccaaa 14040

tgtctgcccc aagagattcc tgggaaatgt agttttttcc catccccagc aaaggctggt 14100

agaatgtgac cgagtttccc ttccccgctt tctctcaggg actggaattg acatcccagt 14160

gctccttctt ctcatcgatg gagatgagaa gatgttaaag gtatctggag ctgcatccct 14220

ggttttgagg tgggaggaga gctgaaggcc aatactgggg caggcaacag tcttagggat 14280

acggggaccg gagggctgaa ccctgactgg ttctcttctt gcttctgaag cggatagagg 14340

atgccaccca agctcagctc ccctgcctct tggtggcggg ctctgggggt gctgcagact 14400

gtctggtgga gaccctggaa gatactctgg ccccagggag tgggggactt aggcgaggcg 14460

aagcccggga tcggattagg cgatacttcc ctaaaggaga ccctgaggtt cttcaggccc 14520

aggtatgccg ttgtggaacc atgatgggaa gggctgggca ctctggctgg caggtcctaa 14580

ggataggtgg aaccacaggg ctgagctggg gactagagtt ctgcattctg caggtagaga 14640

ggatcatgac ccgaaaggag ttgctgacgg tctattcatc agaagacggc tccgaggagt 14700

ttgagactat cgttttgagg gctcttgtga aaggtaggat gggccttcca cattggcctt 14760

gtcttcacct cagcggtggc tgactctgtc tccaccctca atcctctaag cctaagacca 14820

cacctttttt gtgtctccta tgtctttcct gtttcctagg tttctgctag atgttgttgg 14880

tcagttttcc ctagacgttc tcattgtttt caaccattta cttccctgtt ggatccaccc 14940

atccatattc cattaatccc ttcctatttt catctactca tcattccatt tgtccttcat 15000

tcttccatcc attttccgtc ttttcatcca acctcctacc tatttacagc ccacttatcc 15060

atccattccc ctatccatct atatatccac gtatcaatcc tctatccatc atccacccac 15120

ccacttgtcc atccatccac tcacccagct acctatccac ttgtccatcc atccatctac 15180

ttacatatct gtccatccat ccacccatcc acctgtccat ccatccaccc atccacttgt 15240

ccatccatcc acccatccac ttgtccatcc atccactcat ctatgccatc catccatcca 15300

tccatccatn nnnnnnnnnn nnnnnnnnnn nnnnnnnnnn nnnnnnnnnn nnnnnnnnnt 15360

ccacccatcc acttgtacat tcacccatcc acttgtccat tcatctatcc acccacccac 15420

ccacccaccc atccacttgg caatccattc atccatccac ttacatatct atccatccat 15480

ccagtgacct atctatctat tcatctgtct tagtcagggt ttctattcct gcacaaacat 15540

catggccaag aagcaagttg gggaggaaag ggtttattca gcttacattt ccatgttgct 15600

gatcatcacc aaggaagtca ggactggaac tcaagcaggt cagggagcag gagctgatgc 15660

agaggccatg gagggatgtt ctttactggc ttgcttcccc tggcttgctc agcctgttct 15720

cttatagaac caagattacc agcccagaga gggtcccacc cacaaggggc ctttccctct 15780

tgatcactaa ttgagaaaat gccttacagt tggatctcat ggaggcattt ccccaactga 15840

agctcctttc tctgtgataa ctccagctgt gtcaagttga cacaaaacta gccagtacac 15900

catccataca tctagccatg cattaagtca ttctcctagc caccccctca cttgtccatc 15960

tataaattca cccgtacgcc cacctatcca tttatccacc ttaattattc accacccacc 16020

agttctttaa ctattcgttg acacatccat taacaattca atccatgcat gcgccttccc 16080

atccacctta aatctcaacg ttctgcgaga tgtgcatgtc ggttgtaagc ttggctgacg 16140

aggctcacat gaccactgcc accacctcac gctcagaggg aagagaggga ttttgcagta 16200

gtcccccagg tagcagcttt gtgtggctga ggatttaagt ctgtgcaatg cggtctctag 16260

catgggatat ttctgaggag acaaaagaag cctggaatta tgtacttggt agaccatgct 16320

ggagtcaaga gcagcaaggg atgcaggccc caccagctga agagctggaa gccaggactt 16380

tcccctggga ggcagggatg ggggcaccaa gaccagactg tgttcaggag gcccctgtgg 16440

agcagacagg gtggtctggc gaggagagat gagaccatga gcagatgaag agactgaagt 16500

gacacatgct ctgagatgtg ctacggccag tgggactgga tgcgaggcat ggcacagtgg 16560

cttaggggct tatgagctat gggacatgag aggtggatcc tgggattttt gccttgctga 16620

gtagactgac ttcaagaaaa cgtgtgtggg acacggtgag caggaaggtt aagggcagga 16680

ggctctgggg atgagggatg ccaagttaag gcacagggaa ttttgttttt gaggggacat 16740

atgtccaaac tcttcatcca ctcaccaatc ccttcgtcac caagcccttc acccatccac 16800

ccacggacaa gcatgatctt accaaccgtt tgctttcttt ctctgaccct ttggaagaag 16860

taccagaaaa gcacagaatg aaaatgtgcc tcacgcccga tacatttttg cttctcaaaa 16920

taaaatggga aattgtatga tcctgggcat gttcatgacc cctctttgga tatctgttgg 16980

cccagacatt aaccttaggg actgaggagc aagctgaaga ttgccaccat ttggaatcac 17040

ttctcattgt tagcagttga cattgctgtc aagagcgtca ctctgactcc agttctatat 17100

atgaagaaac agcaaaatgg acatcaccct ctgtctctac ccttcccctg agccccacct 17160

ctcccttttg tcccttcagc ctgtgggagc tctgaggcct cagcctacct ggatgagctg 17220

cgtttggctg tggcttggaa ccgcgtggac atcgcccaaa gtgaactttt ccgtggggac 17280

atccaatggc gggtgaggag tcaggacata gacccacttc tctacctggc tgacctcttg 17340

acacctggtc ctgccttaca ttatgatctg tctggccttc ccttaattgg ccactcatct 17400

ctggttgttc ctctgtccct gacttgttcc ttgtctccct gtcaacccac ccatccatca 17460

tccacagtct gtccatccac tcatccatcc atccatccat ccatccaccc atccatctac 17520

ctacccatcc atctctagcc atccacccat ccatctctct agccatccat gcaccaatat 17580

tcacttactc aaacattctt accctctttt cacccaccag cctactccat atatccatcc 17640

tccaatgaat cctttcatca atccacccac caacccatcc acccacttat ttatctgtcc 17700

atccactatt gaccagtcag tcagtcagtc agtcagtctg tctgtctgtc tatcatgctt 17760

cacttgtggc ctctgtctct tgccctccat ttctgagttc accttgcatg tctgcctcag 17820

gtcatccctg tcccatccct ttccctcaca cagtccttcc acctggaggc atctctcatg 17880

gatgctctgc tgaatgaccg gcctgaattt gtgcgcctgc tcatctccca tggccttagc 17940

ctgggacact tcctgacccc cgtgcgtctg gcccagctgt acagcgcggt gtcccctaac 18000

tcactgatcc gcaatcttct ggaccaggcg tcccatgcta gtagcagcaa atcccctcct 18060

gtaaatggaa ctgtgggagc tccggcctcc caatgtgggg caagtgctga ggactctgtt 18120

gggagaaacg tgtgcaccac ggtaccctgc caggaacacc cgagactcct atctgggcca 18180

ggaccacaga gagaatgtaa ggcccatcaa ggctgaggat gcatggcggg gctgcatgtg 18240

gagggggaca gggctttggg aagaggggat agtaagatgg ggaaaggtat tgtttgagag 18300

agggtgtggt aagcccgggg cagtgggtgg ggctagtttc ttgctaggct tcaacgtgct 18360

ttcaggactc tctgcttatg gactgggcca acaagcaacc atcaacggat gcaagctttg 18420

aacaagcccc ctggagtgac ctgcttattt gggctctgtt gctgaaccgg gcccagatgg 18480

ctatctattt ctgggagaag gtaaatgtgc ctcctcattt tggacctcac cttggtacac 18540

tgtgcttaga gacaacaagg ccccttagac ggctctgacc tcagtcagac cttagccaac 18600

ttgcactcca ttctctgtct tccttttgtg gtttgttttg aggcagggtc acagatagcc 18660

caggctggcc ttgaacttga tatcagccca ggatgatctt ggacatgggc tgctgtcctt 18720

ggttcatgcc atgctgagaa tcagacccaa ggttttgtgc atgctgggca agcactctac 18780

ccagtgggtg ctgccatccc cacccctagt tttgatttgc tgagacaggg tcttcctgtg 18840

taacccagac tggccaggac cctgtgattc tcctgcctca tgaatgctgc ctgtgacaca 18900

ccccaccccc acggcctaca cacacacaca cacacacaca cacacacaca cacacacaca 18960

ctctgaatgt tttaaagatg tatgccatga gccgggtgtg gtggcgcaca cctttaatct 19020

cagcacttgg gaggctgagg caggtgaatt tctgagtttg aggacagtct ggtctacaca 19080

gtgagttcca ggacagccag ggctacacag agaaaccctg tctggaaaaa acaaaaaaag 19140

atgtatacga tgatgacatt atcttctcaa actgaattca gtgcccagga aaagagcgat 19200

gcacagtgct ccgggccccc actcggcttt ctcacgccgt tgctggtagc gaccctgtct 19260

attggccgtg tgcatttgtt tgggtgaacc cttggcctta atgactctat taacttctga 19320

tcctccacaa tcacaagatt tctctccttt ccttccccag atgtctaact tcaccatgtc 19380

ctctttctct gtctcttcag ggctccaact cagtggcctc tgctcttggg gcttgtcttt 19440

tactccgagt gatggctcgc ctagagtcgg aggctgagga ggccgcgcgg cggaaggatc 19500

ttgctgccac gtttgaaagc atgagcgtgg gtgcgtgggg cagagtgcct ggggtgtggg 19560

ggtgcacaag gcacctcccg cgtccttcca tcctcatctg tatccttgtc cccagacctc 19620

tttggagagt gttaccacaa cagtgaagag cgagcagccc gccttcttct gcgccgctgt 19680

cccctctggg gagaggccac ctgcctccag cttgccatgc aggctgatgc ccgagcgttc 19740

tttgcccagg atggagtcca ggtgagcctg tgactcagac agccgtgagc cccacccccg 19800

ggagtccact tatgcctaga acaattacac ttaacctctg agcacggaac ggccgtttca 19860

caggggaata tcatatattt gcattaagat tcataacagc aaatcccgtg gtggcggcac 19920

acgcctttaa tcccagcatt tacaggcaga gaggcaggtg aatctctgag tttggaggcc 19980

agcctggtct atagagaaag ttccaggata gccagagaca cacagagaaa ccttaaaaaa 20040

aaaaaaaaag tttttttttt tttttttttt tttgtaacag caaaattaca gttaaaaagt 20100

agcaatggga attttatagg gacttttcta ggtcatcaca gcattaagac cattgagaac 20160

cactgcctta gaaaatgtta cttcataaga tgaccaatca tctataactt ttcgaccctc 20220

cctaagtagt gttgttgcta ccaggaagta ccatttctca cacaagaagt tcctcatttt 20280

cacaggggag ttcctgtcct cctaaaggaa agaccacttc ttcaacagga agctctttcc 20340

attttttttt tttttttccc aagacagggt ttctctgtgt agccctggct gtcctggaac 20400

tcactttgta gaccaggctg gcctcgaact cagaaatccg cctgcctctg cctcccaagt 20460

gctgggatta aaggcatgcg tcatcactgc ccggctaatt ttttttttaa gatttattta 20520

ttattataca taagtacact gtagctgtct tcagatgcac cagaagaggg cgtcagatcc 20580

gtcagatctc attaggggtg gttgtgagcc accatgtggc tgctgggatt tgaacttagg 20640

acctttggaa gagcagtcag tgctcttacc cactgagcca tctcactagc cctctttcta 20700

cttttttttt ttttttaatg tgcattggtg ttttgcctgc atgtgagagt gtgagatcac 20760

ctggatctgg agtggcagac agttgtgagt gggcagctct gctgatttcc ctgtgtcgat 20820

ctgtttttag tcaatcttgt atgtgtgaga aggagaaaat actcgccctt ttgggttaag 20880

tgatttggtc aatgcagagt gcttggcaca gcacctgccc tatgatgtca ataaatgttg 20940

gctgctctgc gcctgcagga atgaatggtt cactggttaa gggcacaggc tgctcctgca 21000

gagaaagttt agattcctac cgcccataag gcagctcaca gccatctgta actccttcta 21060

tgggctctgg tgccctcttc ttacctatct gggcaccagg cacgccatgg cgttaatccc 21120

acggggtgag aataggatta ctaccacagt ttttgagctg aatttggaac acttttgttt 21180

tatagatctt ttaagcacaa taatttaaac ttaaaacata atcttagccc ccacaatggt 21240

gcacagacat gccggcaggc gaaacaccca cacacagtga aagaaaaatt ggctgtttcg 21300

ttttcctggc tgaacctgag gaggctgcct ctgacgactg tgtcccatgc tctaacagtt 21360

gagaccccat atggacactt gctctgtgcc ctggtggcag actccagccc ttgtcagggt 21420

ctctaatgtt ctcacattca acagtctctt ctgacacaga agtggtgggg ggagatggac 21480

agcacaactc ccatctgggc cctgcttctc gccttcttct gcccgcctct catctacacc 21540

aaccttatcg tcttcaggtc agtgccttgg ctccaggctc tgtccctctc tcctgctcat 21600

ttctctctgc cctcttactt agccacctgg catgtctctt ccctgagaag ggcactcttt 21660

cccttctctg gagggaatgt tctggaaggt gggggttgtt gtcctgcctt tgcgttatct 21720

ctctatgcga tttctctact ttttaggcca tggctctgga ggagggcaac tcccttatct 21780

ctctgcactg tttgtggtaa actaagtttg ggtctttctc aagaacccct ctatttctta 21840

ctctgtgtcc tgcccctcac ggcaggaagt cagaggagga gcccacacag aaggatcttg 21900

actttgatat ggacagcagc atcaatggag caggtcctcc tgggtgagtg acagtccagc 21960

attgtgggag gaggggggag ggcgccatca tggacagttt caggatcttg ccaactccca 22020

cttccatcag gcactgtagc aagtcctgaa ctaggggagc aatgggcttt taggagttgc 22080

tggtctcact gaaccacaga agatgccgag gtaagttcta gaaggccatg ctcaaggact 22140

gacttaaaat ttggtccttg gagccaacga gatggctctg caggtaaatc tactcaagtg 22200

gaaggacctg agttcaagag agaaacaatt ctttttctag gatataacta cactgtagtt 22260

gtcttcagac acacactaga agagggcatc ggatcccatt acagatggtt gtgagccacc 22320

atgtggttgc tgggaattga acccaggacc tctggaagca cagtcagtgc tcttaactgc 22380

tgggcctctc tccagccctg gagaaacaat tcttaaatgc tcctgacttc cacctttctt 22440

agttcaccca ttggagtgca gagcgccagg ggcagaagga tgatgtcatc acaagaactt 22500

agtttgggtt gggcgacctg cgctcccttg ctgttggtta cttagtattt attcactttt 22560

ctattatgtt tctgtccctg ggctcttgcc cctgtcacta tgttgcagtc cccatggaga 22620

tcttttaaag accaggctca atacgtatag ttgctaagta tttgtctaag ctcctcatag 22680

ccctaggttc catcccaggc caaacaacac aaaacctaca ggaaacctcg gggagtcagc 22740

cagttaggtg cctagcgcac aagcataaag agctgagttc agactcccag cctctgtaat 22800

aaaatctaag gtgtggcaac gcatgcctgc actagtgctg ctagacagag aaggaagggt 22860

ccctggggtg ttgtgggcca gtcgggatag ccgaatcagc aagtcctgtg tttaaaaata 22920

agagtgaaaa cgactgaagg agacatcaaa ccccaacctc tcacgtctta gtctggctga 22980

taaaacgatt ttgaaatgga ctcagaagga agcttgggac ccttttatta gatgctgaga 23040

gaaaaggaac agagcaggcg actatgaaga ggaagggagg aagccactcc ttttgctggg 23100

gaggttagaa aacagtagca tgctggtgag aagggacggg tggcccagag caccaaggaa 23160

attaagtcta ggctttggga agaatccaaa acaaacaaag acaaagaaac ggagaaggaa 23220

agattgtttt tctgagttag gagtcagaaa atcgggcagg ttgcatgtgg aggagtagga 23280

ttgcggtctc cagggaaaca aaagtaaact gtatcgtatt gtatcattta ggagataatt 23340

aatctttccc tctccttcaa gtgactcaga ctccctgggg cagagaaaga ctctgggaca 23400

agtgactggc aagctgtgct ggtttaaccc tttagacact ttaaaagatt ttatttttaa 23460

ttatgtgtgt acatgtggat ttgtgtgttg tagggtctat gcatgtggtt gccctctgag 23520

accaggactg ggaattgtca cacacacaca cacacacaca cacacacaca cacacacaca 23580

cacacacacc atgacaaacc aaccaaccaa cccccacagc tgccaaacgg aataagagac 23640

agcttattct cgagccagat gtgagtgacc taagtgtgga aacacggggc taagttagtt 23700

ctatgtccca acgtggaaac tgtttcatgg agtttttata gtaacagaac aaagaaagtt 23760

ataaatcaaa gcacttaaaa atacagtggt agaaatatta agtgagcaga cagggcaggc 23820

aggagaatcg cagccttggg ctcagatcct atcttactgc attcttaggt gtctttaggg 23880

ttttgtgaca ttagtacatt gcaaaaattt gtatctgttt atcaagatgt tagctatgag 23940

actgaggtag gctggaggtt aaagagagct caaaagaaaa taaactatct gatagtaaag 24000

atagctcaaa agaaattaga ctatctgata ctaacaacca ggttctgcag tctctgaagt 24060

ctttatcagt tgatcagcct ctgcagacag tgtctttcta ggaattccca gtgacagagt 24120

caaacccgat ccagtgctca gaactgctgg atcaattctt cagcccctta aagtttttct 24180

aaattagatt ttattttgtt atttgtacat tgttttttga gggtgtgtat gctgcccgca 24240

agggtgtgga gtccagagga caagttgtag aagccggttc cctcctttca ccttggcgac 24300

caaggtcatg tggcagacgc ctgtgtttgc tgagtctcct ccttcctggg tcataagagg 24360

aggcaacgat gctatgtgat gtttggagta gtttagaatg tcctgtctcc atgcaaggcc 24420

agatgaagat cgttgggagc acaagggtcc tgacacccac agatcatcag ggaagctagg 24480

aatgttatac acacgggggc cctcttctca gtggaggaga gaaaattcct gtttttctgc 24540

ccagatgcct gggaatggat tttgtaaata acctggtgac ctttctgctc tctgtggagg 24600

cagacctcac ccaaatcccc cagactgcct atcccagact ctctcttcct agaccattgc 24660

ccatgctttg ggggagaggt cgaatgtact tcatggccac tgacctctat gctattagtc 24720

agagaccaca ctacattcta ggccttttag ctataggacc tggcctcctg gtcacatgta 24780

tttcataaaa gcatttctag gtgtcacaca gtaagcttac tgtgcacctg gaattgaact 24840

taggaactga ttgttgcctg gaaacctttt cccagattgg actgtgtttt tcttttgttt 24900

gtttgttctg tttttgttgt tgttgtgttt ttttggtttt tcgagacagg gtttctctgt 24960

gtagccctgg ctgtcctgga actcacttag tagaccaggt tggcctcgaa ctcagaaatc 25020

tgcctgcctc ccaagtgcta ggattaaaga tgtgcgccac cactgcccgg ctatggactg 25080

tgttttaaat atgctgacag tggccctagc attaaactct tcgcatcaac aatcagccca 25140

cactggcttt tcaatctcat cagtttgcgg gtcacggcct gcatggacac ctaccgggtc 25200

ccccggcttt tcaatctcat cagtttgcgg gtcacggcct gcatggacac ctaccgggtc 25260

cccctcacac gcccccacct tttcatgtgg tttaagatct aaagcactcc aaggctcctg 25320

caacattctg catgtttctg gtcagtttta aagacagcta ttgtgctcag tggtttgttg 25380

ttgttgtgtg gattgttttt tgtttgtttt gcataaggtt tctttctata tccttggatg 25440

gcttggagct gactaggtag acagcactgc ctcgccggga tcctttgcct ctgctgggac 25500

aaaccatgct gggtttatgc tcagttttgt ttctttcttt gatgtttgtt gtgtttcttc 25560

acaaagctca ggctagcatc aaacacctag caatcctcct gcctcatctc tcgaagtgta 25620

atgaatcctc attccatgtt ttatgttcct tcttccctca ctctatgtat caggctggcc 25680

tgaaacttga ggcagttctc ctgcctcaat ctcaaacgtg ggattgactg ggtcttcagg 25740

ttcccagagt ccaaagtggg acagtgtaga agttagactg caagatgggc agccagtcca 25800

ggctcattgg tccctgaagc tcgctgtctt gcaggactgt agaaccctct gcaaaggtgg 25860

cccttgagag gcggcagcgg cgacggccag gacgtgccct ctgctgtggc aagttctcca 25920

agcgctggtc ggacttctgg ggtgccccag tgactgcctt cctgggtaat gtggtcagtt 25980

acctgctgtt cctgctgctg tttgcccacg tgttgctggt ggatttccag cccacaaagc 26040

caagtgtctc cgagctgctg ctgtacttct gggccttcac gttgctgtgc gaggagctgc 26100

gccagggcct gggtggtggc tggggtagcc tggccagtgg gggacgtggt ccggacagag 26160

ctcccctgcg ccatcggctg cacctctacc tctcagatac ctggaaccaa tgtgacctgc 26220

tggcactcac ctgcttcctg ctgggcgtag gctgccggtg agtgagtaag agactggcag 26280

ggcccctgcc tagaatcccc cagtgagagg atgtggctat ggctcagtgg tggagtaccc 26340

tgggtgatgg agagtcacct acgtccccct ttactgaagc gatcctcagc tggcatacca 26400

agaggtagat ttcctccagt ccatgtgcgt ttgaaacaga gtaacttggt atgaataatt 26460

tatccaaaca gaagggagat aagtggactt agcaaactaa tatttctgct tgtcccagcc 26520

tttatttagt gttatgtgtt gtctgagaag acagggccac ggccctgccg attgcaggaa 26580

agccactgtc aagggtatat ttgatacatt ttatcaagca cccttttcca agggctgaca 26640

aatcattgca tctagggagg aagaaacact gtcacatcca gtataattgg cttgtcctcc 26700

tttaagcccg tgccctccag atccacactt aggttattgc aaaacaaaaa acaaaaccga 26760

ataaaaccaa gtcacgtccc tggtgtaaag tgggtggggt caaaggccgg gtggatagtt 26820

tcatccgcgg atttctgtct aaatagctgg ccccattgtc tgcctgcctg agagcggtag 26880

ctgtgagcgc ccccatgagg acatgctagg caatacctgg tgctttgtaa cctcaagctg 26940

tcaatcaaat cagacttaca acctctgtca gtctgcagcc tgtaaatgtt ttcatttatg 27000

cacccctgac cactccatat tttaggggat ccctcccctt ttgtggggaa acagggtctc 27060

actctgtagc cctggcaggc ctgtctggaa ttcccagaga tccttcttcc ctgacctccc 27120

ccgaacttgg ataacaggcg cgcggcatgg cacattctaa acacgattct tagaaggcca 27180

tccatccata ctcagtccag agaaagctga tcagagggtc cctgcctctg ccctgtgaga 27240

cctcagcttc gtctgaggag acaccccgga tctgagctgt gttctctccc ccaactcctc 27300

aggctgaccc ctgggctctt tgacttggga cgcaccgtcc tctgccttga cttcatgatc 27360

ttcacactgc gcctgctgca catcttcacg gtgaacaagc agctggggcc caagattgtc 27420

atagtgagca aaatggtgag tctgaggagg aggggcagag caaggtggga gagaatggga 27480

cctggagtgc aggggggtgg agccagcggt gaagggcaga atctcgaagg accacagagg 27540

acatggggag ggggttggnn nnnnnnnnnn nnnnnnnnnn nnnnnnnnnn nnnnnnnnnn 27600

nnnnnnnnnn nnnnnnnnnn nnnnnnnnnn nnnnnnnnnn nnnnnnnnnn nnnnnnnnnn 27660

nnnnnnnnnn nnnnnnnnnn nnnnnnnnnn nnnnnnnnnn nnnnnnnnnn nnnnnnnnnn 27720

nnnnnnnnnn nnnatgctgt gtctaagaca gggagagtag ccagctggag taggcgttct 27780

gggaaggggc ggagccaggg aggaaagggc tgggtcattt caccccacat cttcctctgc 27840

ttctagatga aggatgtatt cttcttcctc ttcttcctct gcgtgtggct tgtagcttat 27900

ggggtggcca cagaggggat cctgaggccc caggaccgca gtctaccgag tatcctgcgc 27960

agggtcttct accgaccgta tctgcagatc ttcggacaaa ttccccagga ggaaatggac 28020

ggtaggaaag ggctctccag ctgagtctgc gtctctctca gtgggtctct gactgtgtct 28080

ctgtctccca tctcctcccc tctccctgct tctctctgct cacacctgct gttccttttg 28140

cagtggccct catgattcca ggtaactgct caatggagcg gggctcctgg gctcatccgg 28200

aggggcccgt cgcaggctcc tgtgtgtccc agtatgccaa ctggctggtg gtgttgctcc 28260

ttatcgtctt cttgctggtg gccaatatcc tgttgctcaa cctgctcatc gccatgttca 28320

ggtgtgcctg gccttgccac ccggggcttc ccccaacttg aacctgttca atatgtgcca 28380

tgtcctgact ttgacccagt gctggcctga tcctgtgcct tccttacctg ggctttgaaa 28440

ctcgttgcta ttgttttggc atctctgagg cagtgtcttg gaacttaggg taatcttgcc 28500

tctacctctt agcaccggga tcacatgcgt gcctccaggg cactaaacgc ttttcttttg 28560

aagaagccca ctttagtgac ttgtttgcag tttgttttat ttgtgtgtat gaatgttttg 28620

cctgcatgta tgtatgtgta ccatgtgcgt ggaaagtgcc tatggtggtc agaagaagga 28680

catcggatcc tctgggactg gagttacagg tgactgtgag ctaccgagta gctgctggaa 28740

actgagtctg gatccttttg caagagcagc cagtgctctt aaccttagct gagccatcgc 28800

tccagtccgg tttttttttg tttgtttggt tttggttttt ggtttttgtt gcttatattc 28860

ttgctcttgt cgcatgtaat gtgatttatt ttgtgtgtct ggtccttcaa aggatcccca 28920

gttgctagtt aactggttct ttaagatttc ttagctgagt caggccttta atcctagcac 28980

ttgggaggca gaggcaggca gagctctgag ttccagctca gcctggtcta cagagtgagt 29040

tccaggacag ccagggctac acagagaaac cctgtctgga aagaaaacaa aaagaaaaac 29100

agacaaacaa acaaaagagc tcatagctgt tagaattgcc tgatgccagg gacttcagtg 29160

gtgattgcct tttggatttc cgggaacggg agaggatata taaggccagc tgaggctgag 29220

aaaggacttg ggcttgggca accagggtga gaagagaaga cagttggaga cagttgagcc 29280

aggagaagct gagctgagaa gataggctag accagttgtt acagtttgga gctgctagat 29340

tgggaaaact gggagaaggg acacactcac gcaggctctt aacctagtgg atctagagcc 29400

cagtccctac gcatgctgaa tcccagtcgc tcgctcacgt ggtaacgcat ttattcccta 29460

tttgtgcaga gtcagggaga caatattggg gtggagactc ttagctgtct ctttacgttg 29520

gcataagcag agccctgcct cagattcctc acctctgctg tcacacagcc ttagcaaatg 29580

agcagggacc tgggtcacac ctccactcat gacattgtgc tcacaatgct gcccatgtat 29640

tgacttccca gggcatcttc acagagcttt taacacagag gaaacaacga gctatttggt 29700

attaaatgga cccaagttga tttagtcact aatgcacacc ttcattctga ccaaccactc 29760

attctgagcc tgttatgcgc caaacacaag gacctcagct cgatgcccgg aatccgaagc 29820

agactgtccc cagctgaggg tagatcagtt gggagaaacc tttgaagggg cggggctgga 29880

aactaggagg agggctggca gggagttaag agaactggtt attatttgta agaataggaa 29940

acattaaaag gaaaaagaaa ttttaaaaaa cccacacatt ttacagtccc atacagaggg 30000

tcaatctaaa ctccagagtc tggaaactta agagttgatt ttggggttta gctcttagct 30060

attgatcatt agcccatttg gagttaattt agttcgcagt gtgaggacag gccctacttc 30120

agtctttgtg tcagttcagg agagtgcatt aaagacactg ggcataaagt ccttgttaga 30180

aaccagctgg gctcaggatg gtgtggtgga gcaagcttgt cccagcactc ggaaggtcaa 30240

ggcaggagga tcagaagcct ggggtacatg aatccctata tcagaatcaa acaggggatg 30300

gagagatggc tcagtggtta gggtcactga ctgcttttat agaggacatg ggatcgactc 30360

ccagcactca cgtggaagtt cacaacttcc tgtaatccca gttccaggag atccgacatc 30420

ctcttctggc ctctgtgggt aatatgcaca gacatacatg taggcaaaat agttatgcat 30480

gtaaaaataa attaaccttt aaaaaaaaaa tccaggcaat ggtggctcat gcctttaatc 30540

ccagcacttg ggaggcaggg gccagtgaat ctctgagttc aaggccagcc tggtctacaa 30600

gagttccagg atagccaggg ctacacagag aaaccctgtc tcaaaaacaa aaacaaaaac 30660

tatcaagaac ccactgtaga attttgaaaa catcaaagcc agaatgttgg agttgggtgg 30720

ccgtggctca gtcagtaaag catttgtctt gcaagctcaa ggacctcagc ttgatgcctg 30780

gaacccgtgc agactcttca tccattgtat gtatgtatgt atgtatgtat gtatgtatgt 30840

atgtatacat gcatgtacac cacatgagta cagtgctggc agagaccaga agaaggcact 30900

gatcttttgg aattggagct tccatattag tgctgagaac tgaatgtgag tgctctgcgg 30960

gtgccacgtg ctcttgcggt ctctgactac tttatctcga ctcctaatcc agtcctatga 31020

tcagggtcct ttattggctt cccgtgacta ttgacctctg gcctccacag ctacaccttc 31080

agcaaagtgc acggcaacag cgacctctac tggaaggcac agcgctacag cctcatccgg 31140

gaattccatt cgaggcctgc cctggcccca cccctcatca tcatctctca tgtgcgcctc 31200

ctcatcaagt ggctgcgcag gtgtcgcagg tgtcgcaggg ctaacttgcc cgcctcccca 31260

gtcttcgagc acttccgtga gaatggagct tggtctgcat gggagtgggg ccggggatgg 31320

gggggacgtt tggggagggg agaagaaaag caggtgggtg gggctcgggg aaggtgtcag 31380

tatctgcacc catctgggga cgaggcatga ggaatttggt ggctttgggg aggggacaag 31440

ttcccaagtg gtcctgccct ttgactcctg ggacttggcc ctgcaggtgt ctgtctctct 31500

aaggaagcag agcggaagct gctgacttgg gagtctgtgc acaaagagaa cttcctgttg 31560

gcacaagctc gggacaagcg agacagtgac tcggagcgtt tgaaacgcac gtctcagaag 31620

tgaggcgggg gcctgctgtg ggcaggacct gcaggtggga cgttctgtct tcagctagga 31680

gaactctctc tctttttagg gtggacactg cactgaagca gctgggacag atcagagagt 31740

acgatcggcg tctgaggggg ctggagagag aggtgaggac ctgggcgtgg gctggcgggg 31800

ctgggacccc ctgcttccct gctcctgcct tgacttattc ctgtcttccc aggtccaaca 31860

ctgttctcga gtcctgactt ggatggctga ggcccttagc cactctgcct tgctgcctcc 31920

aggggcccca ccccctccga gccccactgg ttccaaaggt cagtgtggtc ctgggcttct 31980

gcaagacatc cccccgtggg ccttcccgtt gttcctgggt cccttgcttg gtctgctgaa 32040

ggctctgttg tctgcttgtg tggggatgtg tgtagctggc gtgagtgtgc atgcctgtgc 32100

aggccagggt tgacctctct ctctctctct ctctctctct ctctcattca ttccttgaaa 32160

cgggacctat ccctaggccc aggtgtctcc agttggcttt gggttgctgt cccgacacac 32220

tgcgaaatcc ttctgtacct gccccactcc cccgtctttt gaagtgggtc cggtgttcac 32280

cccagggtgc cactgtctct ccccgctctt tttaatatgg ctgctgtgtg cctgttctgg 32340

tttcatatct gttgctagga taaaataccg accaaaagca acttagggga tgaagaattt 32400

acttggctta tgcttccagg ttacaatcca tcagtgaggg aaacagggag agagcgtgga 32460

cgttctcacc tataaccaga aagctcacag ccaaagaaac gcaccagaag ccacgaacaa 32520

cgctgttcgc tggaccacag caccttccgt ccagccagct tcagcaaaca gttcaggact 32580

cctcacccca ggaaatgggg ttgcccacag tggcctgggc ctttctacac caattggcaa 32640

tcaagataat gccccccaga catgcccaca ggtctatttg atctaaacca tcgaggctct 32700

tcctctcaaa tggctcaggt tgacagagct taaccagggc acagtctgag ctcaggtctc 32760

ctgcttgtgt gacaggcact ttgcccactg actgtgtccc ctgcactgct tctttttgcc 32820

attctctcat ttcctgggac tcatcaccca ctgtcctctc cgtctctctt ccagactgag 32880

tgttcctggt ggatgtctag gtgtttgcat ccagagaggg ttcattcctc aagctgggcc 32940

cagtctgcct caagcgacac aactggtggc cttatcctca ccacgaatcc catgcttatg 33000

ggttgctgtc cagacacact gcgagtatca ccgagggact ccttcccaga gcagcctcac 33060

cttgggtgaa tcaagctctg agctggcaaa gttggcctct ttgtgggccc agccacacag 33120

cagaggaagc aggagggtcc tggggtgcag gaaccacaga ccctacacac agcttcccca 33180

caatggggaa ataaaaccat ttgatgagga ttttcgcaca tgcttggctc ctgtggagca 33240

ggtgtgtgat gttgggaatc tgtccttccc cagaccattg cagctgccga cttagaccca 33300

gagcctctgt ttgggtggct ttccctgcag gagacagggg cgtctgagcc cctgccgctt 33360

tctgctttcc cgtgggaaca taacagggtg gtaataaaga gagctggtcc cagaggaggg 33420

tgggcgctgc tgtcatctgc tcaggcaaca tacaaacaga tgatgcagat ttgagtaggt 33480

aaatgcatat gtgtctttaa aaagtctaat tagtggaaaa ttgcaggcca cactcacacg 33540

ggggtggagg caaatccaca tcattataca ttgcaattta actaaataac ccataacaaa 33600

ctaagacggt aatgacaagg ctgaactgac agcgattatt agttaaatag cgagaaagcc 33660

accagcagcc aacacatcca aaaatcaaaa ccaaaacatc ctccaaattg gagggattcc 33720

agctccgggg cctctctggg tgtagagcca tgttggaaca cggccagacg tacaaccaga 33780

ctctccctgg aaatcctggg tccattctgc caacccaaac ggtcaccatg gtggcagtaa 33840

gaaggggcta gtgctagcac tcactgacac atgttgcctt ctgtgtggct ggccttgtgt 33900

tgtctggttc ataaatccga cttttcctag agtgcatcaa gggagggcag atccattttt 33960

cttgggtgct aggaatttag atgtttaaag attccttttt tttttagatt tatttattta 34020

ttgttatatg taagtacact gtagctgtct tcagaagctc cagaagagag tgtgagatct 34080

cattacagat ggttttgagc ctccatgtgg ttgctgggat ttgaactcag gacctctgga 34140

agagcaatca gtgctcttac ccgctgagcc atctctccag ccccaaagat tccattttta 34200

ttttgtgtgt gtgttggtgt gtatgtgtgt gtgtgtgtgt gtgtgtgtgt gtgtgtgtgt 34260

atgtgcctgt gcatgttcgt tcctgcctgt gtaggtcgtc gtattgatgc cacaatgtat 34320

ggatgaagat cagagtgaca agttagggag caatgttttc cttctaccac gtgtctccca 34380

ggaatcaaac tcaggttaca gcaagcatct tccagcacca cgtaatggtg gcccagccct 34440

tccctggggg attctagaca ggagctctct accactgggc tactctctta atctctctct 34500

ggactctagg aaactgtgct agctgcctcc ccagacccct tgagcttttt ttgatctagg 34560

aaccagcata tctcgtcggc agaaatgtca ctccagatgg ctaaatttga gtggatctgt 34620

ttgtaagaat cctttaaact cagtccagga gtttggttca ggaccccatg gaggagcctc 34680
aatgtctact acatcccttg gcactgtgga gtccagggaa accaatgctc atgtgttagt 34740
accactttct gaactgagaa aaggcgagcc ccctccccct ctctgatctt ggagtcttat 34800
gccttttggc tgcatctata acacaacact agactaactt gtaagcaagg caaagtcaaa 34860
ccccagacct ggccaggcag tggtggcgca tgcctattaa tcccagcact tgggaggcag 34920
aggcaggcag atttcccgag ttcaaggcca gcctggtcta cagagtgagt tttaggacag 34980
ccagggctac acagagaaac cctatctgga aaaccaaaac caaaaccaaa aaacccagac 35040
gtgacggtgt tgatgaggaa aatgtacttt tagtgagggg tggcttcctg ctataggaga 35100
atgggagctc tgtaggtcgg gatgagagga gcctggccag gatctagctg ggatttctgt 35160
cagacaagtg gagggggtga ccaggaacaa gggtctgtat gggttacaaa agtacagaca 35220
gaagcaacag aggagaggtt tattttggct tatgatttta aggaatattg tccactgctg 35280
tgggggtagc atggttatat tgaatctgtg gtgttagccc gttcatgtca tggcttacta 35340
ggaagcagct atacgccgga aacggatccg ctcaaagcct cattcccaac gacctacttc 35400
ctgtcttcag cttttcacca ccaaaagtcc cactatttcc caaaggatgc gacctgttgg 35460
agacaacaga tgttcaagca catgaacacc tttcacaccc aaacacaagg agctcaggac 35520
aggatagcaa cagcagtgta gagtgctcct ggcaaagtgc tcatgatacc tccttgggcc 35580
aagctggtca ccgtgatggg catgatattg aggctggagg ctgatggtgg atgagggctg 35640
tggtatggag cgggatcctt cttcctagtg caggcaggtc ctaggggctg ccctggcacc 35700
cttactggga cccccaaagg aagctcatca tgatcttcac tgttctagca cgctagacga 35760
tccatgctcc tggctggtgg ctgaagctat aattattgac tataggtttg cctatagcag 35820
ggaaaatgcc caaatccaat tgatgaggct cacagtggga aaggactgcc agtggagccg 35880
cccggctttc cgttgtcatg gaagcgtggc atgagcggct gttgcagagg cttctatttc 35940
ctgggtacca ggggatcctc caggtcagga actgctcttt gctcccatgc tacctgaaaa 36000
atctggaacc ccacagagaa gaaggaaaag gcagctttgg tgtcttcacc ttcagggaat 36060
cctttagatg ccgctttcca tcacaacagc gccacctgcc agatgcatct gcgagcacat 36120
gcatttggag aatggcctct atcattctct agagatcctt gcagagtcag gctggagccc 36180
agagactttg atttccctat ctcacacccc cgtttcagga ttagttagtg gaaaactgac 36240
tactacaagg ctgggaactg ctgaatgtaa gttctgtgct caagaactca ccatgtccag 36300
tcctcggact atttcaggac gtttgtttat ttttatttat ttatgagata aggccttgct 36360
atgtagctaa ggctggcctc taactcatca ctcttcatct tcagccaccc aaaagctggg 36420
attataggca tacaccttcg ccacactccc acaactaggt catctcggtt tggcatgaaa 36480

aagtagcaac tacccctttg tccctttgaa taaaatctta gccacctcct caccccccac 36540

attgctggtt cctcaattca cagacatcct accccttgaa tgactggttg ggactgtggt 36600

aagggctgat gtgttcgcta ggtggcagca tnnnnnnnnn nnnnnnnnnn nnnnnnnnnn 36660

nnnnnnnnnn nnnnnnnnnn nnnnnnnnnn nnnnnnnnnn nnnnnnnnnn nnacgcagct 36720

tatgccattt gttaatgcgt gtggccaccc cccaggaaac ttcatatctt cttggttgtg 36780

ttttgcatag taacagacca aagcaggggg agagctatat aaaccgtttg aaaattccag 36840

gtcctgctgc ctgttgtttc ttggggctga tgtgccttta cccactgaac accctgacag 36900

agaagtcggt tcagtctact gaggtgtcgc agccctcagg ggctgaggga gattattctg 36960

ggctatggaa tgaattcaag ggtcagcctg ggcaacttag tgaagctatt tcaaagttaa 37020

aataacaaca acaaaacaac aaataagact ggggaggtaa ctcagggggg agatttcctg 37080

cctaggttag ctgcatgagg ggttgggggt gtgactcagg ggtagagccc ctgcctagag 37140

tcgcccagtg agcatgaacc agacatttag ttcagtccta tgtatcaacc ccccacccac 37200

cacacacaca cacacacttt ggtcgagtca tagttttcct taaactcaca tggctctaga 37260

tcagcaaagg actgactggg ataaatttgg ccagtcacct ggtccacaag ctaacatgta 37320

tttgtaccta atgtgaatat acagtaaagg ccatatgtcc tgcagaactg aaattttttt 37380

gaccttttag gtcttagccc gtactgcttg ctgagggtgt tcacagctta atatgcaagt 37440

ctccaaaaac aaagtggagt gactagctag gtggaactgg tacctgtctt taaccccagc 37500

actcaggagg cagaggcagg aggatctctg tgactttgag tgagttccag gagagccagg 37560

gctgtgtagt gatgtgtcgc atgacgatgc tgagtgggca ggtggtggtg atgatgtcac 37620

aggtgaacac gaagctcaca gtggctgatg gggtctcttt ggatctactg cttcctggag 37680

tcaccccagt ggcctctgat gggtggcaca gaggttggca gcacatgtga catgccccta 37740

agatgggttc tagtaactgg gagctaaaga tagtggtcaa gggagggaga gtctgccata 37800

gtccttctgg gggatttggg tgaggcctgt ctccacagag agtaaaaaag tccccggggc 37860

gtaaacgata tccactccca gctttctgga caggaaagta ggcattctct cctttgagaa 37920

aacacccctg tgtgattaac attccttatg aatctctccc tggtgatctg tgggcaaaaa 37980

gaccttcgtg ccccagcctt aacagcacag ccggggttgt tcagagagag aagcaggccc 38040

ctcctttctt ctccctgctg caatttttaa gacagttccc tagtctgttg cccagtctgg 38100

cctgcaactt ttatatcact tagactgctg acctcagctt catggtgatg cccacgcttt 38160

aatatctttg ttaggtacaa aggagactcc agttccctca aactccaaat gtccagaaac 38220

gggctcaacc ttagagtata ggaggatttc tagtggttag ataaaagcca ccaacatcct 38280

ccaggaactt gtgaagccag gtccctctag caaaaaagtg tcattccccc ttcctcgagc 38340

agaagggaca aatggctgtc tgtggtgcct gttatcatac tcaagctcat gccggcctcc 38400

aggttctctc agcatcacga gtacctgtta tccatttcag agtccaggtt agttcttctc 38460

acctccagct cactggcttc ctttgcccgg cttagtcctt ctccttataa ctctgctaat 38520

tttggctata ctctctgtct tagttagggt tgctatttct gtgaggagat accatgacaa 38580

cagcaacgcc cataaggaaa acatttaatt ggggctggct tacagttcac gacatttaat 38640

ccactattgt cacacaggaa gcatggcagt gtgcaggcag acatggtgct ggagaaggag 38700

gtgagagttc tccatctggg tcagtaggta gggagtgaac tggcttgagc ttctgagacc 38760

ataaagccac acctcctcca acaaggccac acctcctaac agtgccactc cctatgggtg 38820

aatgccccac cccccacccc atatttgcta aaaccaccac acccttgctc tgctaggcca 38880

ccctttctct cttttctcta ttctctattc cctgtctctt agttagggtt tctattgctg 38940

tgaagagaca ccatgatcgt ggagactctt ataaaggaaa acatttgatt gtggctggct 39000

tacagtttta gaggttcagt ccattactat ataaagttgg aagcatggcg gctgcaggta 39060

gatgcagtgc tggagacata gccaagaggt ttacatctgg atgcacaggc agcagtaaga 39120

gagcatgaca ctaggcctgg cctgagctga tgacacctca aatagtcccc tgtgactatt 39180

ccatatgcct ctggctgctc tctcatatct acagtaaaaa ccttccccgg aaacattccg 39240

cactctgtca ggtgtcagtt tatacagcac cggagtgtgg cgtgctggaa ttactacctc 39300

atcatgtttg ttcctgagct gtctttctgc tctttttgac catttgtttt gttttgaaac 39360

agggtctcac tccataggcc tggctgtctg ggagctcact atgtagactg aattgggctc 39420

aaactcatag cggtctgcct gtctcttctt caagtgctga gactgaagat gcccaccgct 39480

atgccagtgt agctgcttgt ctttttttcc ccctccctag tgggttcttt gtgggttcct 39540

cctctggctc agctcagctt ctcctgagtt aggattgctg tctctgattt ttgctctgct 39600

caatctcttc ttcagcccta ctcagtcttc cttccagcac caactgtctt ctctgatact 39660

actcttctgc cagctggacc ctacccacct ctcccagttt cggctggctt tttatagtct 39720

ctgcttgccc agtagttcaa gaggcaacca aagagcatag ggccaagcag tcctaaaggc 39780

taagcatccc aaaagagcca gctgactagc aactggggat cctttaaagg gccaggcaca 39840

caaaataaat cacattgcat ttctccccat ctggtttagc tgctaattat gcagctttga 39900

atctgccctg ggcgctccct gtagccatgc tactgttgtg atttttgctt ctccattctg 39960

tttaatgtcc tttccagggt tttcttgcat ctgctgtgtc agtctttcac atcacatcca 40020

caactgagat aagaacctaa cagtgatgct tcccgtcctg tgcttggcta ctgcttcctg 40080

aaccttttat gctcctggat tgtttcttgt ccaggtttct ctggctggca gggtcagtag 40140

ctggtcaggc tctcttctct ctctctgggt cagaagacct cctctgctca actctgagcc 40200

ttattcgttt tgatggtatc tacagctttt tgtttcctat gaaaatataa acaaatttgc 40260

atccccaagg caaaacattc actgacttcc atctgatgtt aagccatcct tatcagtgta 40320

aagtgttttg ccctggatgg gagggtaccc tgggactcag aagcccagga gccacacagt 40380

tcagaggtgg gacagtgtta cagtcagtga tagggacatt ttgacaggac cctccatttt 40440

gatggctttt gcagcatgcc acttagggac tacaatcagc agtttctata tgcagaacaa 40500

ctctttctgc atatcgagag tcaggaatta tattaagaag ttcaggaaaa tctaataata 40560

tcacaaaagt tgcatacaac tctgattttt gagctaaagt ataagggctt agaagtattt 40620

tgcttaattt ttttctgatg tatgtgggag aatccaatta gtccatttta ataaacagaa 40680

gatgattatt tttaggatat ctattattaa tttctcccaa atagttgtat aagttctttg 40740

ccagtcatca ttgatcactc ataatgacat agtctcagta ttagtatatg gtactacaat 40800

ttctactggg tctgctccta acaattgaca tagtgttatt cttcctttca taattaaatc 40860

agaaaccatt tctatataaa tatataactt tttagtttat atgttatgaa ggtccattct 40920

aaaatactat cttccctttg cataatgagc ccagtagggg agtgagttga aggcaaaata 40980

accaaaacac agtccagttt gggatctatg agatccacat gagcattttg cagtctctgt 41040

actaccagag ctaactttct ctctgcttca gctagcaacc atcttagact gtttgagtcc 41100

aaatctcctt gtaaaaactg aaaaagaaaa ttacttaact cttgagttgt tcatctaatg 41160

atggaccata gccaattaat agctctcatt gatttttaaa aatcattaag agttgcaact 41220

gatctcttct gtatcttctg tggtggatca atttttttag atttatttat tttatttata 41280

tgagtacatt gtatctgtct ttagatatgc cagaagagag catcagatcc tattatagat 41340

ggttgtgagc caccatgtgg tttctgggaa ttgaactcag aacttttgga agagcagtca 41400

gtgctctttt tttttttttt ttttttaaag atttatttat ttatttgttt atctattgta 41460

tgtaagtaca ctgtagctgt cttcagatgc accagaagag ggagccagat ctcattacag 41520

gtggttgtga gccaccatgt ggttgttggg atttgaactt tgtaccttcg gaagagcagt 41580

cgggtgctct tacccactga gccatcttac cagcccccag tcagtgctct taaccactta 41640

gccatctctc cagccctgca gtggaatttt ttgttgacta attttatagc ccaaatagtt 41700

aatagaatct cctctatgat ttttttcaga agcaatctgc aaactccatc aagacaaaat 41760

ttttttgttt catcattttc tctaaagtag ctctatcaga attggccaac aaaatatcat 41820

ccatgtaatg ataaataatg gattgaaaaa attgcctaca aattatttct agtgattgtt 41880

gcacaaacta ttgacataga gttggactat gtaacattcc ttgtggaaga gctgcccact 41940

gactttttac aggctgagca ttatacgtat gcacctatga aggcaaaatt ttcttcatcc 42000

tgctcttgca agggatagta aaacaaacag acaaacaaac agacaaaaaa aaaaaaacag 42060

cctttaaaat aaattactat catagggcat gccttaggta ataaagaaag taaaggaatt 42120

ccaggctcta atggacccat tagttggatt actctattca ccattctcaa atctgttgtc 42180

atatcccatt tcccaatttc tttcttataa caaatgcagg ggaatcccat aggtagatcc 42240

ttctatatgt tgtgcctcca actgctcttg taccaactgt tcaagtgtct atagttagtc 42300

tttagttaag gccattggtc tatccacaca ggcttgtctg tcaaccattt taagggtaat 42360

gccattggta tactagatag ggttgaacag ttgttaaagc tgggagctgt tagtattggg 42420

gaaactggga gaagggactg cttaagcaga ctgttgggga gctgagggag gaagaattgg 42480

gagaaatatt tgaaggggct gggctggaaa ctgggaggag aactggcagg aagttaagag 42540

tttgtaaaaa taggagacca ttgagccagg agaagctgag ctgatccaga aaagagaatg 42600

ttagcagaca gttaggagga ggacccacgt ggagcccaca aagtagcaga aaagttacat 42660

gccaggctat tttactttta ggtgtgtgtg tgtgtctgtg tgtctgtgtg tctgtgtgtg 42720

tgtgtgtatc tgtgtgtgtg tgtctgtgtg tgtgtgtgtg tctgtgtcta tgtctgtctg 42780

tctgtctgtc tgtgtgtgta catgggttct cctggaattc tagttttgag ccacctgaca 42840

tgctcttaac cactgagcca tctctccagc tcagtgttcc ttgtatattg agacagggtc 42900

tttcactggt gttacttact agggtggcta gtaagtagcc ccagggatct agctgcctct 42960

gcctccccag tcctgggatt acaagttcac cctactatac caagtcctca tgcttttaag 43020

cacaacatca actgagttac ttccagccta gagtgaggtt gctgctcttg ttcaaactag 43080

atccaggtgg gatgggagat atcttgcttg ctctcagctt ctcaggcttt tcctgtttgt 43140

aagtgggtca ggaggctcct gacacacaca cactcctata agtctgccag tgagccacat 43200

ctctctgttg tgccttcaac acacttttga tgtctttgct atttgattcc aacatttgta 43260

acacttctgt ctctgaccta gaagtcattc acatctcttt caaggaaatt gtgccaggtt 43320

tgggctccct cataccctct gtagtggttt gaaagaaaat ggcccccata ggctcacagg 43380

gagtggtact attaggaagg gtggccttgc tggagaaagt gtgtcactag aggtgggttt 43440

ggaggtctca gatgctcagg ctaggccagt gtctttctcc ctttctgctg tctacagatc 43500

aagatctaga acttttgcta ctactccatc atcacatctg cctgtgtgcc accatgcttc 43560

ctgccatgat gacaatggac taaacctctg aaactgtaag ccagtcccaa attaagtgtt 43620

ttcttttaca agggttgttg tggtcatggt gtctcttcac agcaatagaa cactggccac 43680

tggccgagac accatctaaa ataacaacag tcctgaattc acctcagaga tggcccaaat 43740

tatacctgaa gcttgagtga ccactcacca tattcatata cctcggagcc ctggaccttc 43800

tggtgaggtg gaatggagca gccagacagg gttcatgcta cactgctctc caggacagga 43860

gcaacccact caagtttgca ctcagcctag tctcttggaa gttctctgtg gcaggctttt 43920

cttgcttctg gtcctgatct tgggtgcagg tcaatccaat aagctcagga aatatgccaa 43980

cagggatggg taatattgtc aactagccag aatttagaac tgtcaatcag ataatgccaa 44040

gggattatcc ataattggtt tattgatgta tgaacatata cactaactgt gggctggggt 44100
cctgtcctga ctaaagagga gaaagctggc tggacagctg tatccattgg gctgcatttt 44160
gcctgtctcc tcaactcaga ggtccccaga tgacaagctc ctaccactac cacttctcag 44220
tggaccctga agcttgagct gcctcagatt attcttttat tattatctta ttttattatt 44280
cttttaatag tatccccaaa gagtcaagaa tggtgcatgt ctgtgacacc tgcactcaag 44340
gagtggaggg agaaggatca ggagttcaaa gtcatcctgg gctgcctagt gaagcggaag 44400
ctagactgag ctgtaagaga ccctgtctcc aagagcaaac aatcaggcat ggcaggggtc 44460
tttattttct attgctttga agcaatgtca caaccaagga aacttattgg atttattgga 44520
gcttatcgtt tcatgggcat ggcactggag tagcagctga gagctcacat cattatctcc 44580
aagcaggaga gaggagagga gaggagagga gacgagacga gatgagacga gactacttgg 44640
aaatggcatg ggcttttgaa acctcaaggc catcctgtgt gacacacctc ttacagtgag 44700
gccatacctc ctaatccttc ccaaacagtt ccactaactg gggaccaagt attcaaataa 44760
tcgagcctat gagagttatt ctcatttaaa ctatccatag tggcacacac ccttaatttt 44820
agcacttgaa agacagacgc agggggagca gggggagctt ctaggtctat cagggctata 44880
tagtgagacc ctctctaaca agcacacaag gaaaaaaaaa aaacctaaaa caaaactatc 44940
cccaaagaat catctgtcca catttagcta aacaagtatc atctatgtcc ttgactaata 45000
tctctgacat tgtcctgttg gtgtgtggct tgatggaacc tctcttacct gcctgacaac 45060
ttcatgattg ctgaaacata ccaagagatt cactctcctg aactctgtta ctaactaaaa 45120
agatagcaac ggcctccgtg tgtcctcttg actcttttgt ttaagaggtg gttctgacct 45180
ccttgctgcc caaattctgc cttctaaaac ttctccaagg gggtgctgtg ctcagttgct 45240
ttagagtcct gaagaggacc caaggtcagc tctcagagct tgtgttggga ggctcacaac 45300
tgctgttaac acaggggctc tgaccactat gggtactgca ctcccatgta cacacacagt 45360
atatacattt taaaagagaa agctattttg ttcaccttgg tcaatgagac agtcttatct 45420
ctcagcatgc ttcctgtttc tgtgataaac accaggacca aagacaaccc agggaggaaa 45480
gggtttattt cagcttacag tttctagtct gtcgtctcag gacagtttct agtctgttgt 45540
ctcaggacat gagggcagga gcttgaggac agaccctgga ggcgggagct tacatagaga 45600
ccatgactct ggaaggatta tgcttcccag cttgctccca tactcagtcc catctttctt 45660
acacagccca agaccacttg cccaggaagt gacattggtg agctggccct ccaccatcaa 45720
ttaacaatca agaaaaggcc acggaccaca ggccaggctc aaaaaggcca tccctcagat 45780
gggaaaccct cttcccagat gtccctaatt tgtgtcaagc tgacaaatat ctagccagca 45840
cagcctcctt aacataagca gtctctgctg gaattaagca ccccacccca tgtctcctgg 45900
gtcacctctt ccacctcaac tccgcttatc tgttcaaaag actccaccta ggtgagggcc 45960

caggcctggt ctcaggctga gacccaactt ctttccagga ataatggtca actccaaaat 46020
tcccattaat ctctcctctt cccaatgtgc ggtgtgtgcg cactcgcacg tgtacgtggc 46080
atgtgtatat gccagcaaag gcccgattag gatattagat cctgtaactt tttactatca 46140
agttgggtac gacttgagac ctacctggga agagggaacc tcaatagaaa atttgcctcc 46200
gtcagattgg catgtaggca agtctgtggg ggcattctct tgactaattc tcttgattga 46260
tgtgagaggg tccaacccac tgtggccttt gccattccta ggcaggtggg tctggtatat 46320
atgttaaagc taactgagca agccaggaag tactgtctct ccacagtttc tgctgcaagc 46380
tcccgcccgg cttccctcct tgatggcctt gaactgctat tcggaaggaa accctttccg 46440
gctgaagttg cctttgttta tcgcagcaac aaaaagcaaa ttaggacaca tcagctgtca 46500
gggtgtgtgt tggagaacca aactccaatc ctttgcaaaa gccacagaga gctcttcact 46560
gctcagccac ctctccagcc ccagcagctc atcctgatct acatctgatt tacagaccca 46620
ttcatttctc ttaggtgcca caagggcttt tccctctctt ttcttcaatt tccacttccc 46680
cccatttcct tatttacttt atatccccat tgcagccagc ctctggtgtg tctgtctgtt 46740
ctcagccgtc ccccatggac tgacattata caggtcagac agcctcttcc ttggaatttg 46800
tttgccctgg ttttttgggt tttgttttgt tttgttttgt ttgttttgtt tgttttttgt 46860
tttttgtttt tttgtttttt ttgtttttgt tttttgtttt ttgagacagg gtttctctgt 46920
gtagccctgg ctgtcctgga actcactttg tagaccaggc tggcctcgaa ctcagagatc 46980
cgcctgcctc tgcctcctga gtgctgggat taaatacaaa ccagctgtct ctctttgggc 47040
tttttgatgg ctcctcacag gtaaggtcag gtgacctctg accacccagc tggctttggg 47100
gatcaagaat tcggcactga ctgcttccag gatcaagctg aataagtgcc actgatttgg 47160
ctggagtgtc atcagagtgt cttagacaca cccacttcca gtgaacgaaa agtattccga 47220
agtcaggtaa aaatcatgct tacctctcaa gtctcgaggc tggagagatg gtgcagcagt 47280
tggagcgctg gctgcttgtc tagatttggc tcccagcaca gaaatactta caactccagt 47340
tccggggggg ggggtccaag accctcgtcc caacttcacg cttcgttagg gaaccaagca 47400
cacacgtggt ttatgtatgc agtgcacatg tgaataaaac gctcttatac acaagagacc 47460
tgggcccctt ctgctgtggg gccacaaagc tctaagcggc tcactaggaa gtgcgcgcat 47520
gtacacaccg ccggtatgtg tcgtccaccg cctcatttat tgacaggcaa cattgctatc 47580
ttggtgccat ctgtgatggc catccctggg aggtcagcct cacttctgac aggtacgatg 47640
gctaggcagg gttaggggga tcactgtgtc tctcggtaac tgggactgtg ccagatgccc 47700
atctgggcac accaaaattg tgtccgtatc aaagagaggg aaggcggcct ggggcctgcc 47760
ctgggcacag tgtccagtgc tagaggatgg accaatgcgt catccatgac tgtctggggg 47820

ccgtgggggc tcccgcagaa gtctgggctg gggtctgcat accctgtctt ttgctttccg 47880

tggggccttg cctgtgctaa aggcttggcg cccatcactg gttaccgaga cccgacgatg 47940

tggctggccg ctgctaagca gggctctcac gagcctctcc ttcacaagtc gcaagcctgt 48000

cacttctttc cagtgttttg ggagtccaaa aaggattctg agtggccggt gtactttgac 48060

acctcatgga tggccaaggt ggagaagcca acctgagaag gaagcgtggc atcgggcaag 48120

ctgaaggtgg aatagttctc actgtccttt tttgttatca gggtctgggg gtgaaggaag 48180

ttctccgggg gactcttgct ttggaataaa gccttgcttt tctcctgatt tcggagggtg 48240

agtttcctca tggggtgcac aggccactgg gtgactctgt tgggcaccgt gactgtcttt 48300

ttggaggaca gaggcttttc taaggtttca cagatgtttg ggtcatccag ataccaccag 48360

tgctgcagcg ggtacacgag gatcggcagc agtgccagga cactgaggaa ggtgacccag 48420

cccaggggta tctttttgta aggctgcttc acttcatggc tctggggaca gggatagtgg 48480

gccacagggg aggcatgggg ggctcagtct gggtgctctg agtgcagggt tcccccaagt 48540

ccctggggta gggtaggaga tgtaggatgg gggcctgtgt ctatgtgctt gggatttggg 48600

gtctccaggt ctctgagggt aggagataca gggcggggtg ctgggccccc aggtccccac 48660

aggcaggggc cgcaccatgc tatcattcca ggagacatag tagggggatc cactgtagta 48720

gagattcagg aagtagatgg tgagcaggac caacagtgtg ggcaacgtca cgtagctcca 48780

caggaaggtg aagggtgacc acaccaggct gcccagctgg ccgaacacct cagccctgaa 48840

cctgggggaa ggaggtaact gtcaggacat ctgtaacctg gacccaggga gagcttctat 48900

ctctggttct ctcagatgct cttttcgctt gatgtgggga ctgggccctc cctggctccg 48960

ggacagccat gttgtcaccc cccatgagaa gtgaaccacg actgtccctt ggccttcagt 49020

gtacctgaga ctatgtctcc ctgtaggagc tgactgctgt ccgaggaccg gtgggggggt 49080

gcctgtatcc ctggaaggcc taggatggac ttaagagcta cgcatccctc ccacccctcc 49140

cgacctcccc gagtctggaa atcacctctt gattccatag agccaagcca gagacaggtt 49200

ctggatcacc acaattatga tgagcaccat ggggaccagg aggtcatcga acaggaatac 49260

cacgtagctg ccagggcggc tggagaagac gaggctcccc agaaatcctc ccaagcagac 49320

aagcactaaa ggtcaaagtg gattggggca cactggagtt tgtgagttgg gggggggtcc 49380

ctgtgaatgc tggaatactt ggggtgagca tgggggaggt tacagggtat ggttgttttt 49440

tttttctacc ttgtaccaga ttgggatgct ttgcaaaggt agagatgctt ttctgcagag 49500

gaagcacgat gccctccagg agagttagca tggtgcccag acccatgata gccagggaca 49560

tgaagaagat gatggcccag aaagaggagc cgggaaacag tgagatggct tgagagaagg 49620

ccacatacga caggccaggg ccctccataa tctgtagggg gnnnnnnnnn nnnnnnnnnn 49680

nnnnnnnnnn nnnnnnnnnn nnnnnnnnnn nnnnnnnnnn nnnnnnnnnn nnnnnnnnnn 49740

nnnnnnnnnn nnnnnnnnnn nnnnnnnnnn nnnnnnnnnn nnnnnnnnnn nnnnnnnnnn 49800

nnnnnnnnnn nnnnnnnnnn nnnnnnnnnn nnnnnnnnnn nnnnnnnnnn nnnnnnnnnn 49860

nnnnnnnnnn nnnnnnnnnn nnnnnnnnnn nnnnnnnnnn nnnnnnnnnn nnnnnnncga 49920

ggctgccggg cgggcccggg cccctgacaa acacaccttt tccttttgta tcaggatgct 49980

gcagggcagg gtttgatgga tgacctgact ctttaagttg ttgggaagct ggttgatcca 50040

ggctaggtat tccagtgggg gtttatggat gatgtctccg ggaggccagg cggccttggg 50100

gaggaccccg ttggctatca ggttgatcag attggagaca ctcctgatgg ggtcagaagg 50160

gaaaagaaga cagacagaga atgggctggc tcatcagccg tcaccatcga aggagagtaa 50220

ggaccaggta tggtgggacg gtccaggagg cacgctgccg tggttctgga cgtccacggt 50280

tgaggagttc catcttgtga gagcatcttt gtgttgtgag gacgcagcag ggtcctgaca 50340

cagcacaggg agctgcaggg ccaggcagag caagcttcct agctgccttt tccatccctt 50400

ggtttgcatt tgttttgttc atttttgaga cagggtttct ctgtgtagcc ctggctgttc 50460

taaaactggc tctgtagacc aggctgtcct cgaactcaga gatctgcctg cctctgcctc 50520

ccaagtgctg ggattaaaga catgtgccac taccacctcg cttttttttt tttaatttaa 50580

aaaaagatta aaagaaaaca acaacaaaaa aatcgactca gcagttcaga gcactcgctg 50640

ttcttgcaaa ggacctgagt tttgttccca gaaaccacgt gacagctccc agaagtctct 50700

agcccttcag agttcccttg cctcctgcat gcaggtggaa cccatacaag tgcagataaa 50760

tacacataga taaaaatttt gaaaatttgt tttgagacag agtctcagta tacagccatc 50820

actggcctgg gacttactgt gttgcccagg ctgtcccata aaggcttgcc tagcccaagc 50880

tgtcctatag aggcttgcct agcccaggct gtcccataaa ggcttgctta gcccaggctg 50940

ttcgataaag gcttgcctga tttttgcctc ctaagtctgg gattaaaggc atttgccact 51000

atgctcagtt attttatttc tttttaaaga ttataatgat gcatttaatt tttctatagg 51060

tatgggtgtt ttgcctgcat gtatatggtg catatgtctg gtgcccaagg aggccagaaa 51120

agagggttgg atcccttagt aatagaggtg cagaggttgt aagccaccat gtggatgttg 51180

gaaatcaaac ccaggtcctc tggaagagca agtattcgca actactgagc caacagttct 51240

caacctgtgg gtcacaacct ctctggggat aaacatgctt ttcacattta tgatttataa 51300

cacagtatcg aaaggagctg tgaggtagca acaaagagaa ctttatggct gggggtcagc 51360

acaccctgag gagctgcatc caagggtcta agcatcagga aggttgagaa ctgattccct 51420

gctgctttgt tttcaagata aagagtttct ctgtgtagcc ctggccgcca cctcaatcac 51480

tttctcttct tatagagcca ttaactgtat cccgagagtc ccagcctagt gaccttgtct 51540

aactgtcacc ggtgctcaga caccctcatc gacatacaac tttggggagt aagcgtgtca 51600

tgcacgaagc cacagaaacg cagcagtcaa gtccttggca ctagtgccta gtggtctcag 51660

actgtagtca cacggtcatc tcttggtaga cagtggtcag tcactatttg ggtcactgtg 51720

aacagagcca tgacgcagat gtctaccaga tggtgagcag cggcagtgga ctgggcgtcc 51780

cagcgtgggt gtgattggac tttgagaagg acttgtctac aaaggcattg ttttacacag 51840

ccgacaacaa agcacacagc tcccggttgc tttccattgc ttcccgtgca ccgactctca 51900

cagttttctg tcttaatttc tcagtctact tggtcggtaa gtgaccctgg caggtatggc 51960

agtgcgatct cattgtgact ttcggtttct cagatgcaag ctgggttact gcgatgcatg 52020

cttatcacct cggaactcgg gaggacaaga gagtgggatc agagattcaa ggtcttcctt 52080

ggctacctag ggaggtcaaa ggcagccttg gccatatcag gccctgcctt tgaataaaag 52140

ctcaacttct gtggacagat cccttgccgt acaaacctga gctacggaac tgcacaacag 52200

ggagaaacag actccacaaa gttgtgtcca ggcctcaaca atcgtgccca gatgtgggca 52260

tgcggtgtgc acaagcatca agcaacaaac aaacaaaaac ctgggctagc cccagctttc 52320

ccagctttga catcctaatt ggaaggaaaa ctgtggagct gttaaacatc tgtgaatgtg 52380

gggccagaga gatggctcag cggttaaaag cactgactgc tcttcccaag atcctgagtt 52440

caaatcccgg caaccacatg gtggctcaca accatccgta atgagatctg acgccctctt 52500

ctggtgggtc taaagacagc tatcgtgcac ttacatagaa caaatctttt tttttttttt 52560

aatctatgaa tgttacaaca attaatgaaa aaaaaaaaac caggctcatg aatttgaaaa 52620

gagcaaggta aagtatatgg gagaggttgg aagggggaaa agggaaggga aatgaaataa 52680

aacatcaaaa aattaaaatt aagagcattg ctgtgtgcaa cacaaactcc agtgctgcac 52740

ccactgtggc agcacacacc tttattattt tatgtgtcca ggtattttgt ctatgtgtgt 52800

atgtatttgt accccatgtg aacagtgctc tcaggggcca gaagagtgtt ggatctcctg 52860

ggaccagagt tatagatggt tcatttgggt gcttacgatt gaactcaggc cctctgcaag 52920

agcacccact gttcttgttt tactcttgct ttttttgaga cagggtttct ctgtatagcc 52980

atggctgtcc tgctctgtag accaggctgg cctagaattt cacatatcca cttccctctg 53040

ccttccttgc cagtgttaga agtaaaggcg tgtgccacca ctgcctggct ggggccagtg 53100

ttcttaacca ctgagaaatc tctcgagtcc tggcacacac ctttaattcc agcacttggg 53160

aggcagagac agtagatctt tgagagtttg aggtctatag agtgagttct ggacagccag 53220

ggatacacat agatactctg tttcaaaaag aagaggagga gaagggagga agaggaggaa 53280

gaagaggagg aggaggagga ggaaaggagc aaacaaccaa aaccctagtg cccagctcca 53340

gccatgacca aagaggtgga acatctggga ggagactcag ataccagccc tccctaagac 53400

ttcccaactc aaaccatcta tgccaacaac tgcttccgga ggctcagcaa gcagtggcca 53460

gcaagctctg tggtgtaaat aagcaagatg gaaatgttcc ctaacttcat ttgcaatttc 53520

taagactgag ataaggtaag gcgggtacca tcgagaggtt tgcttggcag gtcagtgggg 53580
cccctcccac cttggctgcc cgataatatc acccacctct taatacacat agtgccgctg 53640
gttgtggtcc agaagcccat cacgaggaag atgacgaatg agctcagcaa cgaagtcgcc 53700
aggttgacca tggccacaaa ggaggcccac ttgatgtaat tctcacctct aggctggtag 53760
gtggagaaag ttatgatagt gcccatgccg aggccgaggg agtagagaac atggcctcca 53820
gcctgacgcc atgcgtccag tgatgacagg atggaaagct gtggaagaca gaggcatttg 53880
gagtggtttc ttgggtgaag ggaagagaag gggtgaaggc caggcagagg gacaaaagat 53940
gcagagaggg atgaagtggg aggcctgtgt gtgtgtgtgt gtgtgtgtgt gtgtgtgtgt 54000
gtgtggtggg gggtgataag gagtctggag ccccggcctt acctctatgg tcaccaggcg 54060
ccagaggctg gccctcacgc tgccgttcag gaagagggtg cggatgaaga agcagaggag 54120
gaggacgttg gagaacaata tggagaaaat catcatctgg ccgggtgggg tcaggagaaa 54180
cagggaagag gaagacagag aacgcattag caagggccga gaggaggggg cagggggcac 54240
acaaggggca gggagagtgg atgcgtgcag gaaggcagtg tctgaggaat gccacccaag 54300
gttgacccct ggcctctggg agcctgtgca tctccacctg cacacaagtc tgacgccaag 54360
ctcctgaacc acctggcagt cgcaggcatg ctcctgtctc taaagcctct ccttttcttc 54420
cttccctaca agtcctccca cgcagcctcc ggtcctgcga gctcccttcg gccttgctac 54480
ccaccctggg ttttgagacc aggtctcaca gtgctggatt agacgaggct gactggccag 54540
tgagctgctg gggtccggcc tcccgtctct gtttcccagt tcttgattcc aagcctccat 54600
gtttttgttt ttgtttttta acctgggtgc tggagattaa actcaggaac tcctgcttct 54660
gtttctttgt tttgttttgt ttttgagaca gggtttctct gtgtagccct ggctgtccca 54720
gaactcactc tgtagaccag gctggctctc aactcagatc cacctgcctc tgtctcctgg 54780
gttcagggat tcatttaagg cctgcgccac ccctaccccc acctggttat gcttctctgt 54840
caagccatct atgtccccag cccatgacga ctaaccttgt caacctgaga ggatttagaa 54900
ttatgcagga gttggaagca tgcctctggg ggtgtctgtg aactgtgtta gaagaggatt 54960
aaccaatctg gaaagaccaa ccttaactgg ccatgtctac atcccttgag cctggagctt 55020
gatggaatca aagagggaca aggggcggcc acctcagcag cgacaagcag ctaactctgc 55080
ttcctgctct gtggtgacct gagcatgtgc caggtcccag ccatgtgttc ctagtgccac 55140
agggcctcgt gctgtcacac cttcccctcc acagtggact gtacaccctc acaccacgag 55200
cccaaataaa ttctttctcc atgaagttgt ttcttgtcgg gccacagtga taggaagagt 55260
aatgaaaaga ggcccctttg cactgggtcc actggtcctg gtggagtctg gatttgcagg 55320
atttcatcaa gaatgaagtg ttttggcctc ctaggatgtg caagggagtc cagatgttca 55380

catgacacag aaaccagaag agggcacaga catggagatg gccacacaca cacacacaca 55440

cacacacaca cacacacaca cacacaccac atatgctaca gatgggatgc cagaaaggag 55500

aacacttgcg catattaacg tgcacgtggt aaagcctggg aaggctggga cccagggtct 55560

gtttaggaga ggtgaaagat ggatcaagtt tctgcatagt gatggtgacc aggaagctgg 55620

gtcacacagc ttccttgaag tgggtatggc ctcttgcatg gaacttctgc tggggacact 55680

ggtggccccg agccccagga ggaacgtgtc cattggggca cttactagca ctgagatctt 55740

tatcctggtg accatggtga catagaggaa gatccacgtg gcaaagaggc acagggtcac 55800

gttcaggacc agcacctcta tcccttcttc catttgattg gaggcttgaa ggagagtgtg 55860

ataccagaaa tactggtgag gctctgacca aaggcaggta ttgcctgctg aggagggtag 55920

ggaggtccag acacttacag gccatctcac agggcatctc ccctagctat tgcacccctg 55980

tccctctcct taccaaccat gcttaagttt tctaccaagg ggcagttttg ccagggtaga 56040

gggtaatcga aggactggcc aaggtagaaa aggctccagg agaccagggc actgttatac 56100

aggaccacca agacacaagc ctgtggagaa gaaggtgggt ttgagggcat catggagaga 56160

gagaggacac tggggacttg ggtctctgcc agcccacact taccagtatg ttggagtagc 56220

ccacaccact aagccagggg accagatatt tccaggcctg gatgttgtcc atacgtagcc 56280

actgtccaat gaccatctcc atgtacatga gggggatgcc caggagcagg agaaggatga 56340

aatacatcag cagaaagctt cctgtggaga gagcagaggc ttgctcaggc agctgggaac 56400

ccctcatccc ttacattctt cccggggaga gggaggtgtc ttgtcacccc ttccttaatc 56460

tcagaacaca gtccctggcc tcaccgcctc cgttctggtg gcagaggtac gggaagcgcc 56520

acgtgctgcc gagtccgatg gagaaggcca ccaggattag gatattctcc gtcttcttag 56580

cctggaagtg gtttgggatc ttggtaacgc ggacctctct ggtcctgaag ccccgggaca 56640

cagatgtggg gttgtcgctc ttcttgcttg ctgtgtgcac ttcctccacg ggcttgttct 56700

tgagttctag aaagtcttca aaggattggt ccatggtgct ctctgcactg gctcgactcc 56760

tgatggggtt ttgaggtcac caaaaacgga ctttagggac agactgtggt gtggcaccag 56820

catccagaga ggcacaaggg gatggagggg cacaaagtat ttgctttata atgaattgtc 56880

ctggctgggg atgggggaca gataatcctg taaaggtgct tgctgccaaa tctaatgaca 56940

tgagttcaat tcctaggacc cacgttctgg aaggagggac cgaatcctga aagttgtcct 57000

atgacctcct tatgtgtcca gtggcatgtc tgtgttaaca cccacgcaca tagcacagca 57060

aatcaaatat ttaaaaagca tacatcatta gatgtttggg ttttatatag ctttggagtg 57120

tataggtttg tttggagaca gggtctcact agctcagaca ggtttcaagc aacgtatgtc 57180

gctgaggatg gcttgaaatg cctcatcctc cttcctccac ctcctgagtc ctggaatcac 57240

acagttttgc tttatagttt atagtttaaa aagaggcaga tgctcaggaa aaggccatgt 57300

gcttccaaac acatgccatt tatgtcacgg aaaatacaca aaaggaacca cacattttac 57360
agtggtcagc actatctggg gaccgaggag gcgggtcagc cactataaga atgcacactg 57420
ctcttgcagt caacccaagt tcagtgccca gaatccacct tgagtggctc acgaccatct 57480
gtgactccag ccccagggga cctgacaccc tccttctatc ctttgcagac acctgcaccc 57540
atgtgcacat acttagacat agacacacac acaatttaaa ataaaagtaa atctcaaaca 57600
atgaatgttc atggatatgc acacgtggag atcaaagctc aacaccatgt cttcctccat 57660
tgttaaccat cttactgttt cagacaacgc tagcacaacc tagtgctcat actatggcta 57720
aattggctaa ttagtgactt ctgggaaata cacctgtttc cacttcccca acatgagttt 57780
acagatcctg gccctgtgtc atacaccagg cttttatgtg ggtgctaggc agaaaactcc 57840
agtcttcgtg ctccgaacac actaagccct ctctccaccg gttttaaggc agcaccttac 57900
acattatacc cttcaagagc cagagtgtca gcaatcctga tttacatgca tacgcagcct 57960
tagatatgga tatgcctgat gttggtcctg agatcgcaga ctcaggcttt tacctcacta 58020
ggacacctca aggaaagcct ttggtcccat tccgcaggca aaaggcagcc agcatcttct 58080
ctgggaacag agatgtccag caaggtttga acttgtggct tgagagggag agtactattt 58140
gctaggtgag gtacctcctg gctgagtttt ttaattgggg gggggcccct aagtatacgt 58200
gcccatgggc cacaagagga tacctgatct cccggagctg atgatataag ctgttgggag 58260
tgcctatgtc tgtggtaggc ctcttagctg ctaagctctc tgcctctcca gttgtctggt 58320
tgacatttga agttttagtt taaggttcaa ggcctgtatg gtgttaaagg ataccatgat 58380
tttagctgag tgtgatggca cacccaccat cccagaactc ccaaggctga cctaggacta 58440
gcaagaattc aaggccaggt tgggatacac agtaagactt tgtctcaaga gtaaaaaaaa 58500
aaaaaagtta tatcatgtga ccttttattt tataacactg ggactgaaga aacttttgtc 58560
atagtatagt ggatatacac aggattgaaa cctatttatt aatatctaca tgtactgtat 58620
gaatttctca taaattatac tatatcataa gtattttcct atgccatatt tatttattat 58680
tttgtttcat gagacagggt ctctctgtat agctctggct tactaggaac ttgctagtag 58740
accaggcagg cctcaaactc ccagtgatcc tcctgcttct gcctcccaag tgcaggaatg 58800
ctcctactta ttgagacaag atctcactgt gtagctctag cggtcctgga cctacgtaga 58860
tcaactgact ccagaaatcc tcctgcctct gcacctgcag tgttaggatt aaaggagtac 58920
ggctccacgt ccagcttcaa agcttggttg ttaacagttg tacttcactc tccaaagtca 58980
ctacagaaac cttctggtac ctttggtcgg cttcctaaat cgagacagcc ccgccagcag 59040
cgtcccgacg gcgcccccgc gcggccagga aggaaacggc ggcctcgccg tggacgggct 59100
tccaagtggg cagggctagc ggtcgggact gactcagtgg tccgtacccg cctcagcgcg 59160

tgaacctcgg ccccgcgccg ctcgtgggcg gagagtccca cgtcgtcgcg gcccccgtgg 59220

tccgagaacc cgcgcgggaa gcggctactt ccctctctgt cccaacgagc tggcctgtga 59280

cgtcaccagg attccctgga gtctgccctg gggctgttag gtcacctgtt gccaggtagc 59340

tatacctcaa tgggctgagt gaggtcactg gttgccagtt agacttcaca cgcacacatg 59400

aaggagggaa aaaaaagatg ccaggaaaaa tgcccactgt cggggcacat agctatgatc 59460

ccagcgcttg agaggtagag aggtccgtga attccaggtc ctcttggaaa cgagactgag 59520

agataaataa aaagtaatga tagaaggagg aggaggagaa aagaaagcag aaaagaaaaa 59580

ggatgaaaca aaccaaagat aatcagaaga ggaaagtgta ggttccaatg ctatttttga 59640

aactatgtga ggaaatgtga gagaaagtag aagtgaaaga agagacagtc tccaaattta 59700

gaat 59704

<210> 2
<211> 13656
<212> DNA
<213> Artificial

<220>
<223> targeting vector pM4-21

<400> 2
gcatgctggg caagcactct acccagtggg tgctgccatc cccaccccta gttttgattt 60

gctgagacag ggtcttcctg tgtaacccag actggccagg accctgtgat tctcctgcct 120

catgaatgct gcctgtgaca caccccaccc ccacggccta cacacacaca cacacacaca 180

cacacacaca cacacacaca cactctgaat gttttaaaga tgtatgccat gagccgggtg 240

tggtggcgca cacctttaat ctcagcactt gggaggctga ggcaggtgaa tttctgagtt 300

tgaggacagt ctggtctaca cagtgagttc caggacagcc agggctacac agagaaaccc 360

tgtctggaaa aaacaaaaaa agatgtatac gatgatgaca ttatcttctc aaactgaatt 420

cagtgcccag gaaaagagcg atgcacagtg ctccgggccc ccactcggct ttctcacgcc 480

gttgctggta gcgaccctgt ctattggccg tgtgcatttg tttgggtgaa cccttggcct 540

taatgactct attaacttct gatcctccac aatcacaaga tttctctcct ttccttcccc 600

agatgtctaa cttcaccatg tcctctttct ctgtctcttc agggctccaa ctcagtggcc 660

tctgctcttg ggcttgtctt tttactccga gtgatggctc gcctagagtc ggaggctgag 720

gaggccgcgc ggcggaagga tcttgctgcc acgtttgaaa gcatgagcgt gggtgcgtgg 780

ggcagagtgc ctggggtgtg ggggtgcaca aggcacctcc cgcgtccttc catcctcatc 840

tgtatccttg tccccagacc tctttggaga gtgttaccac aacagtgaag agcgagcagc 900

ccgccttctt ctgcgccgct gtcccctctg gggagaggcc acctgcctcc agcttgccat 960

gcaggctgat gcccgagcgt tctttgccca ggatggagtc caggtgagcc tgtgactcag 1020

acagccgtga gcccccaccc cggagtccac ttatgcctag aacaattaca cttaacctct 1080

gagcacggaa cggccgtttc acagggaata tcatatattt gcattaagat tcataacagc 1140

aaatcccgtg gtggcggcac acgcctttaa tcccagcatt tacaggcaga gaggcaggtg 1200

aatctctgag tttggaggcc agcctggtct atagagaaag ttccaggata gccagagaca 1260

cacagagaaa ccttaaaaaa aaaaaaaaag tttttttttt tttttttttt tttgtaacag 1320

caaaattaca gttaaaaagt agcaatggga attttatagg gacttttcta ggtcatcaca 1380

gcattaagac cattgagaac cactgcctta gaaaatgtta cttcataaga tgaccaatca 1440

tctataactt ttcgaccctc cctaagtagt gttgttgcta ccaggaagta ccatttctca 1500

cacaagaagt tcctcatttt cacaggggag ttcctgtcct cctaaaggaa agaccacttc 1560

ttcaacagga agctctttcc attttttttt tttttttccc aagacagggt ttctctgtgt 1620

agccctggct gtcctggaac tcactttgta gaccaggctg gcctcgaact cagaaatccg 1680

cctgcctctg cctcccaagt gctgggatta aaggcatgcg ctagccttaa gataacttcg 1740

tatagcatac attatacgaa gttatgcatg cgtcatcact gcccggctaa tttttttttt 1800

aagatttatt tattattata cataagtaca ctgtagctgt cttcagatgc accagaagag 1860

ggcgtcagat ccgtcagatc tcattagggg tggttgtgag ccaccatgtg gctgctggga 1920

tttgaactta ggacctttgg aagagcagtc agtgctctta cccactgagc catctcacta 1980

gccctctttc tacttttttt ttttttttaa tgtgcattgg tgttttgcct gcatgtgaga 2040

gtgtgagatc acctggatct ggagtggcag acagttgtga gtgggcagct ctgctgattt 2100

ccctgtgtcg atctgttttt agtcaatctt gtatgtgtga gaaggagaaa atactcgccc 2160

ttttgggtta agtgatttgg tcaatgcaga gtgcttggca cagcacctgc cctatgatgt 2220

caataaatgt tggctgctct gcgcctgcag gaatgaatgg ttcactggtt aagggcacag 2280

gctgctcctg cagagaaagt ttagattcct accgcccata aggcagctca cagccatctg 2340

taactccttc tatgggctct ggtgccctct tcttacctat ctgggcacca ggcacgccat 2400

ggcgttaatc ccacggggtg agaataggat tactaccaca gtttttgagc tgaatttgga 2460

acacttttgt tttatagatc ttttaagcac aataatttaa acttaaaaca taatcttagc 2520

ccccacaatg gtgcacagac atgccggcag gcgaaacacc cacacacagt gaaagaaaaa 2580

ttggctgttt cgttttcctg gctgaacctg aggaggctgc ctctgacgac tgtgtcccat 2640

gctctaacag ttgagacccc atatggacac ttgctctgtg ccctggtggc agactccagc 2700

ccttgtcagg gtctctaatg ttctcacatt ccacagtctc ttctgacaca gaagtggtgg 2760

ggggagatgg acagcacaac tcccatctgg gccctgcttc tcgccttctt ctgcccgcct 2820

ctcatctaca ccaaccttat cgtcttcagg tcagtgcctt ggctccaggc tctgtccctc 2880

tctcctgctc atttctctct gccctcttac ttagccacct ggcatgtctc ttccctgaga 2940

agggcactct ttcccttctc tggagggaat gttctggaag gtgggggttg ttgtcctgcc 3000

tttgcgttat ctctctatgc gatttctcta ctttttaggc catggctctg gaggagggca 3060

actcccttat ctctctgcac tgtttgtggt aaactaagtt tgggtctttc tcaagaaccc 3120

ctctatttct tactctgtgt cctgcccctc acggcaggaa gtcagaggag gagcccacac 3180

agaaggatct tgactttgat atggacagca gcatcaatgg agcaggtcct cctgggtgag 3240

tgacagtcca gcattgtggg aggagggggg agggcgccat catggacagt ttcaggatct 3300

tgccaactcc cacttccatc aggcactgta gcaagtcctg aactagggga gcaatgggct 3360

tttaggagtt gctggtctca ctgaaccaca gaagatgccg aggtaagttc tagaaggcca 3420

tgctcaagga ctgacttaaa atttggtcct tggagccaac gagatggctc tgcaggtaaa 3480

tctactcaag tggaaggacc tgagttcaag agagaaacaa ttctttttct aggatataac 3540

tacactgtag ttgtcttcag acacacacta gaagagggca tcggatccca ttacagatgg 3600

ttgtgagcca ccatgtggtt gctgggaatt gaacccagga cctctggaag cacagtcagt 3660

gctcttaact gctgggcctc tctccagccc tggagaaaca attcttaaat gctcctgact 3720

tccacctttc ttagttcacc cattggagtg cagagcgcca ggggcagaag gatgatgtca 3780

tcacaagaac ttagtttggg ttgggcgacc tgcgctccct tgctgttggt tacttagtat 3840

ttattcactt ttctattatg tttctgtccc tggggctctt gccctgtcac tatgttgcag 3900

tccccatgga gatcttttaa agaccaggct caatacgtat agttgctaag tatttgtcta 3960

agctcctcat agccctaggt tccatcccag gccaaacaac acaaaaccta caggaaacct 4020

cggggagtca gccagttagg tgcctagcgc acaagcataa agagctgagt tcagactccc 4080

agcctctgta ataaaatcta aggtgtggca acgcatgcgc tagccttaag tgatccataa 4140

cttcgtatag catacattat acgaagttat tgatcaaatt atgcagagac atctctattt 4200

ctcgctattg atcggtgttt atttattctt taaccttcca ccccaacccc ctccccagag 4260

acaccatgac tgctccaaag aagaagcgta aggtaccggt gggtgaagac cagaaacagc 4320

acctcgaact gagccgcgat attgcccagc gtttcaacgc gctgtatggc gagatcgatc 4380

ccgtcgtttt acaacgtcgt gactgggaaa accctggcgt tacccaactt aatcgccttg 4440

cagcacatcc ccctttcgcc agctggcgta atagcgaaga ggcccgcacc gatcgccctt 4500

cccaacagtt gcgcagcctg aatggcgaat ggcgctttgc ctggtttccg gcaccagaag 4560

cggtgccgga aagctggctg gagtgcgatc ttcctgaggc cgatactgtc gtcgtcccct 4620

caaactggca gatgcacggt tacgatgcgc ccatctacac caacgtgacc tatcccatta 4680

cggtcaatcc gccgtttgtt cccacggaga atccgacggg ttgttactcg ctcacattta 4740

atgttgatga aagctggcta caggaaggcc agacgcgaat tatttttgat ggcgttaact 4800

cggcgtttca tctgtggtgc aacgggcgct gggtcggtta cggccaggac agtcgtttgc 4860

cgtctgaatt tgacctgagc gcatttttac gcgccggaga aaaccgcctc gcggtgatgg 4920

tgctgcgctg gagtgacggc agttatctgg aagatcagga tatgtggcgg atgagcggca 4980

ttttccgtga cgtctcgttg ctgcataaac cgactacaca aatcagcgat ttccatgttg 5040

ccactcgctt taatgatgat ttcagccgcg ctgtactgga ggctgaagtt cagatgtgcg 5100

gcgagttgcg tgactaccta cgggtaacag tttctttatg gcagggtgaa acgcaggtcg 5160

ccagcggcac cgcgcctttc ggcggtgaaa ttatcgatga gcgtggtggt tatgccgatc 5220

gcgtcacact acgtctgaac gtcgaaaacc cgaaactgtg gagcgccgaa atcccgaatc 5280

tctatcgtgc ggtggttgaa ctgcacaccg ccgacggcac gctgattgaa gcagaagcct 5340

gcgatgtcgg tttccgcgag gtgcggattg aaaatggtct gctgctgctg aacggcaagc 5400

cgttgctgat tcgaggcgtt aaccgtcacg agcatcatcc tctgcatggt caggtcatgg 5460

atgagcagac gatggtgcag gatatcctgc tgatgaagca gaacaacttt aacgccgtgc 5520

gctgttcgca ttatccgaac catccgctgt ggtacacgct gtgcgaccgc tacggcctgt 5580

atgtggtgga tgaagccaat attgaaaccc acggcatggt gccaatgaat cgtctgaccg 5640

atgatccgcg ctggctaccg gcgatgagcg aacgcgtaac gcgaatggtg cagcgcgatc 5700

gtaatcaccc gagtgtgatc atctggtcgc tggggaatga atcaggccac ggcgctaatc 5760

acgacgcgct gtatcgctgg atcaaatctg tcgatccttc ccgcccggtg cagtatgaag 5820

gcggcggagc cgacaccacg gccaccgata ttatttgccc gatgtacgcg cgcgtggatg 5880

aagaccagcc cttcccggct gtgccgaaat ggtccatcaa aaaatggctt tcgctacctg 5940

gagagacgcg cccgctgatc ctttgcgaat acgcccacgc gatgggtaac agtcttggcg 6000

gtttcgctaa atactggcag gcgtttcgtc agtatccccg tttacagggc ggcttcgtct 6060

gggactgggt ggatcagtcg ctgattaaat atgatgaaaa cggcaacccg tggtcggctt 6120

acggcggtga ttttggcgat acgccgaacg atcgccagtt ctgtatgaac ggtctggtct 6180

ttgccgaccg cacgccgcat ccagcgctga cggaagcaaa acaccagcag cagtttttcc 6240

agttccgttt atccgggcaa accatcgaag tgaccagcga atacctgttc cgtcatagcg 6300

ataacgagct cctgcactgg atggtggcgc tggatggtaa gccgctggca agcggtgaag 6360

tgcctctgga tgtcgctcca caaggtaaac agttgattga actgcctgaa ctaccgcagc 6420

cggagagcgc cgggcaactc tggctcacag tacgcgtagt gcaaccgaac gcgaccgcat 6480

ggtcagaagc cgggcacatc agcgcctggc agcagtggcg tctggcggaa aacctcagtg 6540

tgacgctccc cgccgcgtcc cacgccatcc cgcatctgac caccagcgaa atggattttt 6600

gcatcgagct gggtaataag cgttggcaat ttaaccgcca gtcaggcttt ctttcacaga 6660

tgtggattgg cgataaaaaa caactgctga cgccgctgcg cgatcagttc acccgtgcac 6720

cgctggataa cgacattggc gtaagtgaag cgacccgcat tgaccctaac gcctgggtcg 6780

aacgctggaa ggcggcgggc cattaccagg ccgaagcagc gttgttgcag tgcacggcag 6840

atacacttgc tgatgcggtg ctgattacga ccgctcacgc gtggcagcat caggggaaaa 6900

ccttatttat cagccggaaa acctaccgga ttgatggtag tggtcaaatg gcgattaccg 6960

ttgatgttga agtggcgagc gatacaccgc atccggcgcg gattggcctg aactgccagc 7020

tggcgcaggt agcagagcgg gtaaactggc tcggattagg gccgcaagaa aactatcccg 7080

accgccttac tgccgcctgt tttgaccgct gggatctgcc attgtcagac atgtataccc 7140

cgtacgtctt cccgagcgaa aacggtctgc gctgcgggac gcgcgaattg aattatggcc 7200

cacaccagtg gcgcggcgac ttccagttca acatcagccg ctacagtcaa cagcaactga 7260

tggaaaccag ccatcgccat ctgctgcacg cggaagaagg cacatggctg aatatcgacg 7320

gtttccatat ggggattggt ggcgacgact cctggagccc gtcagtatcg gcggaattcc 7380

agctgagcgc cggtcgctac cattaccagt tggtctggtg tcaaaaataa taataaccgg 7440

gcaggccatg tctgcccgta tttcgcgtaa ggaaatccat tatgtactat ttaaaaaaca 7500

caaacttttg gatgttcggt ttattctttt tcttttactt ttttatcatg ggagcctact 7560

tcccgttttt cccgatttgg ctacatgaca tcaaccatat cagcaaaagt gatacgggta 7620

ttatttttgc cgctatttct ctgttctcgc tattattcca accgctgttt ggtctgcttt 7680

ctgacaaact cggaacttgt ttattgcagc ttataatggt tacaaataaa gcaatagcat 7740

cacaaatttc acaaataaag catttttttc actgcccaga tctgaagttc ctattccgaa 7800

gttcctattc tctagaaagt ataggaactt cttaattaag ttttcgaggg cccctgcagg 7860

tcaattctac cgggtagggg aggcgctttt cccaaggcag tctggagcat gcgctttagc 7920

agccccgctg ggcacttggc gctacacaag tggcctctgg cctcgcacac attccacatc 7980

caccggtagg cgccaaccgg ctccgttctt tggtggcccc ttcgcgccac cttctactcc 8040

tcccctagtc aggaagttcc cccccgcccc gcagctcgcg tcgtgcagga cgtgacaaat 8100

ggaagtagca cgtctcacta gtctcgtgca gatggacagc accgctgagc aatggaagcg 8160

ggtaggcctt tggggcagcg gccaatagca gctttgctcc ttcgctttct gggctcagag 8220

gctgggaagg ggtgggtccg ggggcgggct caggggcggg ctcaggggcg gggcgggcgc 8280

ccgaaggtcc tccggaggcc cggcattctg cacgcttcaa aagcgcacgt ctgccgcgct 8340

gttctcctct tcctcatctc cgggcctttc gacctgcagc caatatggga tcgcccattg 8400

aacaagatgg attgcacgca ggttctccgg ccgcttgggt ggagaggcta ttcggctatg 8460

actgggcaca acagacaatc ggctgctctg atgccgccgt gttccggctg tcagcgcagg 8520

ggcgcccggt tctttttgtc aagaccgacc tgtccggtgc cctgaatgaa ctgcaggacg 8580

aggcagcgcg gctatcgtgg ctggccacga cgggcgttcc ttgcgcagct gtgctcgacg 8640

ttgtcactga agcgggaagg gactggctgc tattgggcga agtgccgggg caggatctcc 8700

tgtcatctca ccttgctcct gccgagaaag tatccatcat ggctgatgca atgcggcggc 8760

tgcatacgct tgatccggct acctgcccat tcgaccacca agcgaaacat cgcatcgagc 8820

gagcacgtac tcggatggaa gccggtcttg tcgatcagga tgatctggac gaagagcatc 8880

aggggctcgc gccagccgaa ctgttcgcca ggctcaaggc gcgcatgccc gacggcgatg 8940

atctcgtcgt gacccatggc gatgcctgct tgccgaatat catggtggaa aatggccgct 9000

tttctggatt catcgactgt ggccggctgg gtgtggcgga ccgctatcag gacatagcgt 9060

tggctacccg tgatattgct gaagagcttg gcggcgaatg ggctgaccgc ttcctcgtgc 9120

tttacggtat cgccgctccc gattcgcagc gcatcgcctt ctatcgcctt cttgacgagt 9180

tcttctgagg ggatcgatcc gctgtaagtc tgcagaaatt gatgatctat taaacaataa 9240

agatgtccac taaaatggaa gtttttcctg tcatactttg ttaagaaggg tgagaacaga 9300

gtacctacat tttgaatgga aggattggag ctacgggggt gggggtgggg tgggattaga 9360

taaatgcctg ctctttactg aaggctcttt actattgctt tatgataatg tttcatagtt 9420

ggatatcata atttaaacaa gcaaaaccaa attaagggcc agctcattcc tcccactcat 9480

gatctataga tctatagatc tctcgtggga tcattgtttt tctcttgatt cccactttgt 9540

ggttctaagt aaacgaagtt cctattccga agttcctatt ctctagaaag tataggaact 9600

tcgtcgaggg cgcgccctta agataacttc gtatagcata cattatacga agttatacta 9660

gtgctgctag acagagaagg aagggtccct ggggtgttgt gggccagtcg ggatagccga 9720

atcagcaagt cctgtgttta aaaataagag tgaaaacgac tgaaggagac atcaaacccc 9780

aacctctcac gtcttagtct ggctgataaa acgattttga aatggactca gaaggaagct 9840

tgggaccctt ttattagatg ctgagagaaa aggaacagag caggcgacta tgaagaggaa 9900

gggaggaagc cactcctttt gctggggagg ttagaaaaca gtagcatgct ggtgagaagg 9960

gacgggtggc ccagagcacc aaggaaatta agtctaggct ttgggaagaa tccaaaacaa 10020

acaaagacaa agaaacggag aaggaaagat tgtttttctg agttaggagt cagaaaatcg 10080

ggcaggttgc atgtggagga gtaggattgc ggtctccagg gaaacaaaag taaactgtat 10140

cgtattgtat catttaggag ataattaatc tttccctctc cttcaagtga ctcagactcc 10200

ctggggcaga gaaagactct gggacaagtg actggcaagc tgtgctggtt taacccttta 10260

gacactttaa aagattttat ttttaattat gtgtgtacat gtggatttgt gtgttgtagg 10320

gtctatgcat gtggttgccc tctgagacca ggactgggaa ttgtcacaca cacacacaca 10380

cacacacaca cacacacaca cacacacaca cacaccatga caaaccaacc aaccaacccc 10440

cacagctgcc aaacggaata agagacagct tattctcgag ccagatgtga gtgacctaag 10500

tgtggaaaca cggggctaag ttagttctat gtcccaacgt ggaaactgtt tcatggagtt 10560

tttatagtaa cagaacaaag aaagttataa atcaaagcac ttaaaaatac agtggtagaa 10620

atattaagtg agcagacagg gcaggcagga gaatcgcagc cttgggctca gatcctatct 10680

tactgcattc ttaggtgtct ttagggtttt gtgacattag tacattgcaa aaatttgtat 10740

ctgtttatca agatgttagc tatgagactg aggtaggctg gaggttaaag agagctcaaa 10800

agaaaataaa ctatctgata gtaaagatag ctcaaaagaa attagactat ctgatactaa 10860

caaccaggtt ctgcagtctc tgaagtcttt atcagttgat cagcctctgc agacagtgtc 10920

tttctaggaa ttcccagtga cagagtcaaa cccgatccag tgctcagaac tgctggatca 10980

attcttcagc cccttaaagt ttttctaaat tagattttat tttgttattt gtacattgtt 11040

ttttgagggt gtgtatgctg cccgcaaggg tgtggagtcc agaggacaag ttgtagaagc 11100

cggttccctc ctttcacctt ggcgaccaag gtcatgtggc agacgcctgt gtttgctgag 11160

tctcctcctt cctgggtcat aagaggaggc aacgatgcta tgtgatgttt ggagtagttt 11220

agaatgtcct gtctccatgc aaggccagat gaagatcgtt gggagcacaa gggtcctgac 11280

acccacagat catcagggaa gctaggaatg ttatacacac gggggccctc ttctcagtgg 11340

aggagagaaa attcctgttt ttctgcccag atgcctggga atggattttg taaataacct 11400

ggtgaccttt ctgctctctg tggaggcaga cctcacccaa atcccccaga ctgcctatcc 11460

cagactctct cttcctagac cattgcccat gctttggggg agaggtcgaa tgtacttcat 11520

ggccactgac ctctatgcta ttagtcagag accacactac attctaggcc ttttagctat 11580

aggacctggc ctcctggtca catgtatttc ataaaagcat ttctaggtgt cacacagtaa 11640

gcttactgtg cacctggaat tgaacttagg aactgattgt tgcctggaaa ccttttccca 11700

gattggactg tgtttttctt ttgtttgttt gttctgtttt tgttgttgtt gtgttttttt 11760

ggtttttcga gacagggttt ctctgtgtag ccctggctgt cctggaactc acttagtaga 11820

ccaggttggc ctcgaactca gaaatctgcc tgcctcccaa gtgctaggat taaagatgtg 11880

cgccaccact gcccggctat ggactgtgtt ttaaatatgc tgacagtggc cctagcatta 11940

aactcttcgc atcaacaatc agcccacact ggcttttcaa tctcatcagt ttgcgggtca 12000

cggcctgcat ggacacctac cgggtccccc tcaatacagc tcacattcta gttttttgac 12060

caggaggaag tagatggacc ccagcaaagc tcacacgccc ccaccttttc atgtggttta 12120

agatctaaag cactccaagg ctcctgcaac attctgcatg tttctggtca gttttaaaga 12180

cagctattgt gctcagtggt ttgttgttgt tgtgtggatt gttttttgtt tgttttgcat 12240

aaggtttctt tctatatcct tggatggctt ggagctgact aggtagacag cactgcctcg 12300

ccgggatcct ttgcctctgc tgggacaaac catgctgggt ttatgctcag ttttgtttct 12360

ttctttgatg tttgttgtgt ttcttcacaa agctcaggct agcatcaaac acctagcaat 12420

cctcctgcct catctctcga agtgtaatga atcctcattc catgttttat gttccttctt 12480

ccctcactct atgtatcagg ctggcctgaa acttgaggca gttctcctgc ctcaatctca 12540

aacgtgggat tgactgggtc ttcaggttcc cagagtccaa agtgggacag tgtagaagtt 12600

agactgcaag atgggcagcc agtccaggct cattggtccc tgaagctcgc tgtcttgcag 12660

gactgtagaa ccctctgcaa aggtggccct tgagaggcgg cagcggcgac ggccaggacg 12720

tgccctctgc tgtggcaagt tctccaagcg ctggtcggac ttctggggtg ccccagtgac 12780

tgccttcctg ggtaatgtgg tcagttacct gctgttcctg ctgctgtttg cccacgtgtt 12840

gctggtggat ttccagccca caaagccaag tgtctccgag ctgctgctgt acttctgggc 12900

cttcacgttg ctgtgcgagg agctgcgcca gggcctgggt ggtggctggg gtagcctggc 12960

cagtggggga cgtggtccgg acagagctcc cctgcgccat cggctgcacc tctacctctc 13020

agatacctgg aaccaatgtg acctgctggc actcacctgc ttcctgctgg gcgtaggctg 13080

ccggtgagtg agtaagagac tggcagggcc cctgcctaga atcccccagt gagaggatgt 13140

ggctatggct cagtggtgga gtaccctggg tgatggagag tcacctacgt ccccctttac 13200

tgaagcgatc ctcagctggc ataccaagag gtagatttcc tccagtccat gtgcgtttga 13260

aacagagtaa cttggtatga ataatttatc caaacagaag ggagataagt ggacttagca 13320

aactaatatt tctgcttgtc ccagccttta tttagtgtta tgtgttgtct gagaagacag 13380

ggccacggcc ctgccgattg caggaaagcc actgtcaagg gtatatttga tacattttat 13440

caagcacccc tttccaaggg ctgacaaatc attgcatcta gggaggaaga aacactgtca 13500

catccagtat aattggcttg tcctccttta agcccgtgcc ctccagatcc acacttaggt 13560

tattgcaaaa caaaaaacaa aaccgaataa aaccaagtca cgtccctggt gtaaagtggg 13620

tggggtcaaa ggccgggtgg atagtttcat ccgcgg 13656

## Description

The present invention provides TRPM4-deficient non-human mammals. The invention further provides the production of such non-human mammals and its use as a test model for developing therapeutic strategy in allergy and hypersensitivity reactions.

### Background of the Invention

Mast cells are key effector cells in allergic reactions and release inflammatory mediators after antigen-mediated FceRI receptor aggregation and influx of Ca²⁺. Here we show that TRPM4 proteins constitute Ca²⁺-activated non-selective cation channels in bone-marrow derived mast cells (BMMC) and critically determine the driving force for Ca²⁺ influx, i.e. TRPM4^{-/-} mast cells display increased Ca²⁺ entry upon FceRI receptor stimulation. Consequently, degranulation and release of histamine from TRPM4^{-/-} BMMC's is augmented as well as secretion of leukotrienes and tumor necrosis factor a. TRPM4^{-/-} mice display a more severe IgE-mediated acute passive cutaneous anaphylactic (PCA) response, whereas the late-phase PCA response is not affected. These results establish the physiological role of TRPM4 channels as critical regulators of Ca²⁺ entry in mast cells and for the development of type I hypersensitivity reactions.

Mast cells are bone-marrow derived haematopoietic cells that are especially localized near surfaces exposed to the environment, such as the skin, the airway epithelia and the intestine, where pathogens, allergens and other environmental agents are frequently encountered (Galli et al., Nat. Immunol. 6:135-142 (2005)). Activation and degranulation of mast cells is a key step in the pathogenesis of allergic diseases such as bronchial asthma and systemic anaphylaxis (Metcalfe, D.D. et al., Physiol. Rev., 77:1033-1079 (1997)). In short, an allergic reaction develops when allergens encountered by antigen-presenting cells are processed and presented to T-cells, eliciting a T helper 2 response that causes B-cells to produce allergen-specific IgE. The IgE molecules function as receptors for antigens and bind to the FceRI receptor on the cell surface of mast cells. Upon renewed exposure to the allergen, receptor-associated IgE's will bind it and subsequent cross-linking of the FceRI receptors will activate mast cells, resulting in: (i) degranulation, i.e. the secretion of preformed mediators that are stored in cytoplasmic granules of the cell, including proteases and vasoactive amines such as histamine, (ii) the de novo synthesis of pro-inflammatory lipid mediators and (iii) the de novo synthesis and secretion of cytokines and chemokines. The instant release of histamine is crucial for the development of immediate-type allergic reactions leading to vasodilatation, increased vascular permeability and smooth muscle contraction (Galli et al., Nat. Immunol. 6:135-142 (2005); Metcalfe et al., Physiol. Rev., 77:1033-1079 (1997)). In addition, IgE-dependent mast cell activation may be complemented by signaling cascades triggered by several endogenous ligands such as adenosine resulting in amplification and maintenance of FceRI receptor-mediated degranulation (Laffargue et al., Immunity, 16:441-451 (2002); Tilley et al., J. Immunol., 171:331-337 (2003)). As such, adenosine-induced bronchial constriction is a well-recognized feature aggravating the disease symptoms in allergic asthma (Jacobson, K.A., Gao, Z.G., Nat. Rev. Drug Discov., 5:247-264 (2006)).

The FceRI receptor is a multisubunit receptor consisting of a ligand binding a subunit, a signal-amplifying membrane-spanning ß subunit and a homodimeric disulfide-linked. subunit that provides the signaling ability of this receptor. Crosslinking of the FceRI receptor results in activation of Syk kinase and Src family protein tyrosine kinases including Lyn and Fyn, which in turn phopshorylate a range of adaptor and scaffolding proteins (Blank, U., Rivera, J., Trends Immunol. 25:266-273 (2004); Gilfillan, A.M., Tkaczyk, C., Nat. ev. Immunol., 6:218-230 (2006)). A major downstream target is phospholipase C.1, which catalyzes the hydrolysis of phosphatidylinositol(4,5)P2 to diacylglycerol and inositol(1,4,5)P3 (Gilfillan A.M., Tkaczyk, C., Nat. ev. Immunol., 6:218-230 (2006)). Adenosine stimulation, on the other hand, involves activation of Gai-coupled A3 adenosine receptors in mouse mast cells, which also leads to activation of phospholipase C via Gß.and phospho-inositide 3-kinase .(Jacobson, K.A., Gao, Z.G., Nat. Rev. Drug Discov., 5:247-264 (2006)). Inositol(1,4,5)P3 and diacylglycerol promote protein kinase C activation and release of Ca²⁺ from intracellular stores followed by influx of Ca²⁺ from the extracellular space (Foreman et al., J. Physiol., 271:193-214 (1977)) through a Ca²⁺ entry pathway named Ca²⁺-release activated Ca²⁺ channel (Hoth, M. Penner, R., Nature, 355:353-356 (1992)). Ca²⁺ influx is indispensable for mast cell activation and degranulation, and depends critically on the membrane potential, which provides the driving force for Ca²⁺ entry (Turner, H., Kinet, J.P., Nautre, 402:B24-30 (1999)).

TRPM4 is a Ca²⁺-activated and voltage-dependent Ca²⁺ impermeable monovalent cation channel (also named CAN channel) belonging to the melastatin subfamily of transient receptor potential (TRP) membrane proteins (i.e., TRP-Melastatin4; Hofmann, T. et al., Curr. Biol., 13:1153-1158 (2003); Launay, P. et al., Cell, 109:397-407 (2002); Nilius, B. et al., J. Biol. Chem., 278:30813-30820 (2003); Nilius B., Vennekens, R., Journal of Physiology PMID: 16680483 (2006)). Further methods for screening for TRPM4 modulators are known from WO 04/039941.

### Summary of the Invention

It was now found that TRPM4 currents are pivotal determinants of mast cell activation, inflammatory mediator release and immediate-type allergic reactions in vivo by setting the membrane potential and thereby modulating Ca²⁺ influx. Inactivation of the TRPM4 gene in mammals such as mice results in increased mast cell degranulation. TRPM4-deficient (TRPM4^{-/-}) cells show larger influx of Ca²⁺ upon cross-linking of the FceRI receptor compared to wild type mast cells, due to altered regulation of the cell membrane potential after receptor activation. This has a profound impact on mast cell activation. Activated TRPM4^{-/-}cells release more histamine, leukotrienes and tumor necrosis factor a. TRPM4-/-animals display a more severe acute anaphylactic response in the skin compared to wild type controls. In summary, it was found that TRPM4 channel activation is an efficient mechanism for limiting antigen-induced mast cell activation *in vivo* and that this channel represents a new target for the treatment of allergic diseases. TRPM4 are indispensable constituents of Ca²⁺-activated cation channels. The invention thus provides
(1) a TRPM4-deficient non-human animal;
(2) a tissue or cell culture derived from the non-human animal of (1) above;
(3) a process for preparing the non-human animal as defined in (1) above, which comprises transfecting cells with a targeting vector comprising 5' and 3' homology arms flanking the selection of the TRPM4 gene to be inactivated;
(4) a targeting vector as defined in (3) above;
(5) the use of the non-human animal as defined in (1) above or the tissue or cell culture as defined in (2) above as a test system for allergy and hypersensitive reactions, especially as a test system for validation or evaluation of substances with respect to their specificity for TRPM4 channels, as test system for TRPM4-mediated reactions, and for identifying substances acting TRPM4 proteins or on protein complexes containing TRPM4 proteins.

### Detailed Description of the Figures

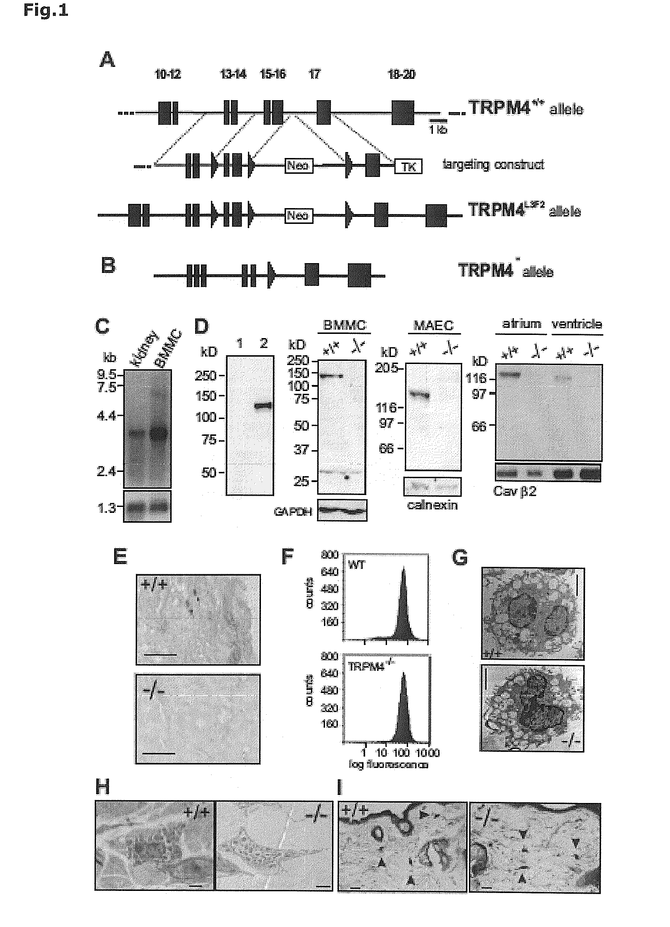

Figure 1: Targeted disruption of the TRPM4 gene and bone marrow derived mast cell culture from TRPM4^{-/-} and WT mice. We used the Cre-loxP strategy to excise exons 15 and 16, encoding the first transmembrane spanning domain of TRPM4. (A) WT TRPM4-allele, targeting construct and recombinant TRPM4L3F2 allele. Translated exons (not in scale) and introns are shown as filled boxes and lines. The TRPM4L3F2 allele contains one IoxP site (filled triangles) upstream and a IoxP flanked PGKneo cassette downstream of exon 16. (B) Cre mediated conversion of the TRPM4L3F2 allele to the TRPM4-allele in mice. (C) Northern blot of poly(A)+ RNA isolated from kidney and BMMC from WT mice hybridized with a mTRPM4-specific probe. Transcripts of ~4.2 kb were identified in both tissues. (D) Western blot of protein fractions from non-transfected (lane 1) and mouse TRPM4-transfected (lane 2) HEK 293 cells (left panel) and WT (+/+) and TRPM4^{-/-}(^{-/-}) BMMC's, mouse aortic endothelial cells (MAEC), cardiac atrium and ventricle (right panels) using TRPM4-specific antibody 578. (E) Immunostainings using TRPM4-specific antibody 578 in skin biopsies (bar 50 µm) from WT and TRPM4^{-/-} mice. Arrows indicate stained mast cells; no staining is observed in biopsies from TRPM4-^{/-}mice. (F) Cell surface expression of the FceRI in BMMC from WT and TRPM4^{-/-}mice was assessed by FACS. Similar expression of FceRI was observed in BMMC cultures from both genotypes. (G, H) Ultrastructural analysis of cultured BMMC cells (G) and skin biopsies (H) from WT (+/+) and TRPM4^{-/-}mice (bars = 2 µm). (I) Representative May-Grünwald-Giemsa stainings of skin biopsy cross-sections showing distribution of tissue mast cells (arrowheads) in the skin of WT and TRPM4-/- mice.

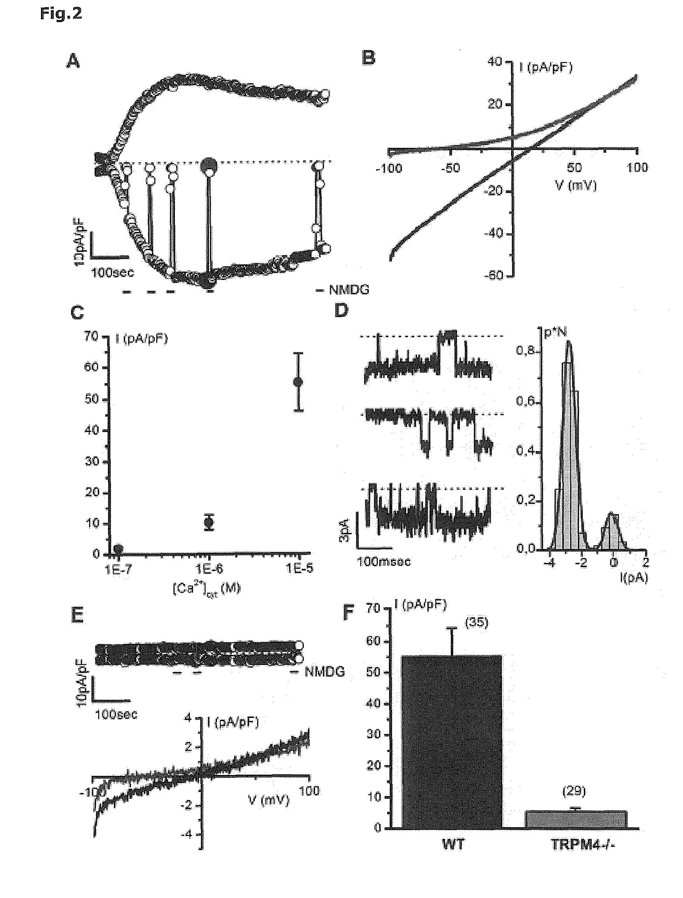

Figure 2: (A) Ca²⁺-activated non-selective cation channel (CAN) in WT mast cells is absent in TRPM4^{-/-}mast cells. (A) Representative time-course of whole-cell CAN currents at -80 and +80 mV in WT BMMCs. Cells are dialysed with a pipette solution containing 10 µM Ca²⁺. Bars indicate perfusion with NMDG-containing bath solution. (B) Representative current traces from WT BMMC cells in response to a voltage ramp from -100 to +100 mV, using standard (black trace) or NMDG-containing (blue trace) bath solution. Conditions as in (A). (C) Mean current densities of CAN currents at -80 mV in response to different [Ca²⁺]cyt. (D) Single channel openings recorded at -100 mV from inside-out membrane patches. The cytosolic side was exposed to 300 µM Ca²⁺. The dotted line represents the zero current level. A representative amplitude histogram is shown. Mean single channel conductance is 25.3±0.7 pS (n=5). (E) Representative time-course and current traces as in (A) and (B) of CAN currents in TRPM4^{-/-}BMMC's. (F) Mean current densities measured at -80 mV in WT and TRPM4^{-/-} mast cells, as in A.

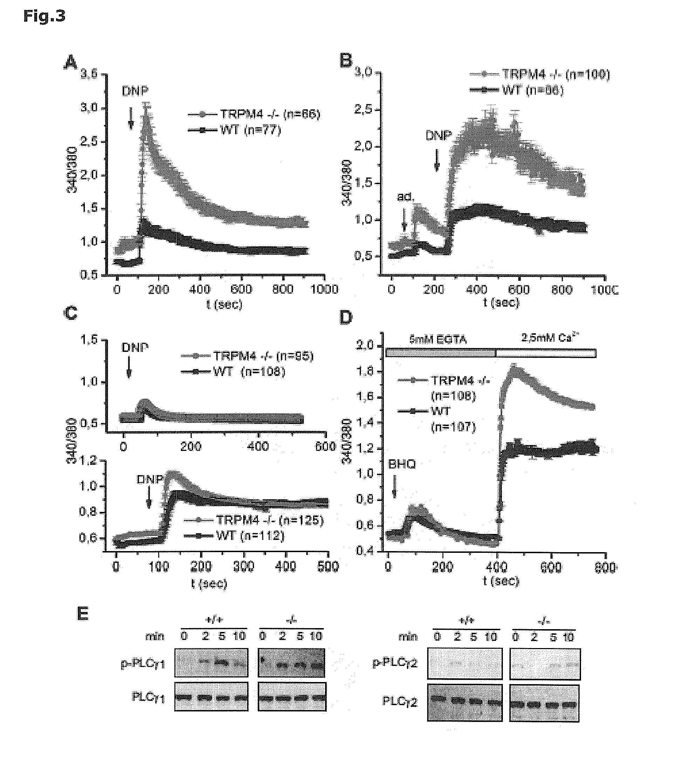

Figure 3: Ca²⁺ signalling in WT and TRPM4^{-/-} BMMCs. Mean traces for time dependent change of [Ca²⁺]cyt in FURA-2 loaded WT and TRPM4^{-/-} BMMCs. Fluorescence ratio 340/380 is shown. Number of cells is indicated in brackets. All measurements are representative for at least three independent experiments. (A) Averaged time course of [Ca²⁺]cyt after application of DNP (100 ng/ml) to anti-DNP IgE pre-treated WT and TRPM4^{-/-} BMMC's. (B) Averaged time course of [Ca²⁺]cyt after application of adenosine (10 µM) followed by DNP (100 ng/ml) to anti-DNP IgE pre-treated WT and TRPM4^{-/-}BMMC's. (C) Averaged time course of [Ca²⁺]cyt after stimulation of IgE pre-treated WT and TRPM4^{-/-} BMMCs as in panel A in the absence of Ca²⁺ (5 mM EGTA) in the extracellular medium (upper panel) and with extracellular Na⁺ replaced by K⁺ (lower panel). (D) Averaged time course of [Ca²⁺]cyt upon depletion of intracellular Ca²⁺ stores with tBHQ (20 µM) in Ca²⁺ free/5 mM EGTA solution and subsequent addition of 2.5 mM Ca²⁺ bath solution. (E) PLC. phosphorylation in TRPM4^{-/-} BMMCs after FceRI stimulation. BMMCs were stimulated with DNP for the indicated time. PLC. phosphorylation was determined by Western blot with anti-phospho- PLC.1, anti-PLC.1, anti-phospho- PLC.2 and anti-PLC.2 antibodies.

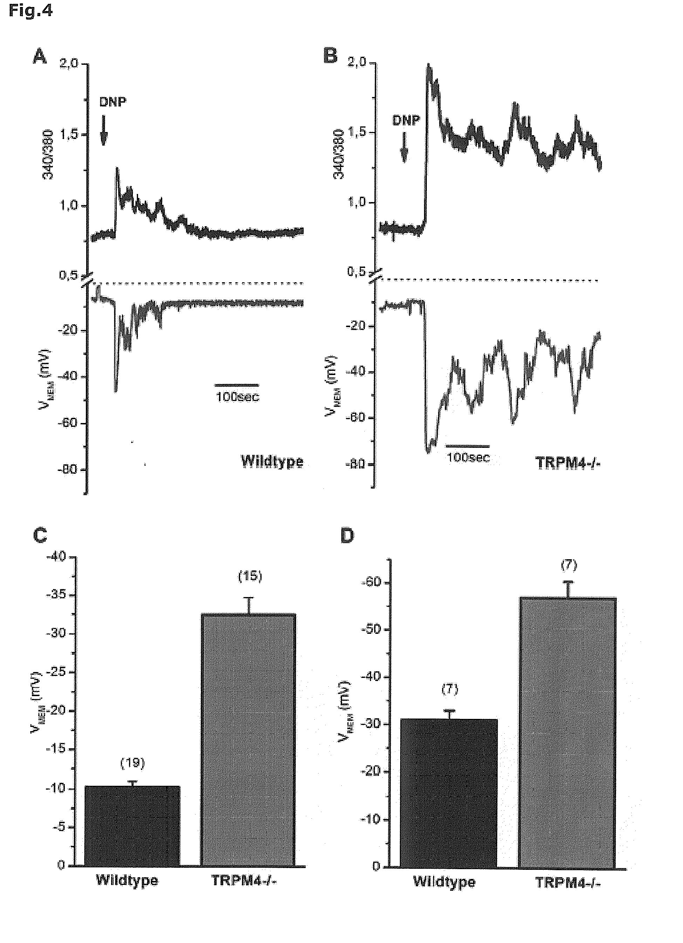

Figure 4: Simultaneous membrane potential and [Ca²⁺]cyt measurements from WT and TRPM4^{-/-} BMMC. Measurements were performed in the whole-cell current clamp mode using perforated patches. (A-B) Simultaneous measurement of [Ca²⁺]cyt and membrane potential after challenging anti-DNP IgE pre-treated BMMCs from WT (A) and TRPM4^{-/-} (B) mice with 100 ng/ml DNP. (C-D) Pooled data for mean membrane potential during a 10min measurement after stimulation with 100 ng/ml DNP (C) or with 10 µM adenosine and 100 ng/ml DNP (D). See also Figure 113.

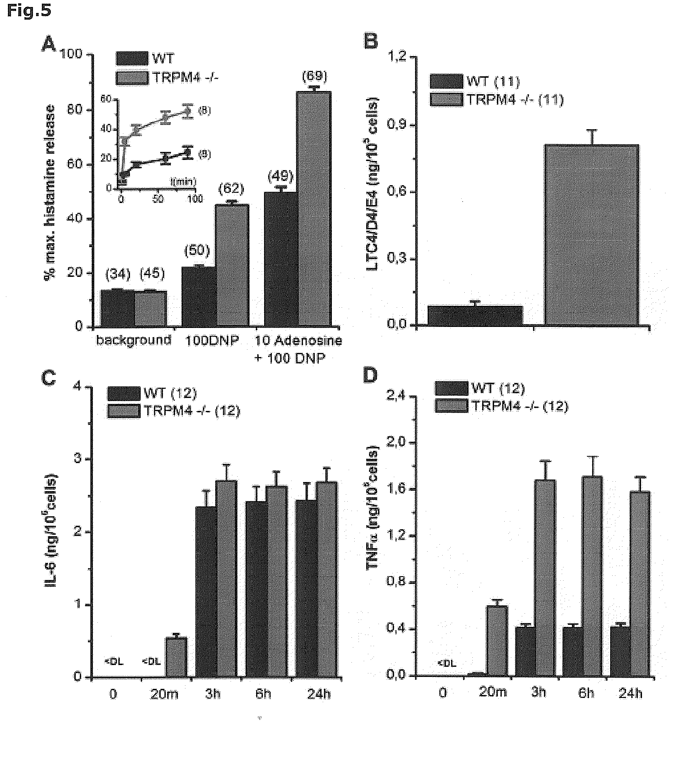

Figure 5: Mediator release from WT and TRPM4^{-/-}mast cells. (A) Averaged histamine release from WT and TRPM4^{-/-} anti-DNP IgE pretreated mast cells without (background) or with stimulation with DNP (100 ng/ml) or with adenosine (10 µM) and DNP (100 ng/ml). Inset: Averaged time-course of histamine release from WT and TRPM4^{-/-} mast cells following stimulation with DNP (100 ng/ml). (B) Leukotriene secretion from WT and TRPM4^{-/-}anti-DNP IgE pretreated mast cells after stimulation with DNP (100 ng/ml). (C, D) IL-6 (panel C) and TNFα (panel D) secretion from WT and TRPM4^{-/-} anti-DNP IgE pretreated mast cells after stimulation with DNP (100 ng/ml) for the indicated time. <DL: below detection limit. Number of independent experiments is indicated for each condition.

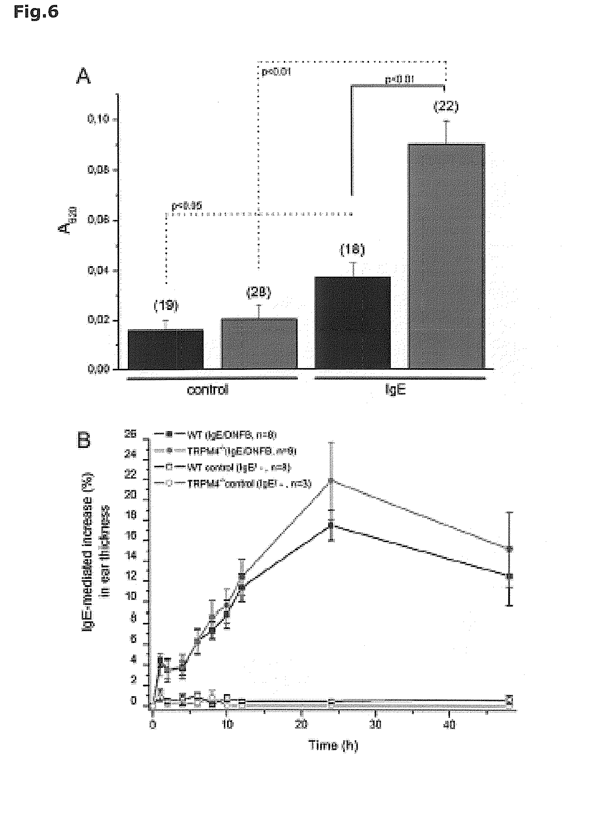

Figure 6: Passive cutaneous anaphylaxis in WT and TRPM4^{-/-} mice. (A) Vascular leakage as a measure of immediate phase passive cutaneous anaphylaxis induced by intradermal injection of anti-DNP IgE followed by an intravenous injection of DNP together with Evan's blue. 0.9% NaCl was injected instead of IgE in the control group. Significant anaphylaxis develops in anti-DNP IgE injected mice, compared to the 0.9%NaCl injected mice. TRPM4-/-mice display significantly higher extravasation of Evan's blue than WT mice. (B) Late phase PCA reactions. DNFB was topically applied to the right ear of WT and TRPM4^{-/-}mice passively sensitized with anti-DNP IgE; the untreated left ear served as control. The percentage increase in ear thickness is given; n, number of animals.

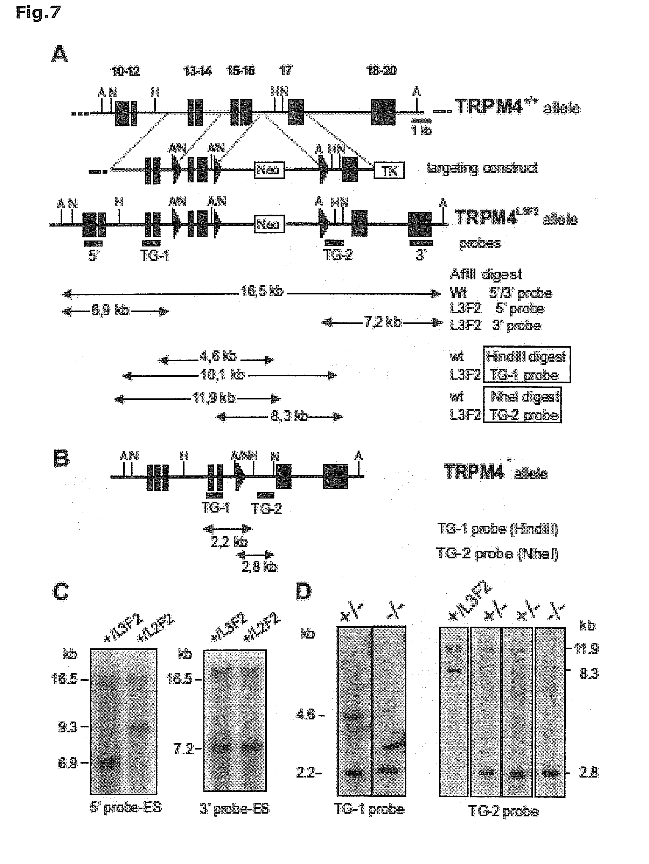

Figure 7: Targeted disruption of the TRPM4 gene. The Cre-loxP strategy was used to excise exons 15 and 16, encoding the first transmembrane spanning domain of TRPM4. (A) WT TRPM4-allele, targeting construct and recombinant TRPM4^{L3F2} allele. Translated exons (not in scale) and introns are shown as filled boxes and lines. The TRPM4^{L3F2} allele contains one loxP site (filled triangles) upstream and a loxP flanked PGKneo cassette downstream of exon 16. H, HindIII; A, AflII; N, NheI. Probes and sizes of genomic DNA fragments as expected by Southern blots are indicated. (B) Cre mediated conversion of the TRPM4^{L3F2} allele to the TRPM4-allele in mice. (C) Identification of the recombinant TRPM4^{L3F2} and TRPM4^{L2F2} alleles in ES cells by Southern blot analysis (AflII digest) using a 5' and a 3' probe placed external to the targeted sequence. (D) Cre mediated generation of the TRPM4-allele in mice resulted in the conversion of the 10.1 kb fragment of the TRPM4^{L3F2} allele to a 2.2 kb fragment (TG-1 probe, HindIII digest) and the conversion of the 11.9 kb fragment of the TRPM4^{L3F2} allele to a 2.8 kb fragment (TG-2 probe, NheI digest).

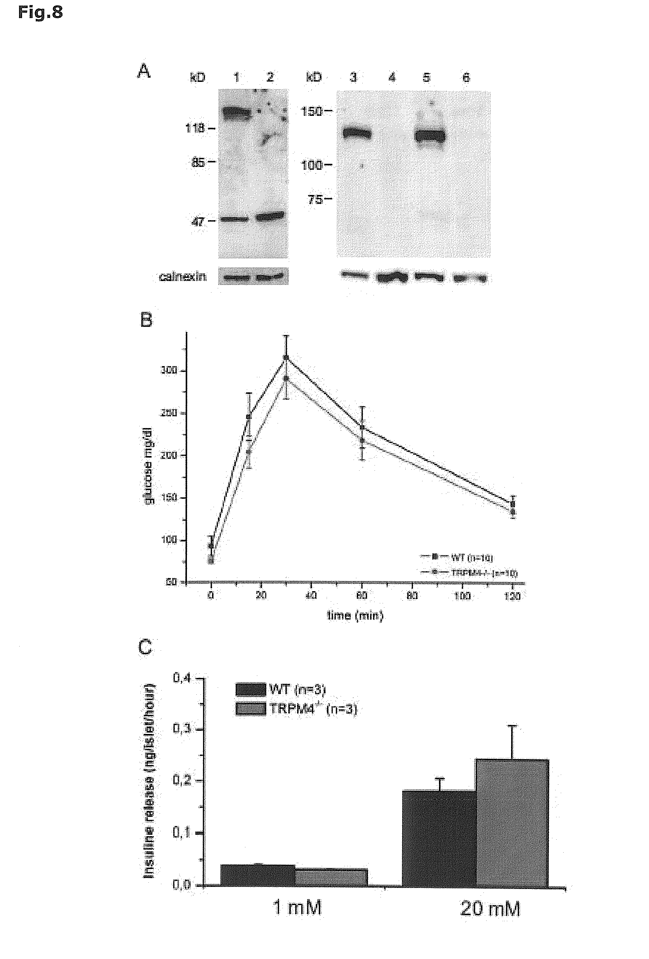

Figure 8: Unchanged glucose-induced insulin secretion and glucose tolerance test in TRPM4^{-/-}mice. (A) Western blot of protein fractions from WT (lane 1, 75 µg) and TRPM4^{-/-}pancreas (lane 2, 75 µg) and WT BMMC (lane 3, 100 µg per lane), TRPM4^{-/-} BMMC (lane 4, 100 µg per lane), WT pancreatic islets (lane 5, 100 µg) and TRPM4^{-/-} pancreatic islets (lane 6, 100 µg) using TRPM4-specific antibody 578. Like in WT BMMC, 138 kDa TRPM4 proteins are detected in both pancreas and pancreatic islets from WT mice but not in BMMC, pancreas and pancreatic islets from TRPM4^{-/-} mice. (B) Time course of blood glucose levels before and after intraperitoneal glucose application in WT and TRPM4 deficient mice (10 mice per genotype). (C) Insulin secretion from pancreatic islets isolated from WT and TRPM4^{-/-} mice. Pooled data from 30 islets derived from 3 individual mice are given from each genotype. Islets were stimulated for 1 hour in the presence of 1 mM or 20 mM glucose. Total insulin content was 31.7 ± 5.6 ng/islet (WT) and 28.0 ± 2.9 ng/islet (TRPM4^{-/-}, p>0.05). Insulin secretion was measured by R. Ramracheya, S. Collins, P. Rorsman, Centre for Diabetes, Endocrinology and Metabolism, University of Oxford, Oxford, United Kingdom.

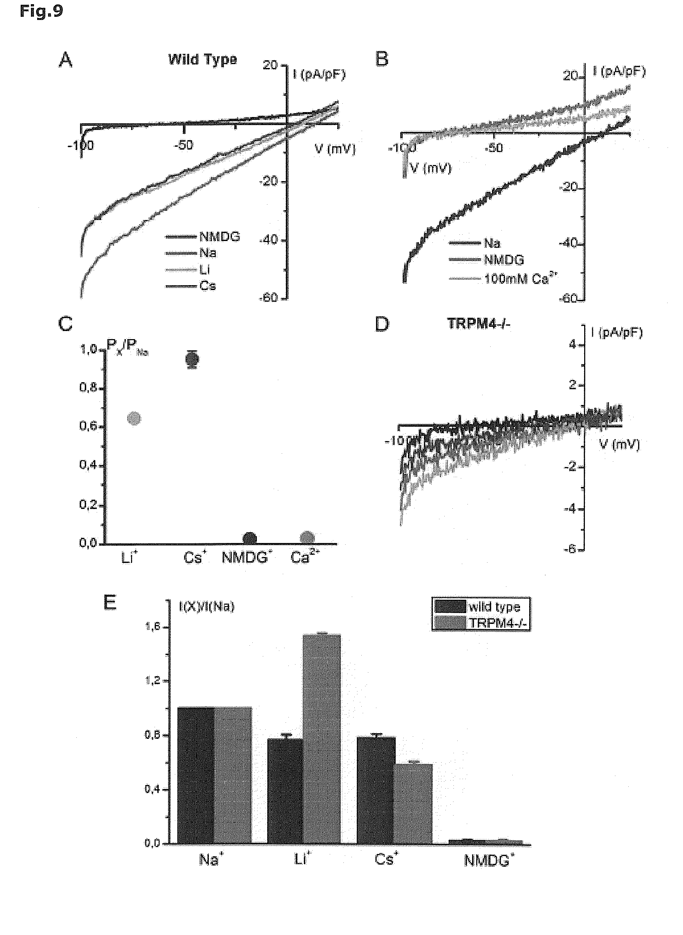

Figure 9: Permeation properties of the endogenous Ca²⁺ activated cation current in bone-marrow derived mast cells. (A) Whole cell current traces from a WT BMMC in response to linear voltage ramps from -100 to +100 mV (Vh = 0 mV), dialyzed with 10 µM Ca²⁺ containing pipette solution, in an extracellular solution containing 156mM Na⁺, Li⁺, Cs⁺ or NMDG⁺ as indicated. (B) Current traces as in panel A, in an extracellular solution containing 156 Na⁺, 156 NMDG⁺ or 100 mM Ca²⁺ as indicated. (C) Permeability ratio's for different cations calculated after correction of the reversal potential of currents as in panel A for the liquid junction potential, as described in Nilius B. et al., J. Biol. Chem. 280:22899-22906 (2005). (D) Current traces from TRPM4^{-/-}BMMC as in panel A. Color-code is the same as in panel A. (E) Normalized current values for WT and TRPM4^{-/-} currents measured at -80mV. Currents were normalized to the value in 156 Na⁺ solution. n is 6 (WT) and 8 (TRPM4^{-/-}).

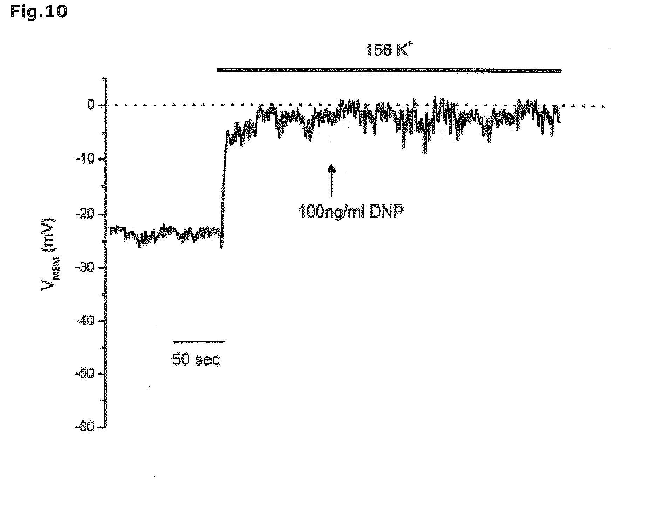

Figure 10: Membrane potential change upon application of 156 mM KCI solution. Membrane potential measurement using the current clamp configuration, in perforated patch mode. Extracellular solution was changed from normal Krebs to 156 mM KCI containing solution. Representative trace from six wild type BMMCs illustrates that in high K⁺ solution the membrane potential of BMMC's is clamped close to 0mV, also when 100 ng/ml DNP is applied. Identical results were obtained with TRPM4^{-/-} mast cells.

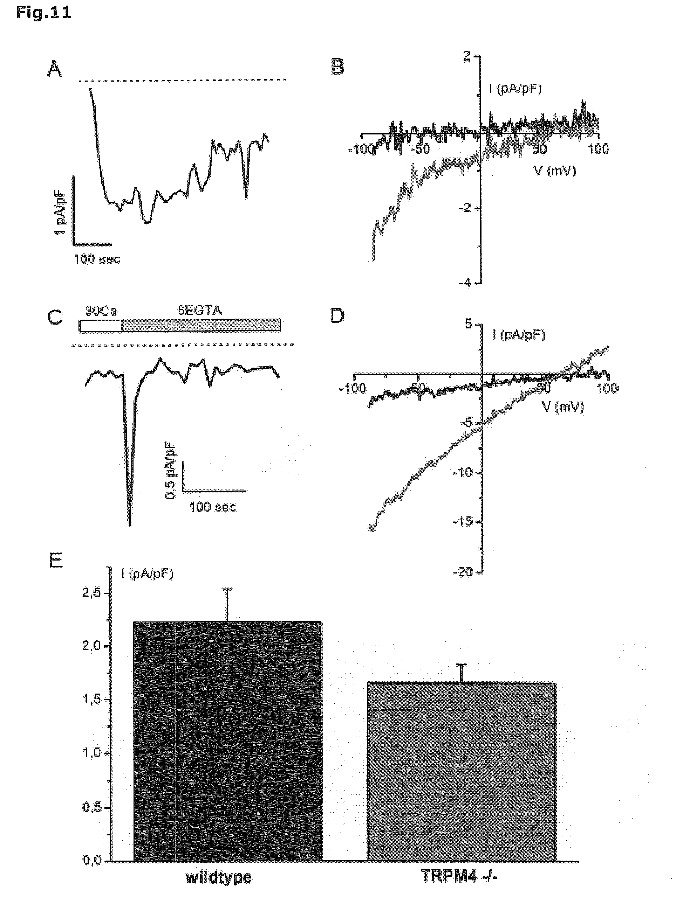

Figure 11: Ca²⁺ release activated Ca²⁺ currents (CRAC) in wildtype and TRPM4^{-/-} BMMCs. (A) Time course of a whole cell recording with a pipette solution containing 10 mM BAPTA and 50 µM IP₃ (+ in mM: 120 CsAsp, 20 CsCl, 2 MgCl₂, 1 Na₂ATP, 10 Hepes, pH7.2). Extracellular solution contains 30 mM Ca²⁺. A representative example is shown for a wild type BMMC. (B) Representative current traces taken at the beginning of the experiment and after reaching the plateau level. Currents were corrected for leak currents as described in (Hoth, M., Penner, R., Nature, 355-353-356 (1992)). (C) Time course of an experiment, taken at -80 mV. Pipette solution as in panel A. After full activation of ICRAC extracellular solution was switched from 30 mM Ca²⁺ containing to a OCa²⁺ (5 mM EGTA) solution. (D) Representative current traces taken in 30 Ca²⁺ solution and at the peak value after switching to 0 Ca²⁺ solution. (E) Mean values for the current in 30 Ca²⁺ solution measured at -80 mV for wild type and TRPM4^{-/-} BMMCs. Number of cells is 10 for both genotypes, p > 0.05.

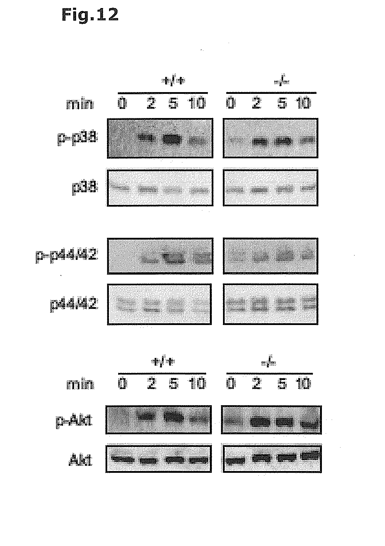

Figure 12: FceRI-activated signalling analysed by immunoblotting with activation state-specific phosphoantibodies against p38, the p44/42 MAP kinases Erk1 and Erk2 and Akt. WT and TRPM4^{-/-} BMMCs sensitized with anti-DNP IgE were stimulated with DNP for the indicated time intervals. Total cell lysates were separated by 7 and 10% SDS-PAGE. Independent immunoblots with anti-phospho-p38, anti-p38, anti-phospho-p44/42, anti-p44/42, anti-phospho-AKT, anti-Akt and anti-β-actin antibodies. Data are representative of at least three independent experiments and independent cell preparations.

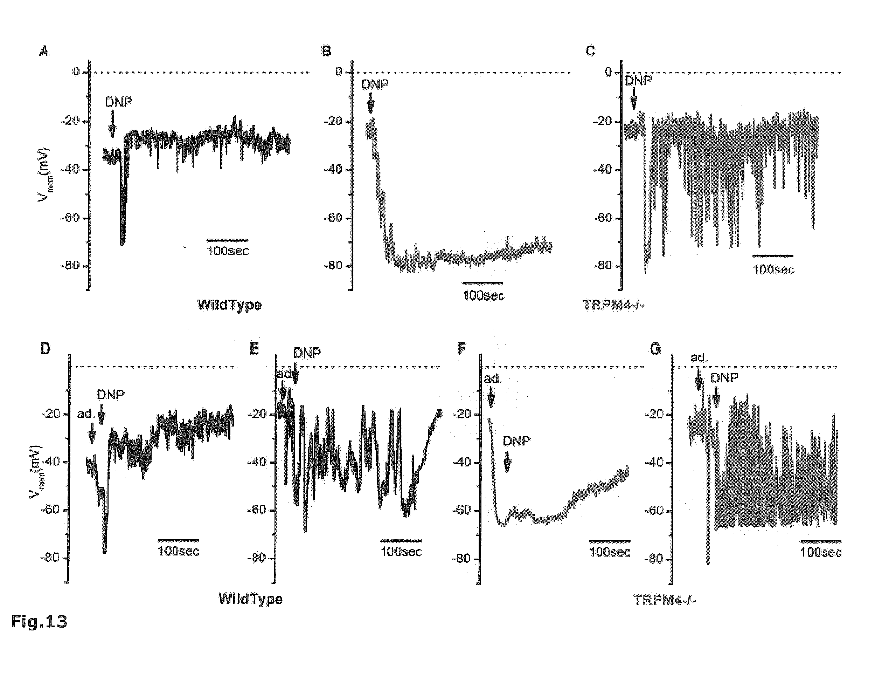

Figure 13: Membrane potential measurements from bone marrow derived mast cells after antigen stimulation. Measurements were performed in the whole-cell current clamp mode using perforated patches. (A) Representative time-course of membrane potential changes after 100ng/ml DNP-HSA application (at arrow) to WT BMMC. From 19 cells, 19 react with a short hyperpolarisation, followed by a rapid and sustained depolarisation. (B-C) Same as in A, but with TRPM4^{-/-} BMMCs. From 15 TRPM4^{-/-} cells, 6/15 develop a sustained hyperpolarisation, 9/15 start oscillating upon stimulation (D-E) Representative traces of membrane potential changes after 10 µM adenosine (ad.) followed by 100 ng/ml DNP-HSA (DNP) application. From 7 WT mast cells 3/7 react to adenosine stimulation with a short hyperpolarisation pursued by a sustained depolarization and 4/7 with a short hyperpolarisation, followed after DNP stimulation by oscillations. (F-G) Same as in (D-E), but with TRPM4^{-/-} BMMCs. From 7 cells, in 4/7 the hyperpolarisation upon adenosine stimulation is followed with a sustained hyperpolarisation when DNP is applied and in 3/7 the hyperpolarisation upon adenosine stimulation, is followed by very fast oscillations.

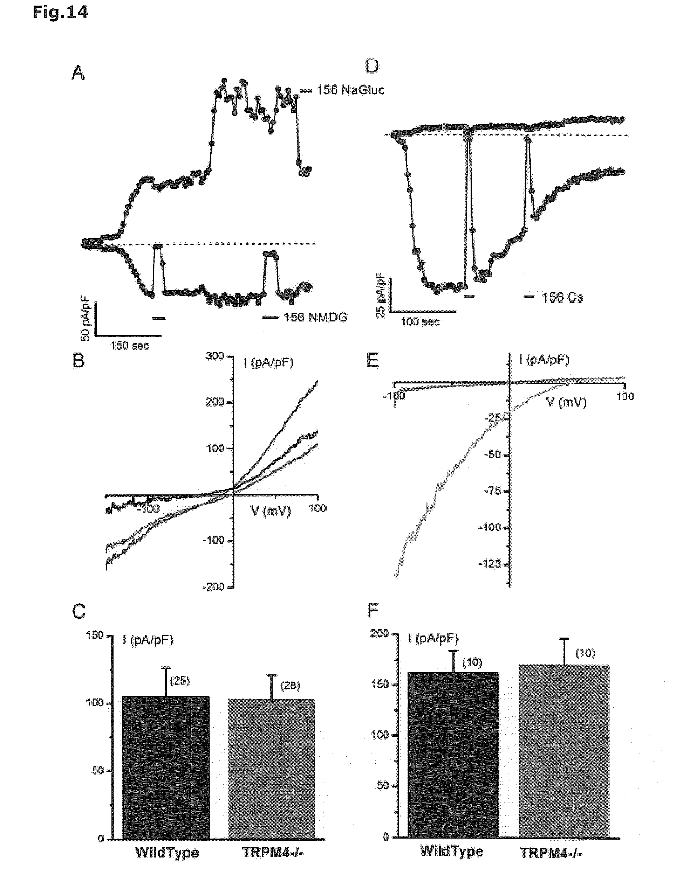

Figure 14: Ca²⁺ activated K⁺ and Cl⁻ currents in wild type and TRPM4^{-/-} BMMCs. (A) Time-course of a whole cell patch clamp recording at -80 and +80 mV from a wild type mast cell. Pipette solution contained 10 µM Ca²⁺, and Na⁺ instead of K⁺. Voltage protocol as in Fig. 2. Upon break-in a Ca²⁺ activated non-selective cation current develops, followed by a Ca²⁺ activated Cl current. The outward component of the latter current can be blocked by replacing extracellular NaCl with equimolar NaGluconate. (B) Representative current traces in response to a linear voltage protocol, taken at the indicated points in panel A. The black trace represents the difference curve of the current in the presence and absence of NaGluconate. (C) Mean amplitude of the gluconate-inhibitable current at +80 mV from wild type and TRPM4^{-/-} BMMCs. Number of cells is indicated in brackets. p > 0.05. (D) Time course at -80 and +80 mV from a wild type mast cell. Pipette solution contained 10 µM Ca²⁺, and Na⁺ instead of K⁺. Extracellular solution contains 156 mM KCI. Upon break-in a large inwardly rectifying current develops of which the inward component is blocked when extracellular K⁺ is replaced by Cs⁺. (E) Representative current traces taken at the indicated times in panel D. (F) Mean amplitude of the Cs⁺ inhibitable current from wild type and TRPM4^{-/-}BMMCs. Number of cells is indicated in brackets. p > 0.05.

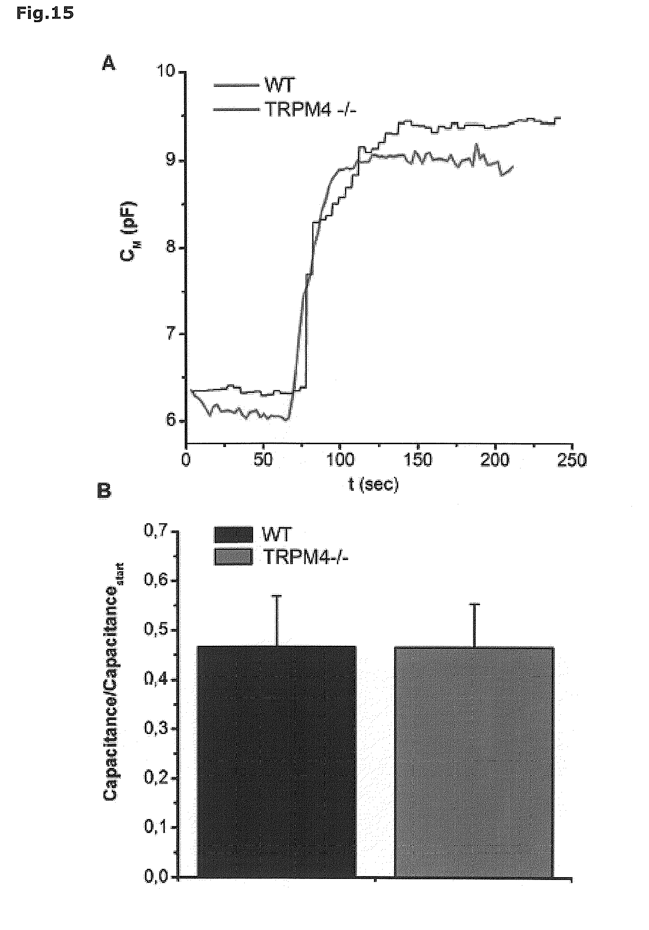

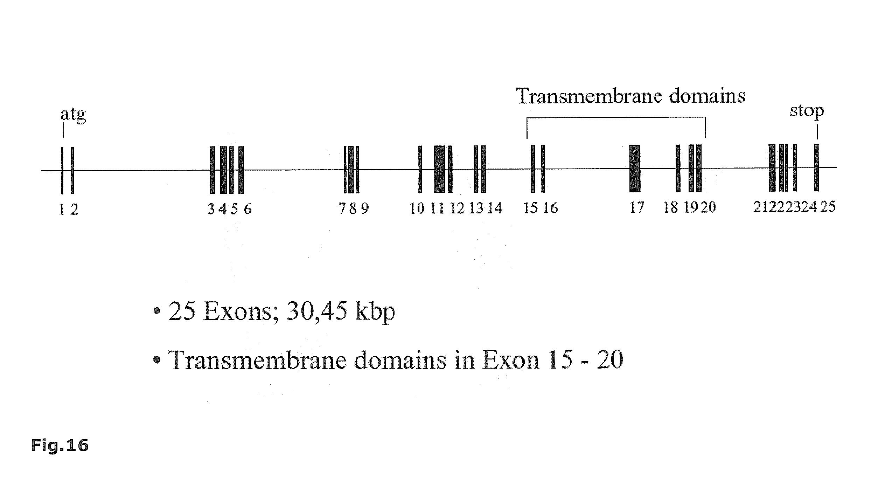

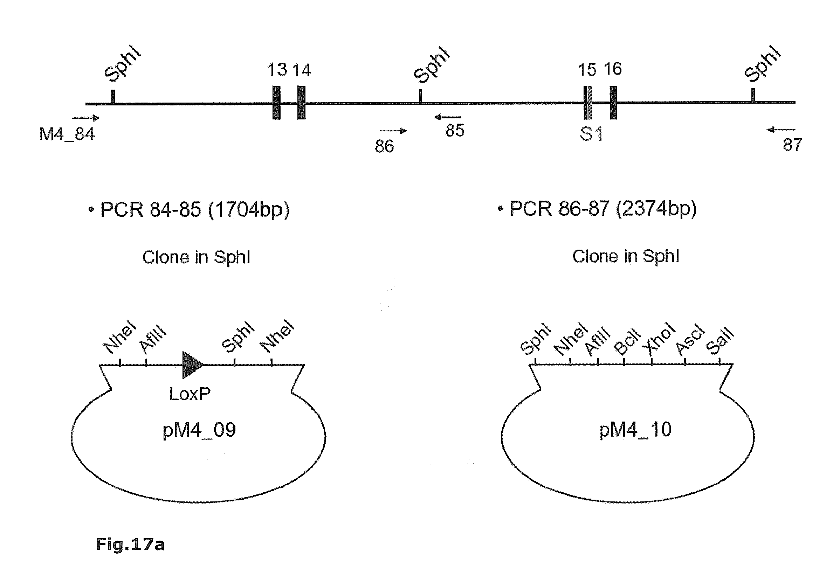

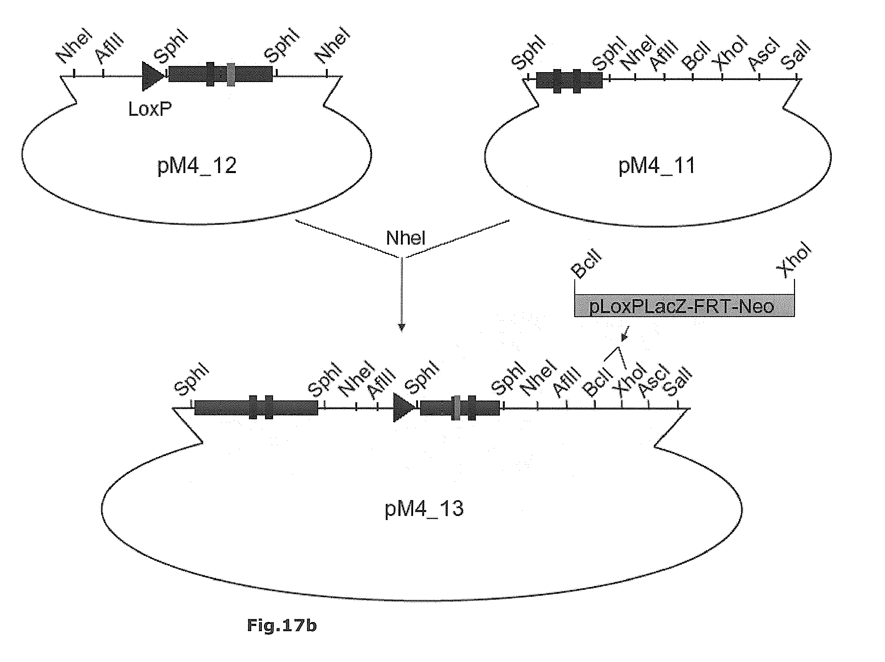

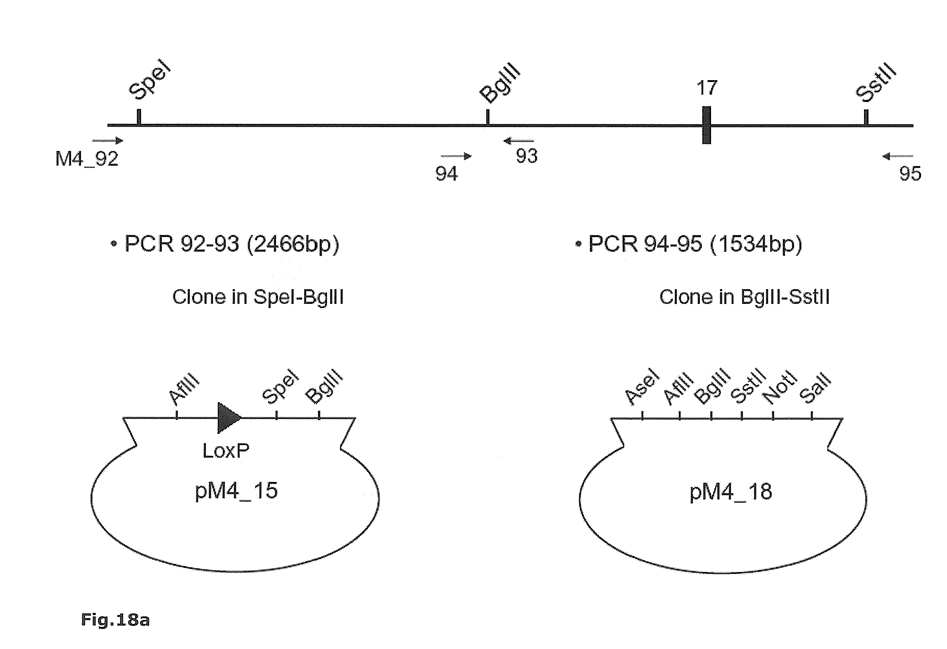

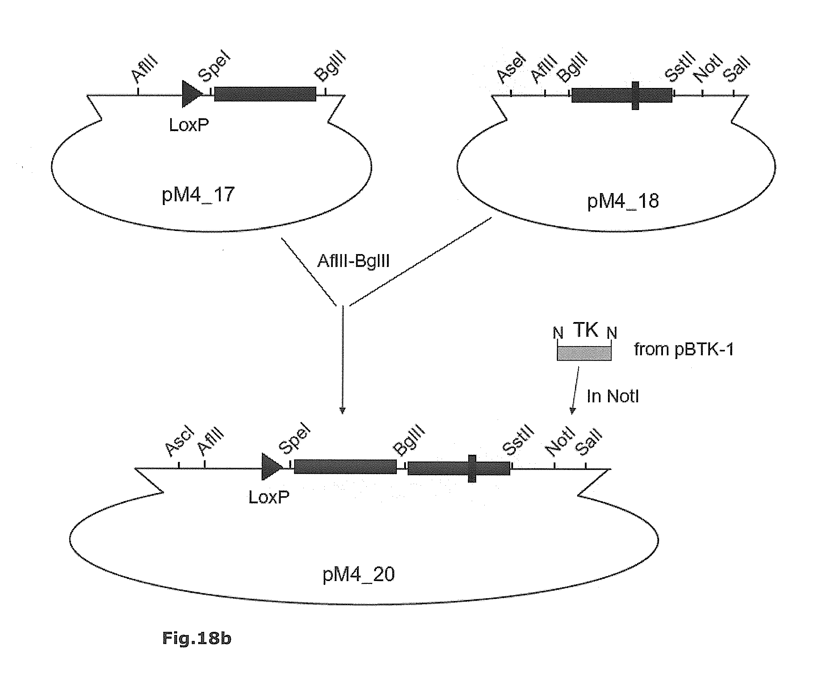

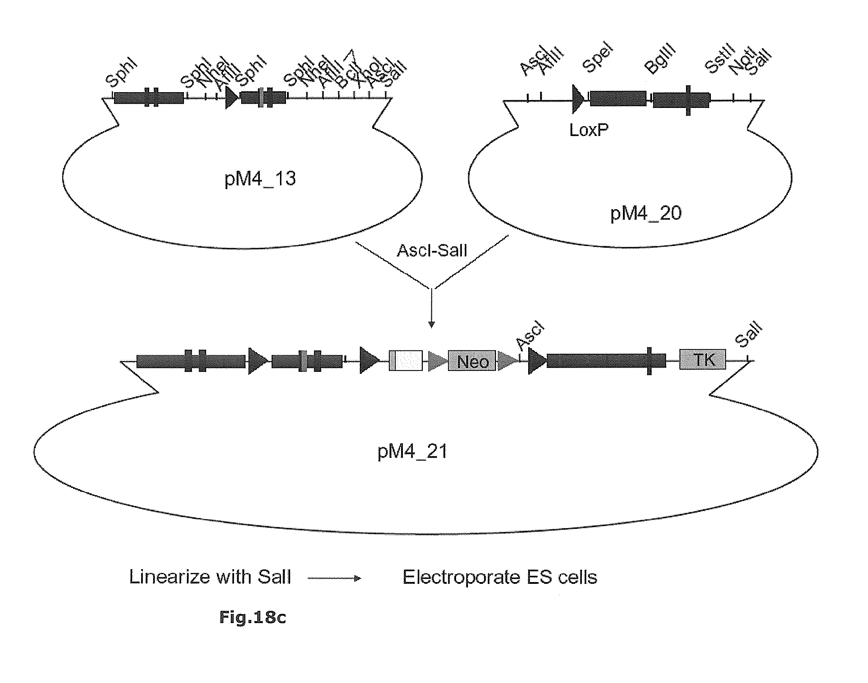

Figure 15: GTPγS-induced Capacitance changes in WT and TRPM4^{-/-}BMMC's. (A) Representative traces for membrane capacitance changes of WT and TRPM4^{-/-} BMMC's after break-in with 100 µM GTPγS containing pipette solution. WT and TRPM4^{-/-} BMMCs increase their cell surface after a variable delay due to the fusion of histamine containing vesicles with the plasmamembrane. (B) Averaged ratio of membrane capacitance 10 min after break-in to the membrane capacitance at the start of the recording (n=6 per genotype, p > 0.05).

### Detailed Description of the Invention

The particular terms and abbreviations utilized to define the present invention are further defined in the following.

The terms "does not produce functional TRPM4" denote the lack of effective TRPM4 expression. A "lack of effective TRPM4 expression" also includes the expression of non-functional (i.e. truncated) TRPM4 proteins which are not excerting the function of the native protein. The term "produces suboptimal levels of TRPM4" encompasses the translation levels of TRMP4 protein, which are insufficient to exert their function. Preferably, the level is reduced at least by 50%, more preferably by 70% and most preferably by 100 %.

The term "homozygous disruption" relates to an identical mutation in both alleles of a gene. The term "heterozygous disruption" relates to a mutation in only one allele of gene.

The term "mutation" refers to a change of one or more nucleotide pairs of a DNA molecule. The term "insertion" is directed to a mutation identified by the presence of one or more additional base pairs in the DNA. The term "deletion" relates to a mutation generated by removal of a sequence of DNA (one or more base pairs), the regions on either side being joined together. The term "substitution mutation" is directed to a nucleotide exchange. The substitution mutation can result in an amino acid change or can introduce a premature translation stop codon. Furthermore, a substitution mutation can affect splicing or expression of the gene when occurring at sites necessary for splicing or gene regulation.

The term "gene targeting" relates to a type of homologous recombination that occurs when a fragment of genomic DNA is introduced into a cell and that fragment recombines with homologous sequences in the genome. The term "gene trap integration" is directed to insertion of a vector, which comprises a reporter gene and which is activated upon insertion into an active transcription unit of the genome. The term "mutagenesis" denotes a chemical or physical treatment that changes nucleotides in the genome of an organism. An example of a chemical mutagenesis is N-ethyl-N-nitrosurea (ENU) mutagenesis.

The term "exon" encompasses a segment of a gene, which is decoded to give a mRNA product. Individual exons may contain protein coding DNA and/or noncoding DNA. The term "intron" denotes non-coding DNA, which separates neighboring exons in a gene. During gene expression introns, like exons are transcribed into RNA but the transcribed intron sequences are subsequently removed by RNA splicing and are not present in mRNA. The term "regulatory region" relates to the nucleotide sequence which comprises regions that are necessary for the regulation of gene transcription. These regions comprise, for example, promoters and enhancers and they can be located in 5' untranslated regions, exons, introns and 3'UTRs. The term "splice site" encompasses the nucleotides at the beginning and the end of the intron that are required for the joining of two exons by removing the intercalated intron during primary transcript processing to functional mRNA.

The term "selecting an agent for treating a symptom" encompasses choosing a composition for management of the condition.

The term "application of one or more agents" relates to administering single compounds or compound combinations orally, by inhalation, parenterally, e.g. intravenously, subcutaneously, intraperitoneally or intramuscularly, or topically, e.g. ophtalmically, vaginally, rectally or intranasally.

In a preferred embodiment of aspect (1) of the invention, the non-human animal does not express functional TRPM4 or expresses suboptimal levels of TRPM4.

In another preferred embodiment the animal is TRPM4-deficient due to mutation, a partial or entire deletion of the TRPM4 gene. Particularly preferred is an excision of one of the transmembrane domains, such as exons 15 and 16 encoding the first transmembrane domain of the TRPM4 gene (bp ... to ... of SEQ ID NO: 1) or of an exon 5' thereof, or by excision of a domain of the channel pore between exons 5 and 6 (bp ... to ... of SEQ ID NO:1).

The non-human animal according to the invention may be a vertebrate or invertebrate. The vertebrate preferably is a mammal, such as a rodent (including mouse and rat), or a non-mammal, such as fish (including zebrafish). The invertebrate includes worm and insects. Particularly preferred is that the non-human animal is a mouse.

In another preferred embodiment of aspect (1) the genome of the animal comprises a homozygous or a heterozygous disruption. The homozygous disruption of the TRPM4 gene, however, is preferred.

The process for preparing the non-human animal of aspect (3) of the invention comprises transfecting cells with a targeting vector comprising 5' and 3' homology arms flanking a section of the TRPM4 gene to be modified by the targeting vector. In case the non-human animal is a mammal and the cells that are transfected in the process of aspect (2) are ES cells. The process may further comprise one or more of the following steps:
- selecting for positive homologous recombination events, and
- generating chimeric non-human animals.

The targeting vector of aspects (3) and (4) of the invention may further comprise one or more functional sequences selected from recombinase recognition sequences (RRS), selection marker genes, reporter genes, expression control elements including promoter sequences, polyadenylation sequences, introns, IRES, splice donor and splice acceptor sequences.

In a preferred embodiment of aspect (3) of the invention, the homology arms have a length of 0.1 to 20 kb, preferably of 0.5 to 10 kb. It is particularly preferred that the targeting vector contains a 5' homology arm corresponding to exons 13-16 of the TRPM4 gene (i.e., bp ... to ... of SEQ ID NO:1) and a 3' homology arm corresponding to exon 17 of the TRPM4 gene (i.e., bp ... to ... of SEQ ID NO:1), wherein exons 15 and 16 are flanked by two RRS of a first recombinase.

Particularly preferred in said embodiment is that the targeting vector contains a first selection cassette with a positive selection marker and a third RRS of the first recombinase being located downstream of the exons flanked by said two RRS of the first recombination. It is further preferred that said first selection cassette contains a pair of RRS of a second recombinase flanking the positive selection marker gene. Furher particularly preferred in said embodiment is that the targeting vector contains a first reporter gene adjacent to the first selection cassette, preferably upstream of the first selection cassette. Finally preferred in said embodiment is that the targeting vector contains a second selection cassette with a negative selection marker gene or a second reporter gene downstream of the 3' homology arm.

According to the invention the RRS of the first and second recombinase are selected from wild-type (wt) or mutant loxP, FRT, C31Int, λ-Int sites. Also, the positive selection marker gene may be a DNA sequence encoding a protein conferring resistance against cell poison, including neomycine, hygromycine, puromycine histidinol and bleomycine or is a DNA sequence containing superior metabolic properties to the cells including the utilization of xanthine and adenine. It is particularly preferred that the positive selection marker gene is a neomycine phosphotransferase gene. The negative selection marker is a DNA sequence encoding a cell poison such as the HSV thymidine kinase gene and diphtheria toxin A. The HSV thymidine kinase gene it particularly preferred. Finally, first and second reporter gene, which preferably are differing from each other, include genes coding for fluorescent proteins such as GFP, Dsred and modifications thereof, enzymes and the like.

In a particularly preferred embodiment of aspect (3) and (4) of the invention the targeting vector has the sequence shown in SEQ ID NO:2.

The invention is further described with reference to a particularly preferred embodiment, namely the targeting of the TRPM4 gene in mice (this is , however, not be construed as a limitation of the invention). The TRPM4 gene was inactivated using a Cre-IoxP-mediated gene targeting strategy in murine embryonic stem cells (Figure 1A, B). Recombination and Cre-mediated excision of exons 15 and 16 encoding the first transmembrane segment in TRPM4, which is present in all known TRPM4 splice variants (Nilius, B. et al., J. Biol. Chem., 278:30813-30820 (2003)), was confirmed using Southern blot analysis (Figure 7). TRPM4 transcripts of the same size as previously detected in other tissues (Nilius, B. et al., J. Biol. Chem., 278:30813-30820 (2003)), are abundantly expressed in bone-marrow derived mast cells (BMMC) as determined by Northern blot analysis (Figure 1C). Using a polyclonal antibody directed against the amino-terminus of the protein, 138 kDa TRPM4 proteins were detected through western blot analysis in bone-marrow derived mast cells (BMMC), endothelium, cardiac atrium and ventricle (Figure 1D) and pancreatic islets (Figure 8A) from wild type (WT) but not from TRPM4^{-/-} mice. Using this antibody, TRPM4 specific staining is observed in connective tissue mast cells of the skin (Figure 1E) from WT but not from TRPM4^{-/-} mice. TRPM4-deficient (TRPM4^{-/-}) mice are viable, fertile, display no obvious anatomical abnormalities and are segregated in the mendelian frequency (84 WT, 122 TRPM4^{+/-}, and 81 TRPM4^{-/-} progeny were obtained from 39 litters of heterozygous crosses). Adult WT and TRPM4^{-/-} mice are of similar size and weight (WT: 30.7±0.5g, n=14; TRPM4^{-/-}: 30.5±0.7g, n=14, 10-16 weeks of age, p>0.05). In isolated right atria the spontaneous beating frequency (WT: 294±26 bpm, n=10; TRPM4^{-/-}: 315±14 bpm, n=12; p>0.05) and force of contraction (WT: 1.2±0.2 mN, n=9; TRPM4^{-/-}: 1.4±0.1 mN, n=11) is not altered in the absence of TRPM4. Also, resting heart frequency of freely moving mice (WT: 517±7 bpm (n=9), TRPM4^{-/-}: 531±4 bpm (n=9); p>0.05) and circadian rhythm is unchanged in TRPM4^{-/-} mice. Endothelium-dependent relaxation of precontracted aortic and carotid artery rings by acetylcholine is not affected by TRPM4 gene ablation (not shown). Further, glucose clearance is identical in WT and TRPM4^{-/-} mice challenged with intraperitoneal glucose. These results are supported by monitoring glucose-induced insuline release from pancreatic islets, which is also not changed upon TRPM4-deletion (Figure 8B, C). Bone-marrow from both genotypes developed highly pure mast cell populations (constantly >95%) in cultures supplied with IL-3, as assessed by surface expression of the FceRI receptor (Figure 1F) and c-Kit (not shown). Both proteins are equally expressed in WT and TRPM4-/-BMMC's. The morphology of TRPM4^{-/-} mast cells was unaltered compared to WT, as assessed by electronmicroscopy of BMMC cultures and of connective tissue mast cells in the skin (Figure 1G, H). The average cell size, the average number of granules (WT: 33±2, n=52; TRPM4^{-/-}: 36±2, n = 53), and the overall cell shape is not different for TRPM4^{-/-} compared to WT cells. Furthermore, the density of mast cells in the skin is equal in WT and TRPM4^{-/-}mice (WT: 3.0±0.8; TRPM4^{-/-}: 2.7±1.3 per 0.076 mm², n=3 individual animals, p>0.05; a representative example is given in Figure 1I). All together these results indicate that mast cell development is not impaired by the loss of TRPM4. Lack of Ca²⁺-activated non-selective (CAN) channels in TRPM4-deficient mouse BMMCs. To test the hypothesis that TRPM4 is part of the CAN channel in BMMCs, we first recorded currents from WT BMMCs (Figure 2). Cells were perfused with a pipette solution containing 10 µM free Ca²⁺. K⁺ was omitted from the recording solutions, to avoid the contribution of K⁺ channels to the whole cell current. Under these conditions, a relatively large, stable cation current was activated after a variable delay (20-100 s, Figure 2A). Inward current could be completely blocked by replacing extracellular Na⁺ with N-methyl-D-glucamine (NMDG), indicating that this current is carried by cations. On average, current densities amounted 55.1±9.1 pA/pF (n=35, VM= -80 mV) in WT BMMCs. The current-voltage relationship (I-V) of WT currents was linear and reversed at +1.4±0.5 mV in the presence of Na⁺, and - 94.8±2.7 mV in the presence of NMDG (n=35, Figure 2B). Step protocol showed no obvious voltage- and time-dependence of currents (not shown). In Figure 2C mean current densities are depicted when cells were dialyzed with Ca²⁺ concentrations between 100 nM and 10 µM. With 100 nM free [Ca²⁺]cyt hardly any current was activated (mean current density was 1.8±0.28 pA/pF at -80 mV, n=10). With 1 µM [Ca²⁺]cyt current densities amounted 10.2±2.4 pA/pF (n=15) at -80 mV. In inside-out patches we were able to record single channel activity in the presence of 300 µM Ca²⁺ at the cytosolic side. Representative traces recorded at -100 mV from 3 different patches are shown in figure 2D. Single channel conductance amounted 25.3±0.7 pS (n=5), which is in good agreement with the single channel conductance of recombinant TRPM4 overexpressed in HEK293 cells (Launay et al., 2002; Nilius et al., 2003). To further characterize this Ca²⁺-activated current, we have determined the channels permeability profile for several monovalent cations and Ca²⁺ (Figure 9). Na⁺ ions in the extracellular solution were substituted by equimolar amounts of Li⁺, Cs⁺ or NMDG⁺. When changes in conductance were analyzed, we obtained a sequence Na⁺>Li⁺~Cs⁺>>NMDG⁺. The relative permeability sequence, PX/PNa, was Na⁺>Cs⁺>Li⁺>>NMDG⁺, which is identical to the permeability sequence of overexpressed TRPM4 in HEK cells (Nilius, B. et al., J. Biol. Chem., 280:6423-6433 (2005)). To assess Ca²⁺ permeability, extracellular NaCl was replaced by 100 mM CaCl2. No significant inward currents were detected even at very negative membrane potentials. In contrast, when TRPM4^{-/-} BMMCs were analyzed in identical whole-cell conditions as WT cells, no significant current developed for at least 10 min. Only a small Ca²⁺-independent background current was active (Figure 2E), with a current density of 5.4±1.1 pA/pF (n=29) at -80 mV. The I-V curve was linear and reversed at +1.3±0.3 mV in the presence of Na⁺, and -95.9±3.2 mV in the presence of NMDG (n = 29). Currents measured under these conditions exhibited a cation conductance sequence Li⁺>Na⁺>Cs⁺>>NMDG⁺, unlike the WT current described above (Figure 9). Enhanced Ca²⁺ entry in TRPM4^{-/-} BMMCs. Physiologically relevant activation of mast cells occurs through antigen/IgE mediated cross-linking of the FceRI receptor, which can be complemented by other endogenous inflammatory ligands, including adenosine, which renders mast cells hyper-reactive towards allergen/IgE complexes (Laffargue, M. et al., Immunity, 16:441-451 (2002)).

BMMCs were incubated overnight with mouse anti-dinitrophenyl (DNP) IgE's, and subsequently challenged with the multivalent antigen DNP-human serum albumin (DNP-HSA, further annotated as DNP), either with or without pre-stimulation with adenosine. Stimulating mast cells with 0.05 up to 0.1 ng/ml DNP elicits, after a highly variable delay, a rise in [Ca²⁺]cyt, which is mostly sustained, but does not occur in 100% of the cells. Concentrations of 10 ng/ml up to 1 µg/ml on the other hand, elicit a sharp and almost synchronized increase in the [Ca²⁺]cyt of all cells, which is sustained for up to 10 min (not shown). In resting conditions the fluorescence ratio showed some variability between cell preparations, but was not significantly different between WT and TRPM4^{-/-} mast cells when all experiments were analyzed (i.e. 0.53±0.11 (n=15) and 0.6±0.15 (n=16) respectively, p=0.2). In Figure 3A, the average response is shown when BMMCs derived from WT and TRPM4^{-/-} mice were stimulated with 100ng/ml DNP. A sharp rise of the [Ca²⁺]cyt is followed by a decrease, until a plateau level is reached. TRPM4^{-/-} BMMCs display significantly higher [Ca²⁺]cyt both during the peak and the plateau phase compared to WT BMMCs. Similar results were obtained when BMMCs were co-stimulated with 10 µM adenosine and DNP (Figure 3B). The increased [Ca²⁺]cyt levels were sustained up to 3 h after mast cell activation. Fluorescence ratio was 0.99±0.13 and 1.48±0.11 (n=3) for WT and TRPM4^{-/-}, respectively, after DNP stimulation and 1.1±0.2 and 1.5±0.2 (n=3) for WT and TRPM4^{-/-}, respectively, after DNP/adenosine stimulation. Six hours after receptor activation [Ca²⁺]cyt was at prestimulation level for both genotypes in both conditions (not shown). The accumulation of intracellular Ca²⁺ in response to DNP and adenosine stimulation is almost entirely due to influx of Ca²⁺ from the extracellular space, through the Ca²⁺ release activated Ca²⁺ entry channel, CRAC (Hoth, M., Penner, R., Nature, 355:353-356 (1992)). In the absence of Ca²⁺ in the extracellular medium, the response to DNP and combined adenosine/DNP stimulation is limited to a transient peak, representing release of Ca²⁺ from the intracellular Ca²⁺ stores, which is not significantly different between WT and TRPM4^{-/-} cells (Figure 3C, upper panel). To test whether increased Ca²⁺ influx is also apparent after FceRI receptor independent stimulation, we induced Ca²⁺ influx using the store depleting agent 2,5-Di-(t-butyl)-1,4-hydroquinone (tBHQ, Figure 3D). Initial application of 20 µM tBHQ in the absence of extracellular Ca²⁺ elicits a transient peak in [Ca²⁺]cyt due to Ca²⁺ leaking from intracellular stores. Subsequent addition of 2.5 mM extracellular Ca²⁺ induces a large stable rise of [Ca²⁺]cyt, due to Ca²⁺ influx from the outside medium, which is again significantly larger in TRPM4^{-/-} BMMCs compared to WT BMMCs. Similar results where obtained when 2 µM ionomycin was used (not shown). CAN channels can play a role in modulating Ca²⁺ entry after activation, since influx of monovalent cations into the cell will depolarize the membrane potential and decrease the driving force for Ca²⁺ entry (Maruyama and Petersen, 1982; Teulon, 2000). To test this idea, we have performed similar experiments as above, in the presence of 156 K⁺ in the extracellular solution. This will effectively clamp the membrane potential of WT and TRPM4^{-/-}BMMCs close to 0mV (Figure 10). Under these conditions significantly less Ca²⁺ is accumulated in response to receptor stimulation in WT and TRPM4^{-/-}BMMCs compared to the above conditions, both after DNP (Figure 3C, lower panel) and adenosine/DNP stimulation (not shown). Notably, except for the first few seconds after stimulation, there is no longer a significant difference in Ca²⁺ accumulation between WT and TRPM4^{-/-}. Since the amplitude of the CRAC current is not significantly different between TRPM4^{-/-}and WT BMMCs (Figure 11), and FceRI stimulation leads to similar phosphorylation in both genotypes of PLC.1 and PLC.2 (Figure 3E), and of protein kinases that are activated upon FceRI stimulation (Figure 12), this strongly suggests that only a difference in the driving force for Ca²⁺ entry accounts for the higher Ca²⁺ accumulation in TRPM4^{-/-}BMMCs. Altered membrane potential regulation in TRM4^{-/-} BMMCs upon antigen stimulation. To further elaborate the latter hypothesis, we have measured the membrane potential of BMMCs upon receptor stimulation, using the current clamp mode. Perforated patches were used to preserve cell integrity. As above, cells were incubated with anti-DNP IgE overnight. Before application of DNP, membrane potential was not different between WT and TRPM4^{-/-}BMMCs, i.e. -27±3 mV (n= 26) versus -26±4 mV (n = 21, p=0.2) respectively. Upon stimulation with 100ng/ml DNP WT mast cells react with a short hyperpolarisation, followed by a rapid and sustained depolarization. In contrast, TRPM4^{-/-} BMMCs develop a sustained hyperpolarisation (6/15 cells) or start oscillating (9/15 cells). Figure 4A and B show representative examples of simultaneous membrane potential and [Ca²⁺]cyt measurements after stimulation of mast cells with 100ng/ml DNP. Similar results were obtained after stimulation with 10µM adenosine and 100ng/ml DNP. WT mast cells reacted to adenosine stimulation with a short hyperpolarisation, followed after DNP stimulation by oscillations (4/7 cells) or a short hyperpolarisation pursued by a sustained depolarization (3/7 cells; see Figure 13 for representative traces). TRPM4^{-/-} mast cells respond with a hyperpolarisation upon adenosine stimulation, which is either sustained when DNP is applied (4/7) or is followed by very fast oscillations (3/7). We have averaged the membrane potential for 10 min after DNP or adenosine/DNP application, to gain a measure for the mean hyperpolarisation after mast cell activation (Figure 4C and D). After stimulation with 100ng/ml DNP WT cells had an average membrane potential of -10±1 mV (n=19) compared to -32±2 mV (n=15) for TRPM4 KO cells. Double stimulation of mast cells with adenosine and DNP resulted in an similar difference, i.e. a mean membrane potential of -31±2mV for WT and -57±4mV for TRPM4 KO mast cells (p<0.01), indicating that in both conditions the driving force for Ca²⁺ entry is significantly larger in TRPM4^{-/-}BMMC compared to WT mast cells after receptor stimulation. Since the amplitude of other ion currents which can determine the membrane potential of activated mast cells, i.e. Ca²⁺-activated K⁺ currents and Ca²⁺-activated Cl- currents, is not significantly different between WT and TRPM4^{-/-} BMMCs (see Figure 14), these results establish an essential role of TRPM4 in membrane potential regulation after FceRI activation.

Antigen-induced mediator release from BMMCs. FceRI receptor mediated mast cell activation leads to rapid secretion of preformed inflammatory mediators, such as histamine, and the de novo synthesis of arachidonic acid metabolites and various cytokines and chemokines (Metcalfe, D.D. et al., Physiol. Rev., 77:1033-1079 (1997)). We have tested the release of histamine, the lipid-derived mediators leukotriene C4, D4 and E4 and of the cytokines IL-6 and TNFα from WT and TRPM4^{-/-} mast cells upon FceRI stimulation (Figure 5). Histamine release was probed using a fluorimetric assay. Total histamine content (ratio TRPM4^{-/-}/WT: 0.97±0.14; n=133) and spontaneous release of histamine (Figure 5A) were not different between WT and TRPM4^{-/-} mast cells. However, upon challenging BMMCs with 100ng/ml DNP for 20min, histamine release from WT cells is significantly increased compared to TRPM4^{-/-}cells (Figure 5A). Similarly, TRPM4^{-/-}cells release more histamine after co-stimulation with adenosine (10µM) and DNP (100ng/ml). When mast cells are activated with 2µM ionomycin a similar difference is obtained between WT and TRPM4^{-/-}cells, i.e. 58.3±3.0% (n = 30) and 83.0±2.1% (n = 34) of total histamine content respectively (not shown; p<0.01). The increased histamine release from TRPM4^{-/-} BMMCs was also obvious when the cells were stimulated for shorter or longer time periods with either DNP or with adenosine/DNP (Figure 5A, inset). Histamine release from mast cells is dependent on influx of Ca²⁺ from the extracellular medium. In the absence of extracellular Ca²⁺, WT and TRPM4-/-cells release 9.3±0.9% (n = 8) and 11.4±0.6% (n = 8) respectively, which is not significantly different from spontaneous histamine release levels. No significant difference is apparent between WT and TRPM4^{-/-} cells, i.e. 25.0±1.3% (n=8) and 28.5±0.9% (n=8) respectively (p>0.05), when the membrane potential is clamped close to 0mV in 156mM K⁺ containing medium. To rule out any non-specific aberration in the exocytosis machinery in TRPM4-/-mast cells which might cause increased histamine release independent of increased Ca²⁺ influx, we have performed capacitance measurements on BMMCs during perfusion of mast cells with GTP-.-S. Under these conditions, the increase of cell capacitance due to the fusion of histamine containing vesicles with the plasmamembrane during exocytosis, was not significantly different between WT and TRPM4-deficient mast cells (Figure 15). The release of lipid-derived mediators and cytokines following FceRI stimulation was investigated using enzyme linked immunoassays. TRPM4^{-/-} cells produced significantly higher amounts of the leukotrienes C4, D4 and E4 when stimulated with 100ng/ml DNP, compared to WT mast cells (Figure 5D). The release of IL-6 was not different between WT and TRPM4-/-at various time-points, up to 24 h, after stimulation, except after 20 min following receptor stimulation (Figure 5C). At this point WT mast cells released no measurable amount of IL-6 whereas TRPM4^{-/-} released considerable amounts of IL-6. On the other hand, TRPM4^{-/-}mast cells release significantly more TNFα compared to WT cells, at each time-point up to 24 h after FceRI stimulation (Figure 5D). The production and release of both IL-6 and TNFα is critically dependent of extracellular Ca²⁺ influx, since in the absence of extracellular Ca²⁺ neither of these mediators is released in measurable quantities (not shown). When the same experiments are performed in 156mM K⁺ containing medium, the released amount of either mediator is drastically reduced, and is not different between WT and TRPM4^{-/-} mast cells. After 6 hours TNFα release amounted 56.8±5.3 pg/106 cells (WT, n=3) and 61.2±2.8 pg/106 cells (TRPM4^{-/-}, n=3), whereas IL-6 release was 424.7±12.3 pg/106 cells (WT, n=3) and 446.4±15.7 pg/106 cells (TRPM4^{-/-}, n=3). Enhanced acute anaphylactic response in TRPM4^{-/-}mice. To test whether the alterations in FceRI-mediated activation of TRPM4^{-/-} BMMC's are also relevant for connective tissue mast cells in vivo, we performed two types of passive cutaneous anaphylaxis (PCA) experiments addressing both immediate and late phase anaphylactic responses. For the immediate response, two days after intradermal sensitization using anti-DNP IgE (60 ng) or saline injection as a control, PCA was induced by intravenous injection of DNP dissolved in Evan's blue containing saline. During the first hour after induction, the PCA reaction is mainly dependent on the degranulation of activated mast cells, with rapid histamine and serotonin release, resulting in locally increased blood vessel permeability and the extravasation of Evan's blue dye into the surrounding tissue (Inagaki et al., 1986). In control conditions dye extravasation 1 h after the antigen stimulus was the same in WT and TRPM4^{-/-} skin biopsies. However, following anti-DNP IgE sensitization, TRPM4^{-/-} mice showed a significantly increased Evan's blue extravasation into the skin after DNP injection compared to WT (Figure 6A), indicative for increased vascular permeability and severed cutaneous anaphylaxis. Similar results were obtained in a second group of animals that received 30ng of IgE intradermally (not shown). In this context it is important to note that the density of mast cells before DNP stimulation is not different in the skin of anti-DNP IgE-sensitized WT and TRPM4^{-/-} mice (WT: 2.8±0.8, TRPM4^{-/-}: 2.1±0.2 per 0.076 mm2, n=4 individual animals, p>0.05). To investigate the late phase reaction, mice were sensitized with an intravenous injection of anti-DNP IgE and the PCA reaction was probed by measuring ear thickness up to 48 hours after epicutaneous application of the hapten 2,4-dinitrofluorobenzene on the ears (Klemm et al., 2006). Studies in mast cell deficient W/Wv mice have shown that mast cells are required for both the tissue swelling response and the neutrophil infiltration associated with late-phase IgE-dependent PCA reactions (Wershil, B.K. et al., J. Clin. Invest, 87:446-453 (1991)). No difference was found at any time-point between WT and TRPM4^{-/-} mice (Figure 6B). These results indicate that TRPM4 is crucial for the immediate passive cutaneous anaphylactic response, but doesn't play a decisive role in the development of the late phase reaction.

The present invention establishes the role of TRPM4 as a key regulator of IgE-mediated Ca²⁺ entry, mast cell activation and immediate-type allergic reactions in mice. We (i) identify the 138kDa TRPM4-proteins in mast cells, (ii) show that TRPM4 is an essential constituent of the endogenous Ca²⁺-activated non-selective cation channel in wild type mast cells, which is absent in TRPM4-deficient cells, and (iii) provide direct experimental evidence by simultaneous recordings of the membrane potential and intracellular Ca²⁺ concentration that TRPM4-currents are essential determinants of the membrane potential, Ca²⁺ influx and consequently, release of proinflammatory mediators induced by antigen stimulation. Finally, we extend the relevance of the results obtained in bone marrow derived mast cells to tissue mast cells of the skin and show an enhanced acute IgE-mediated anaphylactic response in TRPM4-deficient mice. The properties of the endogenous Ca²⁺-activated non-selective channel in BMMCs described in this study are very similar to the properties of TRPM4 currents recorded after overexpression of the TRPM4 cDNA in HEK293 cells (Launay, P. et al., Cell, 109:397-407 (2002); Nilius B. et al., EMBO J., 25:467-478 (2003); Nilius, B. et al., J. Biol. Chem., 278:30813-30820 (2005); Nilius, B. et al., J. Biol. Chem., 280:6423-6433 (2005)). Both currents are Ca²⁺-activated, they have an identical permeability profile for monovalent cations, but are Ca²⁺ impermeable, and they show a similar single channel conductance. The Ca²⁺ sensitivity is in the same range as reported by the Nilius group for the overexpressed channel and for endogenous CAN currents in other cell types (Nilius, B., Vennekens, R., Journal of Physiology PMID: 16680483 (2006)). On the other hand, the endogenous current in mast cells shows less voltage dependence compared to the overexpressed channel, exemplified by a linear I-V curve and little time-dependence of these currents in response to 400 msec voltage steps to positive and negative membrane potentials compared to currents through overexpressed channels. However, voltage dependence of TRPM4 currents is critically relying on PiP2 levels within the plasma membrane (Nilius, B. et al., EMBO J., 25:467-478 (2006); Zhang, Z. et al., J. Biol. Chem., 280:39185-39192 (2005)), which might be more appropriately matched by the endogenous TRPM4 protein levels in the primary mast cells than by the TRPM4 overexpression in HEK 293 cells. It is notable that mice deficient of the type I phosphatidylinositol phosphate kinase, the enzyme that synthesizes PiP2, exhibit an increased mast cell degranulation similarly to TRPM4^{-/-}mice (Sasaki, J. et al., J. Exp. Med., 201:859-870 (2005)).

Mast cell activation after FceRI receptor aggregation is critically dependent on Ca²⁺ influx from the extracellular medium, and this influx is increased in TRPM4^{-/-} mast cells. Our data support the model that TRPM4 regulates FceRI receptor induced Ca²⁺ entry and mast cell activation through its effect on the membrane potential (VM). There is no apparent difference in PLC.activation and phosphorylation of protein kinases in response to FceRI receptor stimulation, and increased Ca²⁺ influx in TRPM4^{-/-}BMMCs was also observed when cells were treated with tBHQ or ionomycin, compounds which induce Ca²⁺ influx independent of receptor-stimulation. Furthermore, when membrane potential changes are prevented by performing FceRI stimulation in high potassium medium, there is no difference in Ca²⁺ signaling and mediator release between TRPM4^{-/-} and WT cells. In normal conditions activation of TRPM4 currents by [Ca²⁺]cyt immediately after FceRI receptor activation, will depolarize the cell and limit the driving force for further Ca²⁺ entry into the cell. By simultaneous recordings of the membrane potential and of the intracellular Ca²⁺ concentration of wild type mast cells we could show that FceRI receptor stimulation leads to a rapid hyperpolarisation followed by a sustained depolarization. The hyperpolarization mirrors the increase of the intracellular Ca²⁺ concentration and reflects activation of the the intermediate-conductance type Ca²⁺-activated K⁺ channel, iKCa1, also known as SK4 (Duffy, S.M. et al., J. Immunol., 167:4261-4270 (2001)). Similarly, 1-EBIO, an SK-4 opener, enhances FceRI-dependent Ca²⁺ influx and mast cell degranulation (Duffy. S.M. et al., J. Allergy Clin. Immunol., 114:66-72 (2004)). In contrast to wild type mast cells, in TRPM4^{-/-} cells the sustained depolarization is lacking and, consequently they display on average a significantly more hyperpolarized membrane potential after receptor activation, accompanied by an enhanced Ca²⁺ influx into the cell. Altogether these results demonstrate that the regulation of the membrane potential in TRPM4^{-/-}cells is altered, resulting in an increased driving force for Ca²⁺ entry, rather than the activity of the Ca²⁺ influx channel as such. A role for TRPM4 as a regulator of membrane potential has been suggested previously by RNAi and antisense knock down strategies (Earley, S. et al., Circ. Res., 95:922-929 (2004); Launay, P. et al., Cell, 109:397-407 (2002)). In Launay, P. et al. Cell, 109:397-407 (2002) it was suggested that suppression of TRPM4 expression converts oscillatory changes of [Ca²⁺]cyt into long-lasting sustained elevations in Ca²⁺ in a Jurkat T cell line. However, a substantial part of the TRPM4^{-/-} mast cells display membrane potential oscillations, indicating that TRPM4 is not essentially required for the occurrence of Ca²⁺ oscillations, at least in mast cells. Oscillations in TRPM4-deficient cells could be caused by an interplay of Ca²⁺-dependent activation and rundown of the intermediate-conductance Ca²⁺-activated K⁺ channel (Fioretti, B. et al., Biophys. Chem., 113:17-23 (2005)) or by an rhythmic activity of Ca²⁺-activated K⁺ channels and Ca²⁺-activated Cl-channels or Ca²⁺ influx channels (Berridge, M. et al., Curr. Biol., 9:R157-159 (1999)).

Upon FceRI activation several downstream pathways elicit degranulation, i.e. release of preformed vasoactive amines such as histamine and serotonin, and de novo synthesis of lipid-derived mediators and a wide range of cytokines and chemokines. Our results show that histamine and leukotriene release is increased in TRPM4^{-/-}cells. This could be anticipated, since degranulation of mast cells is a Ca²⁺ activated process (Blank and Rivera, 2004) and cytosolic phospholipase A2 (cPLA2) and 5-lipoxygenase, the key enzymes for leukotriene synthesis are Ca²⁺ sensitive (Peters-Golden et al., 2005). TNFα on the other hand is both released from preformed vesicles (Wershil, B.K. et al., J. Clin. Invest., 87:446-452 (1991)) and, like IL-6, transcribed under the control of specific transcription factors (Metcalfe, D.D. et al., Physiol. Rev., 77:1033-1079 (1997)). Differential regulation of specific transcription factors by Ca²⁺ is likely the cause for differential secretion of TNFα and IL-6 from TRPM4^{-/-} cells (Hundley et al., 2004; Lorentz, A. et al., J. Allergy Clin. Immunol., 111:1062-1068 (2003)).

The functional significance of the phenotype in mast cells becomes apparent when we induce a cutaneous allergic reaction in a passive cutaneous anaphylaxis model which is mainly dependent on the activation of tissue mast cells of the skin. Importantly, absence of functional TRPM4 protein has no effect on the development and the density of mast cells. In response to an antigen-stimulation TRPM4^{-/-} mice exhibit significantly more fluid extravasation in the tissue compared to wild type mice, indicating a more severe increase in vessel permeability in TRPM4^{-/-} mice, due to increased histamine release from mast cells in the skin. Notably, given that TRPM4 currents are decreased by lowering temperature (Talavera, K. et al., Nature, 438:1022-1025 (2005)), also the increased histamine release in response to cold, leading to cold urticaria (Kaplan, A.P. et al., N. Engl. J. Med., 305:1074-1077 (1981)), could be illustrative for the role of TRPM4 channel activity as a molecular brake on mast cell activation. In contrast to the immediate-type allergic reaction, the late-phase reaction to an allergic stimulus is not different between TRPM4^{-/-} and WT mice. The reason for this is likely that the secretion of at least some mast cell derived mediators essential for the development of the late phase anaphylaxis reaction, such as IL-6, is not influenced by increased Ca²⁺ influx (Nagai,H. et al., J. Allergy Clin. Immunol., 106:S91-98 (2000)).

In conclusion, TRPM4 functions as a Ca²⁺-activated cation channel in mast cells, and thereby serves as an inhibitor of mast cell activation. By activating TRPM4, Ca²⁺ influx will be reduced and release of proinflammatory mediators is constrained. Our results establish TRPM4 as a novel gene involved in hypersensitivity reactions. As such, the TRPM4-deficient mouse model could provide clues for a new therapeutic strategy in allergy and hypersensitivity reactions, which affect more than 100 million people worldwide (AAAAI, 2000). It is also believed that TRPM4 plays a critical role in other cell types such as cardiomyocytes, especially under pathological conditions such as Ca²⁺ overload in heart failure (Wehrens, X.H., Marks, A.R., Trends Biochem. Sci., 28:671-678 (2003)).

The invention will further be described by the following Examples which are, however, not to be construed as limiting the invention.

### Examples

### Materials and Methods

Solutions: Standard extracellular solution for patch-clamp and Ca²⁺ imaging contained (in mM): 156 NaCl, 1.5 CaCl2, 1 MgCl2, 10 glucose, 10 HEPES, pH7.4 with NaOH. Pipette solution for whole-cell measurements contained: 20 NaCl, 100 NaAsp, 1 MgCl2, 10 HEPES, pH7.2 with NaOH. External solution in inside-out experiments contained: 156 NaCl, 1.5 CaCl2, 1 MgCl2, 10 HEPES, pH7.4 with NaOH. Bath solution (cytosolic side) in inside-out experiments contained: 150 NaCl, 1 MgCl2, 10 HEPES, pH7.2 with NaOH. K⁺ was omitted from the above solutions to avoid contribution of K⁺ currents. In both whole-cell and inside-out studies, the Ca²⁺ concentration at the inner side of the membrane was adjusted between 100 nM and 1µM by adding CaCl₂ to 5 mM EGTA as calculated by the CaBuf program (ftp://ftp.cc.kuleuven.ac.be/pub/droogmans/cabuf.zip). For higher Ca²⁺ concentrations, CaCl₂ was added to an EGTA-free solution. For current clamp measurements, pipette solution contained 55 KCI, 75 K₂SO₄, 5 MgCl₂, 5 Glucose, 10 Hepes, pH7.2. For histamine release KRH buffer was used (130 NaCl, 4.75 KCI, 1.2 KH₂PO₄, 1.2 MgSO₄, 11 glucose, 10 Hepes, 2.54 CaCl₂, pH 7.4). All chemicals were purchased from Sigma (St Louis, MO).

Electrophysiology and Ca²⁺ imaging: Currents were measured in the whole-cell or inside-out configuration using an EPC-7 or EPC-9 patch-clamp amplifier (HEKA Elektronik, Lambrecht, Germany). Patch electrodes had a DC resistance between 2 and 4 MΩ. Currents were sampled at 3 kHz and filtered at 1 kHz. Step protocols consisted of a 400 ms step to -100 mV from a holding of 0mV, followed by a 250 ms step to +100 mV applied at 0.5 Hz. Ramp protocol consisted of a 400ms ramp from -100 to +100 mV from a holding potential of 0 mV, also applied at 0.5 Hz. Capacitance measurements were performed according to the Lindau-Neher technique (Lindau, M., Neher, E., Pflugers Archiv European Journal of Physiology 411:137-146 (1988)). A 1 kHz, 40 mV peak-to-peak sinusoid stimulus was applied about a DC holding potential of 0 mV. Data were acquired through a combination of the time resolution pulse software and the lower time resolution X-chart plug-in to the Pulse software. Pipette solution contained 135 KGlutamate, 20 NaCl, 1 MgCl₂, 10 Hepes, 0.2 Na₂ATP, 300 µM GTP, 100 µM GTPγS, pH7.2 (Penner, R. Neher, E., FEBS Lett. 226:307-313 (1988)). Amphotericine-B (250 µg/ml) was utilized for perforated patches as in (Akaike, N., Harata, N., Jpn. J. Physiol. 44:433-473 (1994)). [Ca²⁺]cyt was measured as described in (Vriens, J. et al., Proc. Natl. Acad. Sci. USA, 101:396-401 (2004)). Cells were loaded with 2 µM Fura-2 acetoxymethyl ester for 20 min at 37 °C in standard extracellular solution. For every condition, at least 20 cells in at least three independent experiments were assayed. Time-points after 3 and 6 h of stimulation were gathered by loading 10⁶ cells with Fura-2am as above, and measured in an Aminco-Bowman Luminescence Spectrometer. Electrophysiological experiments were performed at room temperature (22-25 °C). Ca²⁺ imaging was performed at 37 °C.

Histology: For detection of mast cells in skin biopsies a May-Grünwald - Giemsa staining was performed. Paraffin slices were made, deparaffined, incubated with May Grünwald solution 5 min, rinsed with aqua dest. 5 min, incubated with Giemsa solution 15 min, rinsed with Aqua dest. and incubated with 7 drops Giemsa solution in 10 ml Aqua dest. Mast cells were counted in 15 fields (320 µm x 240 µm) per biopsy using KS300 software (Zeiss). The number of mast cells is given as mean±S.D. The experimentator was blinded to the genotype of the biopsies.

Northern and Western Blotting: Northern blot analysis was performed as described (Freichel, M. et al., FEBS Lett. 422:354-358 (1998)). Poly(A)+ RNA (10 µg) isolated from WT and TRPM4^{-/-} BMMC and kidney were hybridized with a random labelled cDNA probe corresponding to nucleotides 2325-3156 of mouse TRPM4 (GenBank accession nr. AJ575814) and a 239-bp PCR fragment from the human glyceraldehyde-3-phosphate dehydrogenase (GAPDH) cDNA. For Western blots a rabbit polyclonal antibody (578) directed against the amino-terminal end of mouse TRPM4 was generated and affinity purified. Specificity of antibodies was confirmed using microsomal membrane protein fractions (BMMCs and MAECs, 150 µg per lane; cardiac atrium, 50 µg per lane and cardiac ventricle, 100 µg per lane) from WT mice and mice deficient in TRPM4 as well as from non-transfected or mouse TRPM4-transfected (pM4-26, Nilius, B. et al., J. Biol. Chem., 278:30813-30820 (2003)) HEK293 cells. Antimouse GAPDH antibody, anti-Calnexin antibody and anti-CaVß2 antibody 425 as controls for protein loading.

TRPM4-Immunostaining: Skin samples were fixed in 4% fresh paraformaldehyde in 0.1 M PBS overnight, pH 7.2. After several steps of washing with 0.1 M PBS the skin sample were transferred to 18% sucrose overnight and then frozen at -80°C. Thereafter 7 µm cryosection was performed. Slices were incubated 1h at RT with the anti-TRPM4 antibody 587 (1:50) that was preabsorbed using microsomal membrane protein fractions from TRPM4^{-/-} BMMCs. As biotinylated secondary antibody a goat-anti rabbit-IgG (E 0433 Dako, Denmark, 1:400) was used for 1 h at RT. Thereafter cells were incubated with extravidin-horseradish-peroxidase-complex (PRN 1051, Amershan, 1:150) for 60 min and visualized by incubation with a DAB / NiSo4 solution for 15 min.

Electron microscopy: BMMC's were fixed with 2.5% glutaraldehyde, 1% freshly depolymerised paraformaldehyde in PBS (3 h at 4 °C). During the first 30min of fixation, mast cells were fixed in suspension, and after brief centrifugation further fixed as a cell pellet in the same fixative for additional 2.5 h. Samples were osmicated with 2% OsO₄ in 100 mM cacodylate buffer, pH 7.4 (1 h, 4 °C), bloc-contrasted with 2% uranyl acetate in 50 mM Na-maleate, pH 5.2 (3 h, 4 °C), dehydrated using an ascending ethanol concentration series and using propyleneoxide, infiltrated with Epon 812 resin and polymerized at 60 °C for 24 h. Ultrathin sections were cut with an ultramicrotome (Reichert) and analyzed with a Tecnai 12 Biotwin digital electron microscope (FEI) operated at 100 kV. Skin samples were fixed in 4% fresh paraformaldehyde in 0.1 M PBS overnight, pH 7.2, dehydrated in a graded alcohol series, and embedded in araldite. Sections of plastic-embedded specimens were cut with glass for thin sections and diamond knife for ultrathin sections on a Reichert ultramicrotome. Ultrathin 60 nm sections were examined using an electron microscope (902A, Leo, Oberkochen, Germany) after further contrasting with uranyl acetate-lead citrate.

Data Analysis: Electrophysiological data were analyzed using WinASCD software (ftp://ftp.cc.kuleuven.ac.be/pub/droogmans/winascd.zip). Pooled data are given as mean±s.e.m. of n independent experiments. Mast cell density in skin biopsies is given as mean±s.d. Origin 7.0 (OriginLab, Northampton MA, USA) was used for statistical analysis. Significance was tested by the two sample Student's t test (P<0.05 for significance).

### Example 1: TRPM4 gene targeting and generation of mouse lines

For construction of the targeting vector shown in SEQ ID N0:2, genomic DNA-fragments were amplified from a 129/SvJ murine BAC clone (RPCI 22 library, Roswell Park Cancer Institute, Buffalo, NY). A floxed TRPM4 allele was constructed **(die folgende Passage sollte detaillierter sein)**. 5' homology contained exons 13-16 of the TRPM4 gene and exon 15 and 16 were flanked with two loxP sites. A lacZ-FRT-PGKneo-FRT cassette (Kulessa and Hogan, 2002) with a third loxP site was cloned downstream of the flanked exon (Figures 1A, and 7A). 3' homology contained exon 17 and was followed by a HSV thymidine kinase cassette (TK). R1 embryonic stem cells (1 x 10⁷) were electroporated with the linearized targeting vector and plated on irradiated G418-resistant embryonic feeder cells isolated from TRPC4^{+/-} embryos (Freichel et al., 2001). Recombinant clones were selected with G418 (0.25 mg ml-1) and ganciclovir (2 µM). Homologous recombination was confirmed using Southern Blot analysis in 25 of 309 double resistant ES cell clones. From 25 clones, 8 clones displayed a TRPM4 allele containing all 3 loxP sites (TRPM4L3F2) whereas 17 clones were lacking the first loxP site (TRPM4L2F2). Two independent TRPM4L3F2 cell clones clones were used to generate chimeric mice. Mating of mice heterozygous for the TRPM4L3F2 allele (TRPM4⁺/L3F2) with the deleter mouse strain CMV-Cre (Schwenk, F. et al., Nucleic Acids Res., 23:5080-5081 (1995)) produced mouse lines carrying a null-allele (TRPM4 -). Mice were kept in essentially specific pathogen-free environment and all phenotypic analyses were performed using mice of a 129/SvJ genetic background, matched of gender and age. Mice were routinely genotyped using PCR.

### Example 2: Bone Marrow Isolation and Mast Cell Differentiation

Isolation and differentiation of murine bone marrow cells (BMMC) were performed as in (Laffargue et al, 2002). In brief, bone-marrow cells were centrifuged from excised femurs and cultured at 10⁶ cells/ml in IMDM (Invitrogen) supplemented with 10% heat-inactivated fetal calf serum, 50 µM β-mercaptoethanol (Sigma), 10 U/ml of each penicillin and streptomycin, and 2 ng/ml recombinant murine IL-3 (R&D Systems) at 37 °C, 5% CO₂. IL-3 was added twice weekly. The culture was weekly diluted to 0.5 x 10⁶ cells/ml. After 4 weeks of differentiation, expression of FceRI in BMMC was analysed after the blockage of Fc.receptors with 2.4G2 rat anti-mouse Fc.RII/RIII antibody (Pharmingen), followed by individual incubations with mouse IgE (Sigma) and FITC-labeled monoclonal rat anti-mouse IgE antibody (Pharmingen). For c-Kit expression, Fc. receptors were blocked as above, and cells were subsequently incubated with PE-labeled rat anti-c-Kit monoclonal antibody (Pharmingen). Flow cytometry was carried out on a Galaxy Flow Cytometry system (DAKO). 96.5 - 99.5 % of cultured cells of different preparations from both genotypes expressed both FceRI and c-Kit.

### Example 3: Mast cell mediator release

Histamine release from mast cells was determined using the O-phthaldialdehyde assay (Yoshimura, T. et al., Anal. Biochem., 164:132-137 (1987)). 0.5 x 10⁶ cells were washed twice and resuspended in KRH buffer (130 NaCl, 4.75 KCl, 1.2KH₂PO₄, 1.2 MgSO₄, 11 glucose, 10 Hepes, 2.54 CaCl₂, pH 7.4). DNP, adenosine and ionomycin were added to the suspension and cells were incubated for 5 to 90 min at 37 °C. To determine total histamine content (Ftotal), one sample is resuspended in 1% Triton^{®} X-100 and incubated at 95 °C for cell lysis. After incubation, cells were centrifuged and 350 µl H20, 100µl 1N NaOH and 25 µl 1% phthaldialdehyde (Sigma StLouis, MO) were added to 150 µl supernatant. After 4 min incubation at room temperature 50 µl 3N HCl was supplemented. The amount of histamine derivative was measured fluorometrically in a 96-well reader (Tecan GENios), at 360 nm (excitation) and 450 nm (emission). The ratio Ftest/Ftotal gives the percentage of total histamine content released. Data were collected from 4 independent BMMC preparations from each genotype. In each preparation results were similar to the overall average. Release of IL-6 and TNFα was assayed using ELISA duokits (R&D Biosystems) whereas LTC4, LTD4 and LTE4 release was assayed using an enzyme-linked immunoassay (GE Healthcare) according to the manufacturers guidelines.

### Example 4: Passive cutaneous anaphylaxis

The protocol for immediate phase PCA reactions was adapted from (Ali et al., 2004). 30 or 60 ng of anti-DNP IgE in 30 µl 0.9% NaCl was injected intradermally at two sites in the dorsal skin from 2 to 4 month old mice under isoflurane (1.5%) narcosis. At a third site only an injection wound was applied (sham). 48h following injection of anti-DNP IgE 100 µg of DNP in 200 µl 0.1% Evan's blue dye in non-pyrogenic 0.9% NaCl solution was injected intravenously through the retrobulbar plexus under isoflurane narcosis. In control mice non-pyrogenic 0.9% NaCl was injected instead of anti-DNP IgE. One hour after DNP injection mice were sacrificed by cervical dislocation and skin biopsies around the injection site were taken with a biopsy punch (8 mm). Biopsies were incubated in 500µl formamid at 48h, 55°C to extract Evan's blue dye. Extravasation of Evan's blue was quantified by absorbance measurements at 620 nm. For background subtraction, A620 values from sham-injected sites were subtracted from A620 values from IgE injected sites. The late phase PCA reaction was probed as described by (Klemm et al., 2006 ??). Mice were passively sensitized by intravenous injection of 2 µg anti-DNP IgE (clone SPE-7, Sigma). 24 h after sensitisation a cutaneous reaction was elicited by applying 10 µl 0,3% DNFB acetone/sunflower oil solution to both sides of the right ear. The cutaneous reaction was assessed by measuring the ear thickness using a micrometer 1 to 48 h after antigen application. The untreated left ear served as control. The increment of ear thickness was expressed as the percentage of the baseline value obtained before antigen challenge. All animal experiments were carried out after approval by the local government, in accordance with German legislation on protection of animals and the National Institute of Health Guide for the Care and Use of Laboratory Animals.

### Example 5: Organbath and telemetry

Five-to-six-month-old mice of either sex were anesthetised using tribromethanol (~0,6 g per kg body weight). The heart or the thoracic aorta was immediately dissected and placed in oxygenated, Krebs-Henseleith solution at room temperature containing (mM): NaCl 118, KCI 4.7, CaCl₂ 2.52, MgSO₄ 1.64, NaHCO₃ 24.88, KH₂PO₄ 1.18, Na-pyruvate 1.0, glucose 5.0. Right atria were rapidly dissected. Aorta and carotid artery were cut into rings of about 3.0 mm. Two pairs of tissues were mounted and attached to force displacement transducers (TIM-1020) in an organ-bath (IOA-5301; FMI GmbH, Seeheim/Ober-Beerbach) containing above solution at 37 °C. The pH of the solution was maintained at 7.4 by passing a mixture of 95% O₂ and 5% CO₂ into the bath. Spontaneously beating right atria were set up to a basic tension of 0.3-0.5 g to obtain stable continuous measurements of the frequency and the force of contractions. Optimal resting tension was applied to aortic (0.8-1 g) and carotid artery (0.5 g) rings before precontraction with prostaglandin F2a (3 µM). Relaxation was induced by Ach (10-8 to 10-5 M). Circadian rhythm and heart rate in freely moving mice was assessed one week after implantation of telemetric devices (Data Science International).

### Example 6: Insulin secretion and glucose tolerance test

Islets were hand-picked in groups of ten and washed twice in RPMI (in the absence of glucose). Islets were subsequently pre-incubated for 60 min at 37 °C in Krebs-Ringer bicarbonate buffer, pH 7.4, supplemented with 0.05% BSA, and 1 mM glucose. Following pre-incubation, the supernatant was discarded and the islets were incubated in buffer solution containing 1 or 20 mM glucose for 60 min at 37 °C. An aliquot of the supernatant was removed immediately after incubation and frozen for insulin assay. The pellets were resuspended in acid ethanol and frozen for insulin content measurement. The ELISA kit used for insulin determination was obtained from Mercodia (Sweden). Insulin secretion data are expressed as ng/islet/h. Student's t-tests were used to assess statistical significance between treatments: differences were considered statistically significant at p<0.05. Seven to eight week old male WT and TRPM4^{-/-}mice were fasted over night (14-16 h). Blood glucose levels were measured using an FreeStyle Blood Glucose Monitoring System (Disetronic) from tail bleedings before and 15, 30, 60 and 120 min after intraperitoneal injection of D-glucose (2 g/kg body weight) as described (Berggren, P.O., et al., Cell, 119:273-284 (2004)).

Signal transduction experiments. For FceRI signaling, 2 x 106 BMMCs/ml were preloaded with 0.5 µg/ml anti-DNP IgE and subsequently activated by adding 100 ng/ml DNP-HSA, for time periods as indicated in the figure. Cells were lysed, and denatured proteins (2x 105 cell equivalents per lane) were separated on 7 or 10% polyacrylamide gels and subjected to immunoblotting using antibodies against PLC.1, PLC.2, phospho-p38, p38, phospho-p44/42, p44/42, phospho-Akt, Akt, (all from Cell Signaling), or ß-actin (Sigma-Aldrich).

### Sequence Listing, Free text

SEQ ID NO:1 human TRPM4
SEQ ID NO:2 targeting vector (pM4-21)
Features: 5' homology arm: 1-4118
   LoxP1: 1732-1765
   LacZ-Neo cassette: 4137-9656
   LoxP2: 4137-4170
   Frt1: 7784-7831
   Frt2: 9555-9602
   LoxP3: 9623-9656
   Neo Cassette between FRT1 and FRT2
   Lac Z between LoxP2 and FRT1
   3' homology arm: 9657-13656
   downstream of 3' homology arm: TK cassette (for negative selection)

## Claims

1. A transient receptor potential membrane protein 4(TRPM4)-deficient non-human animal.

2. The non-human animal of claim 1, which
(i) does not express functional TRPM4 or expresses suboptimal levels of TRPM4,
(ii) is TRPM4-deficient due to mutation, or due to a partial or entire deletion of the TRPM4 gene, preferably by excision of one of the transmembrane domains, such as exons 15 and 16 encoding the first transmembrane domain of the TRPM4 gene or of an exon 5' thereof, or by excision of a domain of the channel pore between exons 5 and 6; and/or
(iii) is a vertebrate, preferably is a mammal, such as a rodent including mouse and rat, or a non-mammal, such as fish including zebrafish, or is an invertebrate including worm and insects, preferably the non-human animal is a mouse;
(iv) the genome of the animal comprises a homozygous or a heterozygous disruption, preferably a homozygous disruption of the TRPM4 gene.

3. A tissue or cell culture derived from the non-human animal of claim 1 or 2.

4. A process for preparing the non-human animal of claim 1 or 2, which comprises transfecting a cell with a targeting vector comprising 5' and 3' homology arms flanking a section of the TRPM4 gene to be modified by the targeting vector.

5. The process of claim 4, wherein
(i) the targeting vector further comprises one or more functional sequences selected from recombinase recognition sequences (RRS), selection marker genes, reporter genes, expression control elements including promoter sequences, polyadenylation sequences, introns, IRES, splice donor and splice acceptor sequences; and/or
(ii) the non-human animal is a mammal and the cell that is transfected is an ES cell.

6. The process of claim 4 or 5, wherein
(i) the homology arms has a length of 0.1 to 20 kb, preferably 0.5 to 10 kb, and/or
(ii) the targeting vector contains a 5' homology arm corresponding to exons 13-16 of the TRPM4 gene and a 3' homology arm corresponding to exon 17 of the TRPM4 gene, wherein exons 15 and 16 are flanked by two RRS of a first recombinase.

7. The process of claim 6, wherein the targeting vector
(i) contains a first selection cassette with a positive selection marker and a third RRS of the first recombinase being located downstream of the exons flanked by said two RRS of the first recombination; and/or
(ii) said first selection cassette contains a pair of RRS of a second recombinase flanking the positive selection marker gene; and/or
(iii) contains a first reporter gene adjacent to the first selection cassette, preferably upstream of the first selection cassette; and/or
(iv) contains a second selection cassette with a negative selection marker gene or a second reporter gene downstream of the 3' homology arm.

8. The process of claim 7, wherein
(i) the RRS of the first and second recombinase are selected from wt or mutant loxP, FRT, C31Int, λ-Int sites,
(ii) the positive selection marker gene is a DNA sequence encoding a protein conferring resistance against cell poison, including neomycine, hygromycine, puromycine histidinol and bleomycine or is a DNA sequence containing superior metabolic properties to the cells including the utilization of xanthine and adenine ... (?), preferably the positive selection marker gene is a neomycine phosphotransferase gene;
(iii) the negative selection marker is a DNA sequence encoding a cell poison such as the HSV thymidine kinase gene and diphtheria toxin A, preferably is the HSV thymidine kinase gene,
(iv) first and second reporter gene, which preferably are differing from each other, are selected from genes coding for fluorescent proteins such as GFP, Dsred and modifications thereof, enzymes
preferably the targeting vector has the sequence shown in SEQ ID NO:2.

9. The process of claims 4 to 9 above which further comprises
(i) selecting for positive homologous recombination events, and/or
(ii) generating chimeric non-human animals, and/or ...

10. A targeting vector as described in claims 4 to 8 above.

11. Use of the non-human animal of claim 1 or 2 or the tissue or cell culture of claim 3
(i) as a test system for allergy, hypersensitive reactions, pancreatitis, arrhythmia and hypertension, especially as a test system for validation or evaluation of substances with respect to their specificity for TRPM4 channels, as a test system for TRPM4-mediated reactions, and for identifying substances acting on TRPM4 proteins or on protein complexes containing TRPM4 proteins;
(ii) as a test system for all kind of hypersensitivity and allergy reactions including cold urticaria, synthesis of prostaglandins, leukotriens or other lipid mediators,secretion of preformed mediators such as histamine, serotonine, proteoglycans or proteases, liberation of tumor necrosis factor alpha and other cytokines and chemokines, mast cell responses to bacterila and parasitic infections including activation of the complement system, to test for the role of TRPM4 in the regulation of calcium entry into cardiomyocytes, smooth muscle cells, chromaffin cells, endotheial cells, pancreatic beta cells and acinar cels, cells of the intestine, neuronal cell ssuch as in the vomeronasal organ, macula densa cells;
(iii) to test for organ and systemic body functions under physiological conditions and disease, such as vascular contractility, taste, smelling, aldosterone and renin secretion, insulin secretion, heart rhythm and contractility, pancreatitis, renal function.
